# EUROPEAN PATENT SPECIFICATION

(11) **EP 2 935 235 B1**
(45) Date of publication and mention of the grant of the patent: **21.02.2018**
(21) Application number: 13794819.6
(22) Date of filing: 21.11.2013
(51) Int. Cl.: C07D 333/78, C07D 495/04, C07D 495/14, C07D 513/14, C07C 13/62, C07F 7/08, C09K 11/06, H01L 51/00

(54) **INDENOPHENANTHRENE BASED COMPOUNDS**
VERBINDUNGEN AUF INDENOPHENANTHRENBASIS
COMPOSÉS À BASE D'INDÉNOPHÉNANTHRÈNE

(30) Priority: 18.12.2012 EP 12008418
(43) Date of publication of application: 28.10.2015
(73) Proprietor: Merck Patent GmbH, 64293 Darmstadt (DE)
(72) Inventor: TONG, Qiong, 64291 Darmstadt (DE); HEUN, Susanne, 65812 Bad Soden (DE)
(86) International application number: PCT/EP2013/003516
(87) International publication number: WO 2014/094954

(56) References cited:
- EP-A1- 2 075 274
- EP-A1- 2 075 274
- EP-A1- 2 098 527
- WO-A1-2012/017184
- WO-A1-2012/017184
- WO-A1-2013/009079
- WO-A1-2013/100507
- WO-A2-2011/139125
- KR-A- 20120 072 784
- KR-A- 20120 122 980

## Description

### Technical Field

The invention relates to novel compounds containing repeat units based on a combination of phenanthrene and indenofluorene, to methods for their preparation and educts or intermediates used therein, to polymer blends, mixtures and formulations containing them, to the use of the compounds, polymer blends, mixtures and formulations as organic semiconductors in organic electronic (OE) devices, and to OE, devices comprising these polymers, polymer blends, mixtures or formulations.

### Background

Conjugated polymers on the basis of polyphenylene backbones have found widespread application in organic electronic (OE) devices such as Organic Light Emitting Diodes (OLEDs), Organic Field Effect Transistors (OFETs), Organic Photovoltaics (OPV) and Organic Photodetectors (OPD).

The conjugated backbone in polymer based OE devices is mainly responsible for charge transport. In order to have faster and more stable transport behavior, new backbones and thus new monomers are required to achieve charge carrier mobilities where OFETs can become competitive and OLED and OPV devices show low driving voltages.

There is still a need for conjugated polymers which are easy to synthesize, especially by methods suitable for mass production, that show good structural organization and film-forming properties, exhibit good electronic properties, especially a high charge carrier mobility, a good processibility, especially a high solubility in organic solvents, high stability in air, and that are suitable in OE devices like OFET, OLED and OPV devices.

It was an aim of the present invention to provide improved conjugated polymers showing one or more of the aforementioned desired properties. Another aim of the invention was to extend the pool of semiconducting materials available to the expert. Other aims of the present invention are immediately evident to the expert from the following detailed description.

The inventors of the present invention have found that one or more of the above aims can be achieved by providing compounds as disclosed and claimed hereinafter. These compounds comprise a conjugated backbone with repeating units comprising a fused system of phenanthrene and indenofluorene.

KR 2012-0122980 A, KR 2012-0072784 A, WO 2013/009079 A1, WO 2011/139125 A2, WO 2013/100507 A1, EP 2 098 527 A1, EP 2 075 274 A1 and WO 2012/017184 A1 disclose small molecules comprising polycyclic units for use in OLEDs or other electronic devices, but do not disclose a conjugated polymer as disclosed and claimed hereinafter.

### Summary

The invention relates to a compounds that is a polymer with a conjugated backbone comprising more than one units of formula I 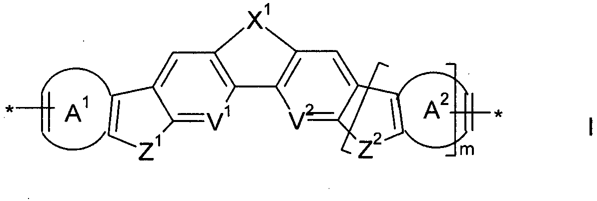 wherein
- X¹: is CR¹=CR² or CR¹R²-CR¹R²,
- V¹ and V²: denote CH,
- Z¹ and Z²: independently of each other denote S, O or CR³R⁴,
- A¹ and A²: independently of each other denote a mono- or bicyclic aromatic or heteroaromatic group that is selected from formulae (2a), (3a), (6a), (7a), (10a) and (13a) as defined below,
- R¹ and R²: are as defined below
- R³ and R⁴: are as defined below,
- m: is 0 or 1,
and the * indicates the bonds that link the unit either with a polymer or other optoelectronically functional groups.

The invention further relates to polymers comprising one or more repeating units containing or being selected from units of formula I and optionally comprising one or more repeating units selected from aryl and heteroaryl units that are optionally substituted, and wherein at least one repeating unit in the polymers contains at least one unit of formula I.

The invention further relates to monomers containing a unit of formula I and further containing one or more reactive groups which can be reacted to form a conjugated polymer as described above and below. This monomer can also be used to form functional small molecules.

The invention thus further relates to small molecules comprising units according to formula I, optionally combined with hole transporting, electron transporting, electron donor, electron acceptor, light emitting, charge generating or solubilising groups.

The invention further relates to the use of the compounds according to the present invention as electron donor or p-type semiconductor.

The invention further relates to the use of the compounds according to the present invention as electron donor component in a semiconducting material, formulation, polymer blend, device or component of a device.

The invention further relates to the use of the compounds according to the invention as hole transport, electron transport, electron blocking, hole blocking, charge generation, conducting, semiconducting or light emitting material.

The invention further relates to a semiconducting material, formulation, mixture, polymer blend, device or component of a device, comprising a compound according to the present invention as electron donor component, and preferably further comprising one or more compounds having electron acceptor properties.

The invention further relates to a mixture or polymer blend comprising one or more compounds according to the present invention and one or more additional compounds which are preferably selected from compounds having one or more of semiconducting, charge transport, hole or electron transport, hole or electron blocking, electrically conducting, photoconducting or light emitting properties.

The invention further relates to a formulation comprising one or more compounds, polymers, mixtures or polymer blends according to the present invention and optionally one or more solvents, preferably selected from organic solvents.

The invention further relates to organic semiconducting formulations comprising one or more compounds comprising a unit of formula I, one or more organic binders, or precursors thereof, preferably having a permittivity ε at 1,000 Hz and 20°C of 3.3 or less, and optionally one or more solvents.

The invention further relates to a charge transport, semiconducting, electrically conducting, photoconducting or light emitting material comprising a compound, polymer, formulation, mixture or polymer blend according to the present invention.

The invention further relates to the use of a compound, polymer, formulation, mixture or polymer blend of the present invention as charge transport, semiconducting, electrically conducting, photoconducting or light emitting material, or in an optical, electrooptical, electronic, electroluminescent or photoluminescent device, or in a component of such a device or in an assembly comprising such a device or component.

The invention further relates to an optical, electrooptical, electronic, electroluminescent or photoluminescent device, or a component thereof, or an assembly comprising it, which comprises a compound, polymer, formulation, mixture or polymer blend, or comprises a charge transport, semiconducting, electrically conducting, photoconducting or light emitting material, according to the present invention.

The optical, electrooptical, electronic, electroluminescent and photoluminescent devices include, without limitation, organic field effect transistors (OFET), organic thin film transistors (OTFT), organic light emitting diodes (OLED), organic light emitting transistors (OLET), organic photovoltaic devices (OPV), organic photodetectors (OPD), organic solar cells, laser diodes, Schottky diodes, photoconductors and photodetectors.

The components of the above devices include, without limitation, charge injection layers, charge transport layers, interlayers, planarising layers, antistatic films, polymer electrolyte membranes (PEM), conducting substrates and conducting patterns.

The assemblies comprising such devices or components include, without limitation, integrated circuits (IC), radio frequency identification (RFID) tags or security markings or security devices containg them, flat panel displays or backlights thereof, electrophotographic devices, electrophotographic recording devices, organic memory devices, sensor devices, biosensors and biochips.

In addition the compounds, polymers, formulations, mixtures or polymer blends of the present invention can be used as electrode materials in batteries and in components or devices for detecting and discriminating DNA sequences.

### Detailed Description

As used herein, the terms "donor" or "donating" and "acceptor" or "accepting" will be understood to mean an electron donor or electron acceptor, respectively. "Electron donor" will be understood to mean a chemical entity that donates electrons to another compound or another group of atoms of a compound. "Electron acceptor" will be understood to mean a chemical entity that accepts electrons transferred to it from another compound or another group of atoms of a compound. (see also U.S. Environmental Protection Agency, 2009, Glossary of technical terms, http://www.epa.gov/oust/cat/TUMGLOSS.HTM

As used herein, the term "n-type" or "n-type semiconductor" will be understood to mean an extrinsic semiconductor in which the conduction electron density is in excess of the mobile hole density, and the term "p-type" or "p-type semiconductor" will be understood to mean an extrinsic semiconductor in which mobile hole density is in excess of the conduction electron density (see also, J. Thewlis, Concise Dictionary of Physics, Pergamon Press, Oxford, 1973).

As used herein, the term "polymer" will be understood to mean a molecule of high relative molecular mass, the structure of which essentially comprises the multiple repetition of units derived, actually or conceptually, from molecules of low relative molecular mass (Pure Appl. Chem., 1996, 68, 2291), and will be understood to include also oligomers and dendrimers. The term "oligomer" will be understood to mean a molecule of intermediate relative molecular mass, the structure of which essentially comprises a small plurality of units derived, actually or conceptually, from molecules of lower relative molecular mass (Pure Appl. Chem., 1996, 68, 2291). Polymers and oligomers can be linear or branched with a branching factor of 0, i.e. a linear polymer, or a branching factor of 1, i.e. a totally branched polymer, also known as "dendrimer". In a preferred meaning as used herein a polymer will be understood to mean a compound having > 1, i.e. at least 2 repeat units, preferably ≥ 5 repeat units, and an oligomer will be understood to mean a compound with > 1 and < 10, preferably < 5, repeat units.

Preferably, the polymers according to this invention have a molecular weight average Mw between 1 000 and 2 000 000 g/mol, more preferably between 10 000 and 1 000 000 g/mol, even more preferably between 30 000 and 800 000 g/mol, and most preferably between 50 000 and 500 000 g/mol. The molecular weight Mw can be determined by standard methods such as gel permeation chromatography (GPC).

As used herein, the term "conjugated" will be understood to mean a compound (for example a polymer) that contains mainly C atoms with sp²-hybridisation (or optionally also sp-hybridisation), and wherein these C atoms may also be replaced by hetero atoms. In the simplest case this is for example a compound with alternating C-C single and double (or triple) bonds, but is also inclusive of compounds with aromatic units like for example 1,4-phenylene. The term "mainly" in this connection will be understood to mean that a compound with naturally (spontaneously) occurring defects, which may lead to interruption of the conjugation, is still regarded as a conjugated compound. An in-chain unit such as a triarylamine is also not considered to break the conjugation.

The polymers according to this invention can also be partially conjugated, contain conjugated and unconjugated blocks, or comprise randomly or regularly distributed conjugation breakers. Examples for polymers with conjugation breaking units can be found in WO2006/061181, WO2010/136110 and WO2010/136111.

The linear and branched polymers according to this invention can also carry additional reactive groups. Linear polymers according to this invention can then be cross-linked to form a subsequently insoluble film. Examples for cross-linkable polymers can be found in EP2401315, WO2010/097155, and EP1671379. Moreover, polymers can be formed directly on a substrate from reactive monomers containing a unit according to formula I (precursor-route polymers).

Further, as used herein, the term "polymer" will be understood to mean a molecule that encompasses a backbone (also referred to as "main chain") of one or more distinct types of repeat units (the smallest constitutional unit of the molecule) and is inclusive of the commonly known terms "oligomer", "copolymer", "homopolymer" and the like. Further, it will be understood that the term polymer is inclusive of, in addition to the polymer itself, residues from initiators, catalysts and other elements attendant to the synthesis of such a polymer, where such residues are understood as not being covalently incorporated thereto. Further, such residues and other elements, while normally removed during post polymerization purification processes, are typically mixed or co-mingled with the polymer such that they generally remain with the polymer when it is transferred between vessels or between solvents or dispersion media.

As used herein, in a formula showing a polymer or a repeat unit, like for example a unit of formula I or one of its subformulae, an asterisk (*) will be understood to mean a chemical linkage to an adjacent unit or to a terminal group in the polymer backbone. In a ring, like for example a benzene or thiophene ring, an number sign (#) will be understood to mean a C atom that is fused to an adjacent ring.

As used herein, the terms "repeat unit", "repeating unit" and "monomeric unit" are used interchangeably and will be understood to mean the constitutional repeating unit (CRU), which is the smallest constitutional unit the repetition of which constitutes a regular macromolecule, a regular oligomer molecule, a regular block or a regular chain (Pure Appl. Chem., 1996, 68, 2291). As further used herein, the term "unit" will be understood to mean a structural unit which can be a repeating unit on its own, or can together with other units form a constitutional repeating unit.

As used herein, a "terminal group" will be understood to mean a group that terminates a polymer backbone. The expression "in terminal position in the backbone" will be understood to mean a divalent unit or repeat unit that is linked at one side to such a terminal group and at the other side to another repeat unit. Such terminal groups include endcap groups, or reactive groups that are attached to a monomer forming the polymer backbone which did not participate in the polymerisation reaction.

As used herein, the term "endcap group" will be understood to mean a group that is attached to, or replacing, a terminal group of the polymer backbone. The endcap group can be introduced into the polymer by an endcapping process. Endcapping can be carried out for example by reacting the terminal groups of the polymer backbone with a monofunctional compound ("endcapper") like for example an alkyl- or arylhalide, an alkyl- or arylstannane or an alkyl- or arylboronate. The endcapper can be added for example after the polymerisation reaction. Alternatively the endcapper can be added in situ to the reaction mixture before or during the polymerisation reaction. In situ addition of an endcapper can also be used to terminate the polymerisation reaction and thus control the molecular weight of the forming polymer. Typical endcap groups are for example aryl, especially phenyl, and lower alkyl.

As used herein, the term "leaving group" will be understood to mean an atom or group (which may be charged or uncharged) that becomes detached from an atom in what is considered to be the residual or main part of the molecule taking part in a specified reaction (see also Pure Appl. Chem., 1994, 66, 1134).

As used herein, unless stated otherwise the molecular weight is given as the number average molecular weight Mₙ or weight average molecular weight M_{W}, which is determined by gel permeation chromatography (GPC) against polystyrene standards in eluent solvents such as tetrahydrofuran, trichloromethane (TCM, chloroform), chlorobenzene or 1,2,4-trichlorobenzene. Unless stated otherwise, 1,2,4-trichlorobenzene is used as solvent. The degree of polymerization, also referred to as total number of repeat units, n, will be understood to mean the number average degree of polymerization given as n = Mₙ/M_{U}, wherein Mₙ is the number average molecular weight and M_{U} is the molecular weight of the single repeat unit, see J. M. G. Cowie, Polymers: Chemistry & Physics of Modern Materials, Blackie, Glasgow, 1991.

As used herein, the term "small molecule" will be understood to mean a monomeric compound which typically does not contain a reactive group by which it can be reacted to form a polymer, and which is designated to be used in monomeric form. In contrast thereto, the term "monomer", unless stated otherwise, will be understood to mean a monomeric compound that carries one or more reactive functional groups by which it can be reacted to form a polymer or copolymer.

As used herein, the term "carbyl group" denotes any monovalent or multivalent organic moiety which comprises at least one carbon atom either without any non-carbon atoms (like for example -C≡C-), or optionally combined with at least one non-carbon atom such as N, O, S, P, Si, Se, As, Te or Ge (for example carbonyl etc.). The term "hydrocarbyl group" will be understood to mean a carbyl group that does additionally contain one or more H atoms and optionally contains one or more hetero atoms like for example N, O, S, P, Si, Se, As, Te or Ge.

As used herein, the term "hetero atom" will be understood to mean an atom in an organic compound that is not a H- or C-atom, and preferably will be understood to mean N, O, S, P, B, Si, Se, As, Te or Ge.

A carbyl or hydrocarbyl group comprising a chain of 3 or more C atoms may be straight-chain, branched and/or cyclic, including spiro and/or fused rings.

Preferred carbyl and hydrocarbyl groups include alkyl, alkoxy, alkylcarbonyl, alkoxycarbonyl, alkylcarbonyloxy and alkoxycarbonyloxy, each of which is optionally substituted and has 1 to 40, preferably 1 to 25, very preferably 1 to 18 C atoms, furthermore optionally substituted aryl or aryloxy having 6 to 40, preferably 6 to 25 C atoms, furthermore alkylaryloxy, arylcarbonyl, aryloxycarbonyl, arylcarbonyloxy and aryloxycarbonyloxy, each of which is optionally substituted and has 6 to 40, preferably 7 to 40 C atoms, wherein all these groups do optionally contain one or more hetero atoms, preferably selected from N, O, S, P, Si, Se, As, Te and Ge.

The carbyl or hydrocarbyl group may be a saturated or unsaturated acyclic group, or a saturated or unsaturated cyclic group. Unsaturated acyclic or cyclic groups are preferred, especially aryl, alkenyl and alkynyl groups (especially ethynyl). Where the C₁-C₄₀ carbyl or hydrocarbyl group is acyclic, the group may be straight-chain or branched. The C₁-C₄₀ carbyl or hydrocarbyl group includes for example: a C₁-C₄₀ alkyl group, a C₁-C₄₀ fluoroalkyl group, a C₁-C₄₀ alkoxy or oxaalkyl group, a 6₂-O₄₀ alkenyl group, a C₂-C₄₀ alkynyl group, a C₃-C₄₀ allyl group, a C₄-C₄₀ alkyldienyl group, a C₄-C₄₀ polyenyl group, a C₂-C₄₀ ketone group, a C₂-C₄₀ ester group, a C₆-C₁₈ aryl group, a C₆-C₄₀ alkylaryl group, a C₆-C₄₀ arylalkyl group, a C₄-C₄₀ cycloalkyl group, a C₄-C₄₀ cycloalkenyl group, and the like. Preferred among the foregoing groups are a C₁-C₂₀ alkyl group, a C₁-C₂₀ fluoroalkyl group, a C₂-C₂₀ alkenyl group, a C₂-C₂₀ alkynyl group, a C₃-C₂₀ allyl group, a C₄-C₂₀ alkyldienyl group, , a C₂-C₂₀ ketone group, a C₂-C₂₀ ester group, a C₆-C₁₂ aryl group, and a C₄-C₂₀ polyenyl group, respectively. Also included are combinations of groups having carbon atoms and groups having hetero atoms, like e.g. an alkynyl group, preferably ethynyl, that is substituted with a silyl group, preferably a trialkylsilyl group.

The terms "aryl" and "heteroaryl" as used herein preferably mean a mono-, bi- or tricyclic aromatic or heteroaromatic group with 4 to 60, preferably 4 to 30, ring atoms, and optionally containing hetero atoms such as B, Si, N, S, Se or O, that may also comprise condensed rings and is optionally substituted with one or more groups R¹ through R⁴ as defined above and below.

Especially preferred aryl and heteroaryl groups are phenyl in which, in addition, one or more CH groups may be replaced by N, naphthalene, thiophene, selenophene, thienothiophene, dithienothiophene, fluorene and oxazole, all of which can be unsubstituted, mono- or polysubstituted with L as defined above. Very preferred rings are selected from pyrrole, preferably N-pyrrole, furan, pyridine, preferably 2- or 3-pyridine, pyrimidine, pyridazine, pyrazine, triazole, tetrazole, pyrazole, imidazole, isothiazole, thiazole, thiadiazole, isoxazole, oxazole, oxadiazole, thiophene, preferably 2-thiophene, selenophene, preferably 2-selenophene, thieno[3,2-b]thiophene, thieno[2,3-b]thiophene, furo[3,2-b]furan, furo[2,3-b]furan, seleno[3,2-b]selenophene, seleno[2,3-b]selenophene, thieno[3,2-b]selenophene, thieno[3,2-b]furan, indole, isoindole, benzo[b]furan, benzo[b]thiophene, benzo[1,2-b;4,5-b']dithiophene, benzo[2,1-b;3,4-b']dithiophene, quinole, 2- methylquinole, isoquinole, quinoxaline, quinazoline, benzotriazole, benzimidazole, benzothiazole, benzisothiazole, benzisoxazole, benzoxadiazole, benzoxazole, benzothiadiazole, all of which can be unsubstituted, mono- or polysubstituted with L as defined above. Further examples of aryl and heteroaryl groups are those selected from the groups shown hereinafter.

An alkyl or alkoxy radical, i.e. where the terminal CH₂ group is replaced by -O-, can be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

An alkenyl group, wherein one or more CH₂ groups are replaced by - CH=CH- can be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are C₂-C₇-1E-alkenyl, C₄-C₇-3E-alkenyl, C₅-C₇-4-alkenyl, C₆-C₇-5-alkenyl and C₇-6-alkenyl, in particular C₂-C₇-1E-alkenyl, C₄-C₇-3E-alkenyl and C₅-C₇-4-alkenyl. Examples for particularly preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C atoms are generally preferred.

An oxaalkyl group, i.e. where one CH₂ group is replaced by -O-, is preferably straight-chain 2-oxapropyl (=methoxymethyl), 2-(=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.Oxaalkyl, i.e. where one CH₂ group is replaced by - O-, is preferably straight-chain 2-oxapropyl (=methoxymethyl), 2-(=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

In an alkyl group wherein one CH₂ group is replaced by -O- and one by - C(O)-, these radicals are preferably neighboured. Accordingly these radicals together form a carbonyloxy group -C(O)-O- or an oxycarbonyl group -O-C(O)-. Preferably this group is straight-chain and has 2 to 6 C atoms. It is accordingly preferably acetyloxy, propionyloxy, butyryloxy, pentanoyloxy, hexanoyloxy, acetyloxymethyl, propionyloxymethyl, butyryloxymethyl, pentanoyloxymethyl, 2-acetyloxyethyl, 2-propionyloxy-ethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxycarbonyl)ethyl, 2-(ethoxycarbonyl)ethyl, 2-(propoxycarbonyl)ethyl, 3-(methoxycarbonyl)propyl, 3-(ethoxycarbonyl)propyl, 4-(methoxycarbonyl)-butyl.

An alkyl group wherein two or more CH₂ groups are replaced by -O- and/or -C(O)O- can be straight-chain or branched. It is preferably straight-chain and has 3 to 12 C atoms. Accordingly it is preferably bis-carboxymethyl, 2,2-bis-carboxy-ethyl, 3,3-bis-carboxy-propyl, 4,4-bis-carboxybutyl, 5,5-bis-carboxy-pentyl, 6,6-bis-carboxy-hexyl, 7,7-bis-carboxyheptyl, 8,8-bis-carboxy-octyl, 9,9-bis-carboxy-nonyl, 10,10-bis-carboxydecyl, bis-(methoxycarbonyl)-methyl, 2,2-bis-(methoxycarbonyl)-ethyl, 3,3-bis-(methoxycarbonyl)-propyl, 4,4-bis-(methoxycarbonyl)-butyl, 5,5-bis-(methoxycarbonyl)-pentyl, 6,6-bis-(methoxycarbonyl)-hexyl, 7,7-bis-(methoxycarbonyl)-heptyl, 8,8-bis-(methoxycarbonyl)-octyl, bis-(ethoxycarbonyl)-methyl, 2,2-bis-(ethoxycarbonyl)-ethyl, 3,3-bis-(ethoxycarbonyl)-propyl, 4,4-bis-(ethoxycarbonyl)-butyl, 5,5-bis-(ethoxycarbonyl)-hexyl.

A thioalkyl group, i.e where one CH₂ group is replaced by -S-, is preferably straight-chain thiomethyl (-SCH₃), 1-thioethyl (-SCH₂CH₃), 1-thiopropyl (= -SCH₂CH₂CH₃), 1- (thiobutyl), 1-(thiopentyl), 1-(thiohexyl), 1-(thioheptyl), 1-(thiooctyl), 1-(thiononyl), 1-(thiodecyl), 1-(thioundecyl) or 1-(thiododecyl), wherein preferably the CH₂ group adjacent to the sp² hybridised vinyl carbon atom is replaced.

A fluoroalkyl group is preferably perfluoroalkyl CᵢF₂ᵢ₊₁, wherein i is an integer from 1 to 15, in particular CF₃, C₂F₅, C₃F₇, C₄F₉, C₅F₁₁, C₆F₁₃, C₇F₁₅ or C₈F₁₇, very preferably C₆F₁₃, or partially fluorinated alkyl, in particular 1,1-difluoroalkyl, all of which are straight-chain or branched.

Alkyl, alkoxy, alkenyl, oxaalkyl, thioalkyl, carbonyl and carbonyloxy groups can be achiral or chiral groups. Particularly preferred chiral groups are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy, 2-ethyl-hexoxy, 1-methylhexoxy, 2-octyloxy, 2-oxa-3-methylbutyl, 3-oxa-4-methyl-pentyl, 4-methylhexyl, 2-hexyl, 2-octyl, 2-nonyl, 2-decyl, 2-dodecyl, 6-meth-oxyoctoxy, 6-methyloctoxy, 6-methyloctanoyloxy, 5-methylheptyloxy-carbonyl, 2-methylbutyryloxy, 3-methylvaleroyloxy, 4-methylhexanoyloxy, 2-chloropropionyloxy, 2-chloro-3-methylbutyryloxy, 2-chloro-4-methyl-valeryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy.

Preferred achiral branched groups are isopropyl, isobutyl (=methylpropyl), isopentyl (=3-methylbutyl), tert. butyl, isopropoxy, 2-methyl-propoxy and 3-methylbutoxy.

In a preferred embodiment, R¹⁻⁴ are independently of each other selected from primary, secondary or tertiary alkyl or alkoxy with 1 to 30 C atoms, wherein one or more H atoms are optionally replaced by F, or aryl, aryloxy, heteroaryl or heteroaryloxy that is optionally alkylated or alkoxylated and has 4 to 30 ring atoms. Very preferred groups of this type are selected from the group consisting of the following formulae 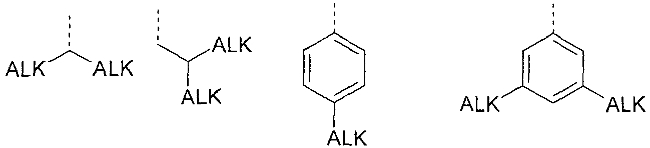 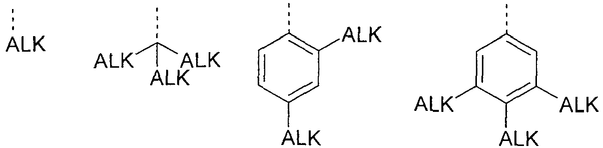 wherein "ALK" denotes optionally fluorinated, preferably linear, alkyl or alkoxy with 1 to 20, preferably 1 to 12 C-atoms, in case of tertiary groups very preferably 1 to 9 C atoms, and the dashed line denotes the link to the ring to which these groups are attached. Especially preferred among these groups are those wherein all ALK subgroups are identical. In a further preferred embodiment R¹⁻⁴ are cyclic alkyl or alkoxy.

A used herein, -CO-, -C(=O)- and -C(O)- will be understood to mean a carbonyl group, i.e. a group having the structure

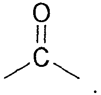

The polymers of the present invention are easy to synthesize and exhibit advantageous properties. They show high charge carrier mobility, good processability for the device manufacturing process, high solubility in organic solvents, and are especially well suited for large scale production using solution processing methods.

The synthesis of units of formula I, and of the functional derivatives, monomers, homopolymers and co-polymers based thereon, can be achieved based on methods that are known to the skilled person and described in the literature, as will be further illustrated herein..

Formula I encompasses both units having a symmetrical core structure of subformula IA (if m is 1), and units having an asymmetrical core structure of subformula IB (if m is 0), as shown below 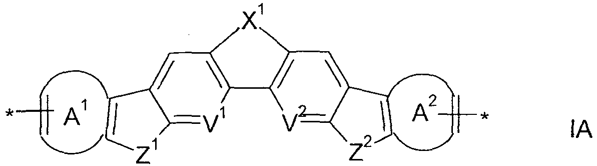 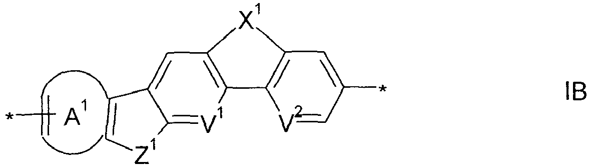 wherein X¹, A¹, A², V¹, V², Z¹ and Z² are as defined in formula I.

The units of formula IA have a symmetrical structure, wherein at least four aromatic rings are forced into a flat conjugated structure, and wherein the central bridge X¹ further increases the electron delocalization area. Both effects lead to a smaller bandgap and a more rigid-rod like structure, which enhances charge transport along the polymer backbone.

The units of structure IB are asymmetrical, which increases disorder and intramolecular transport. As 3-ring based units they also have a larger bandgap than a larger ring system and are thus especially well suited for application in blue OLEDs. Due to the asymmetric monomer structure, polymers comprising units of formula IB can have a high regioregularity which results in further improved charge transport properties. This can be achieved by using asymmetrically substituted monomers that contain a unit of formula IB with two different reactive groups, e.g. a boronate group and a halide group, and by preparing polymers from such monomers by known aryl-aryl coupling methods like Suzuki coupling etc. Further details about suitable and preferred synthesis and polymerisation methods are disclosed below.

The groups *-A¹ and A²-* in formula I and its subformulae are selected from the group consisting of the following subformulae and their mirror images: 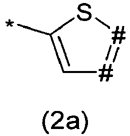 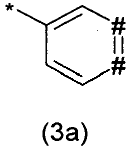 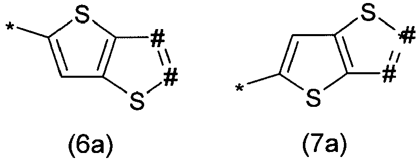 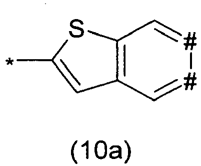 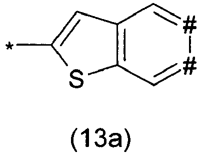 wherein the individual aromatic and heteroaromatic rings can also be substituted by one or more groups R¹.

Highly preferred structures according to formula I are thus: 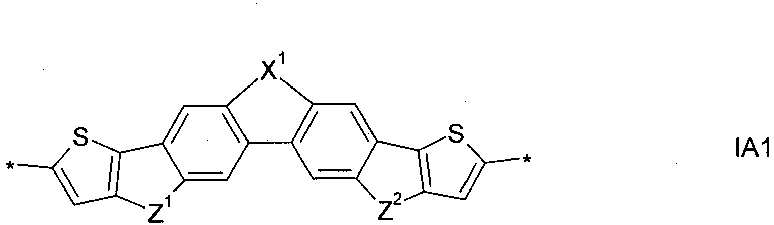 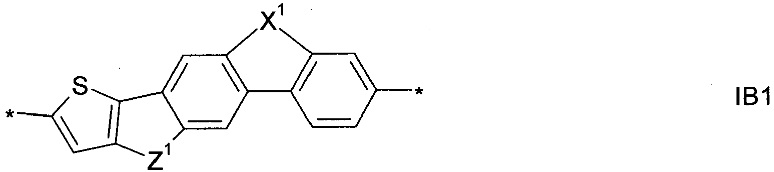 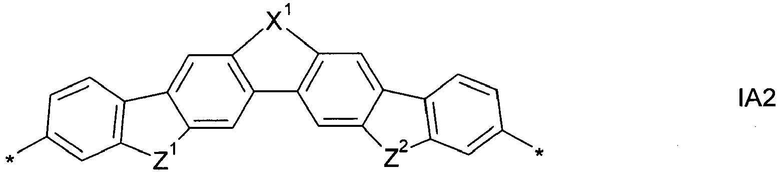 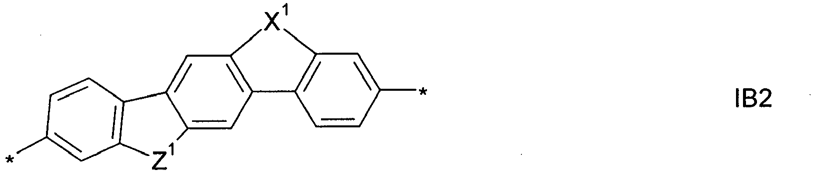 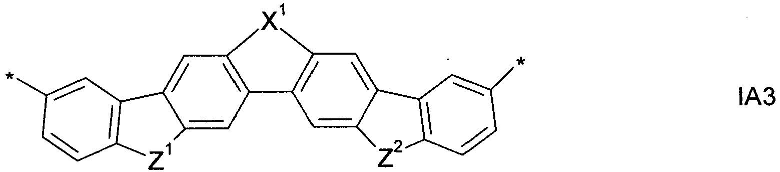 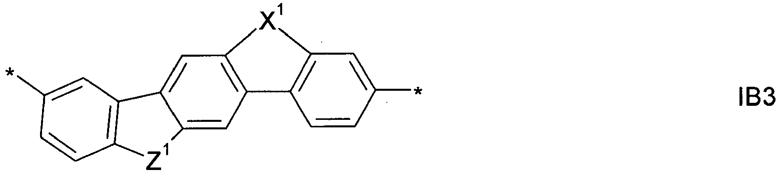 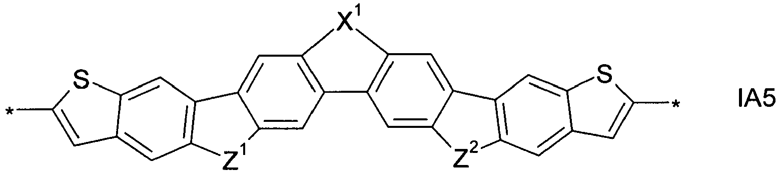 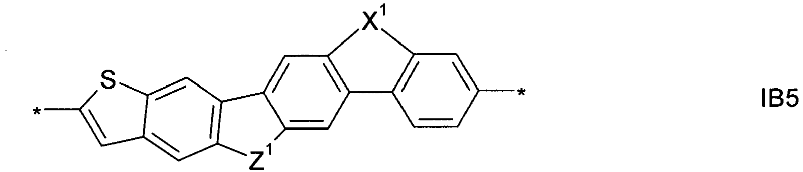 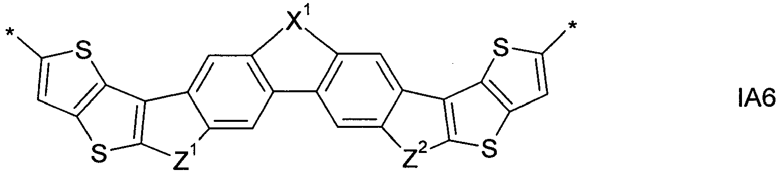 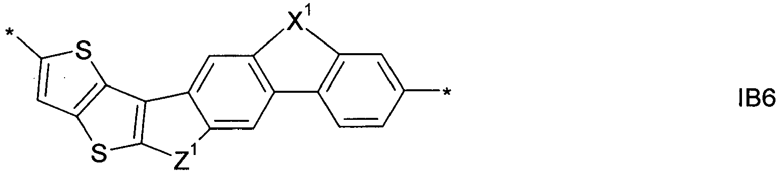 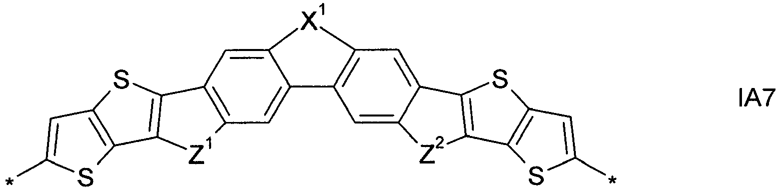 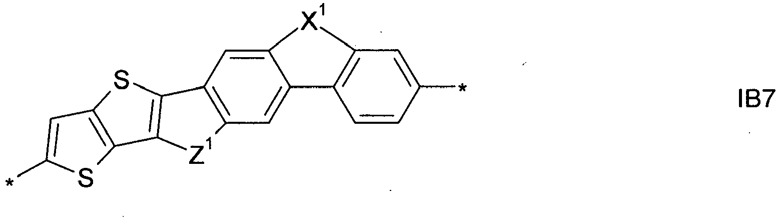 wherein X¹, Z¹ and Z² are as defined in formula I.

In formula I and its subformulae,the bridge X¹ is preferably a phenanthrene-derived bridge or C(O)-O, leading to preferred structures of subformulae IA1a through IB1c as shown below, which are based on formula IA1 or IB1, respectively. The systematic can be applied to all substructures of formula I. In a very preferred embodiment X¹ is CR¹=CR² or CR¹R²-CR¹R², respectively, as in formulae IA1a through IB1b below. 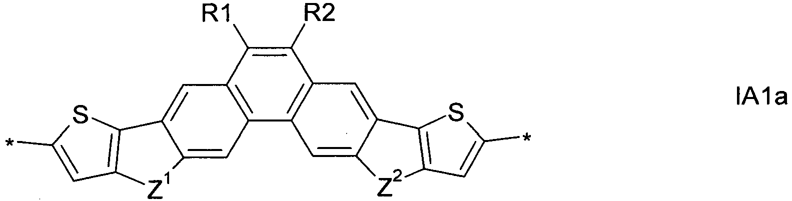 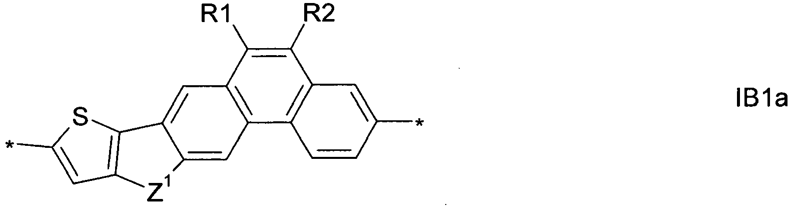 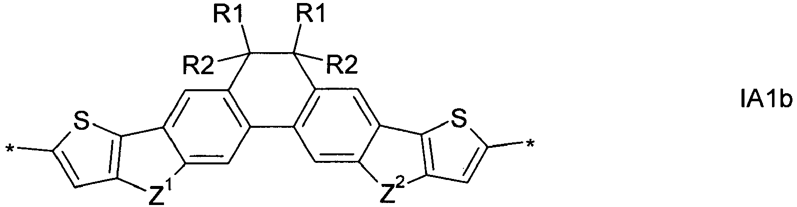 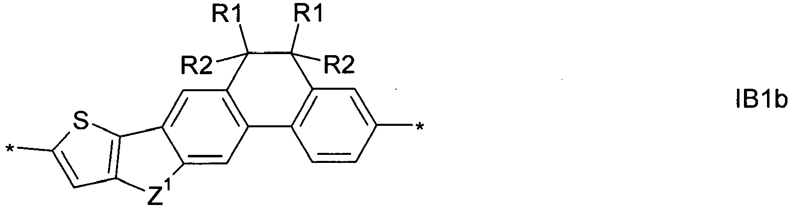 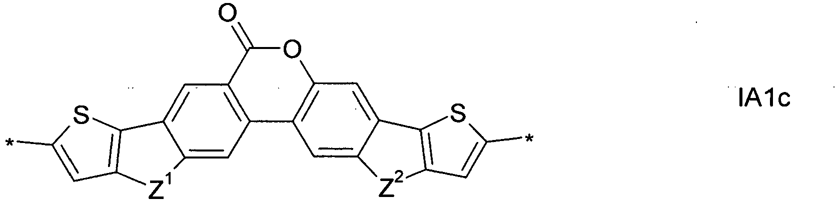 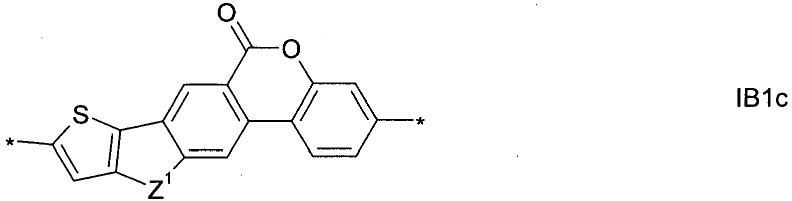 wherein R¹, R², Z¹ and Z² are as defined in formula I.

In the units of formula I and its subformulae, Z¹ and Z² denote CR³R⁴, S or O.

This leads to the following very preferred structures of the following subformulae: 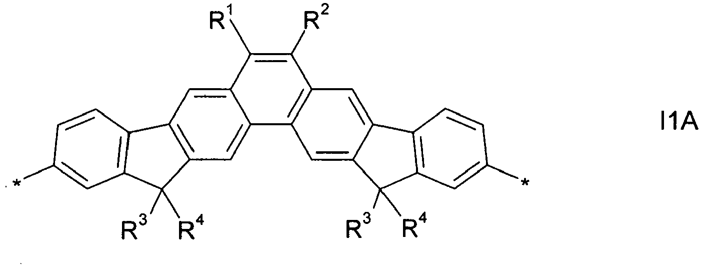 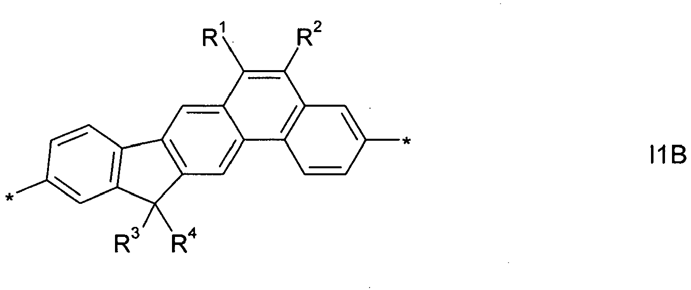 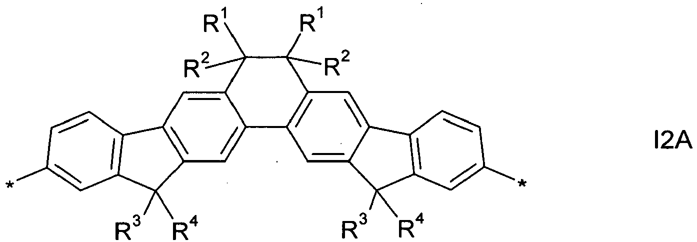 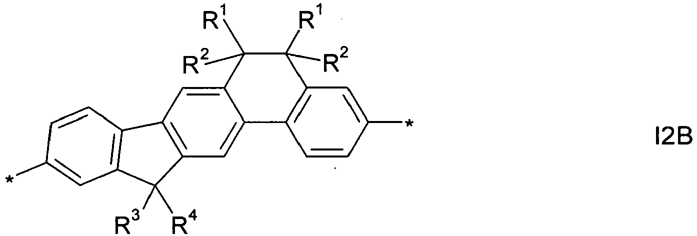 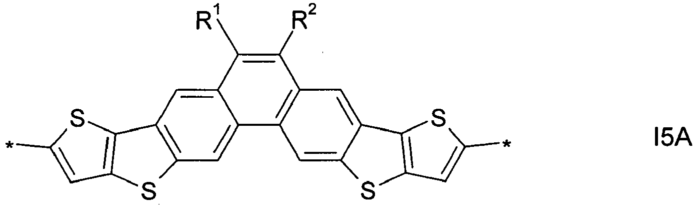 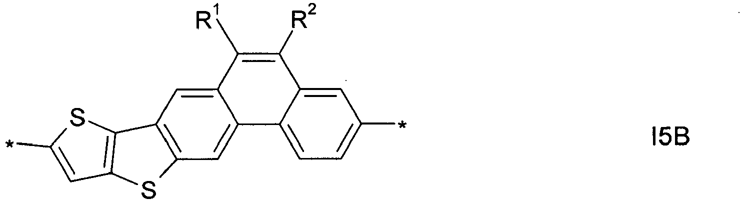 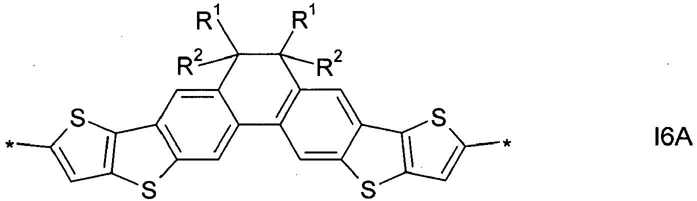 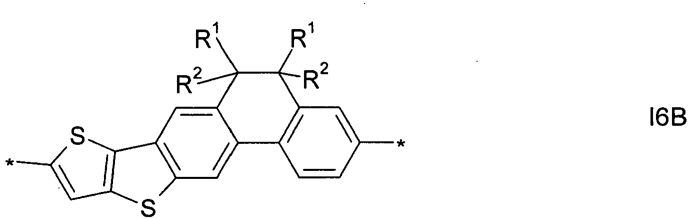 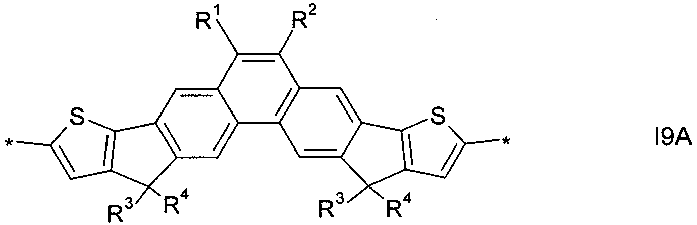 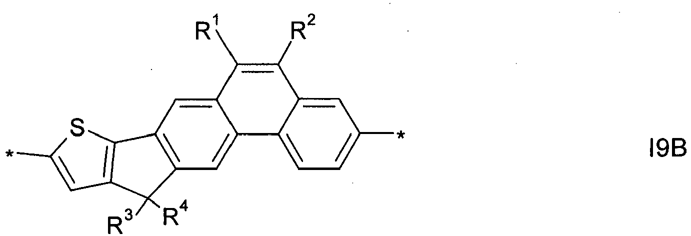 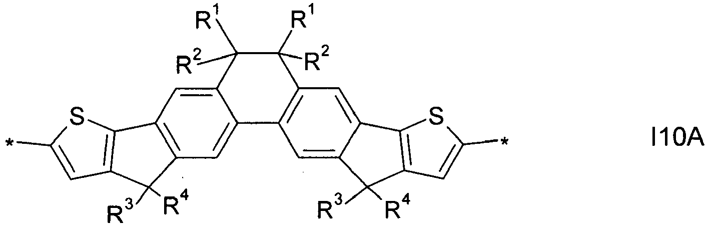 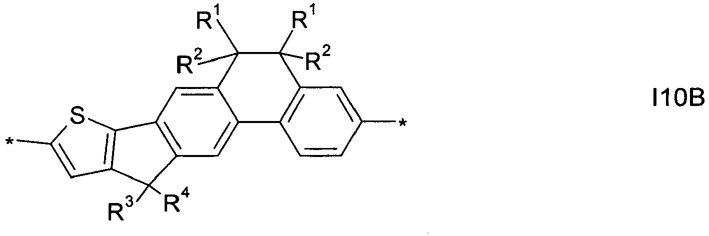 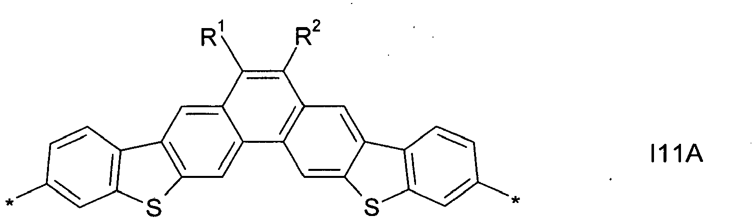 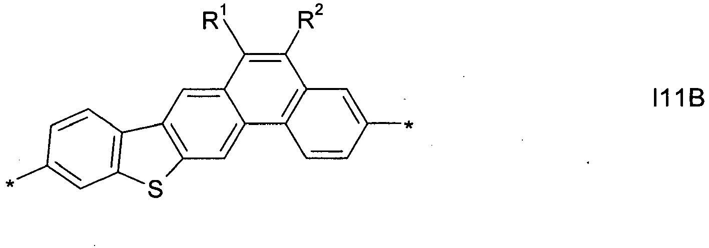 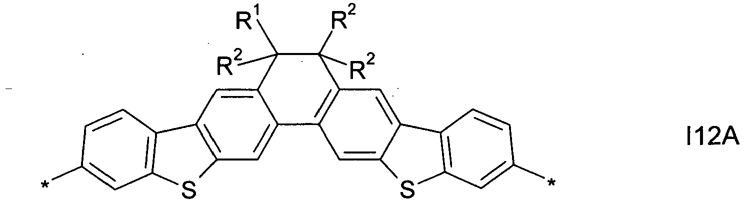 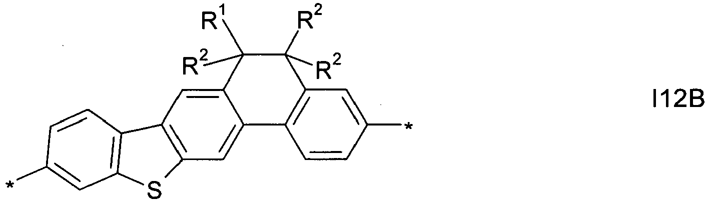 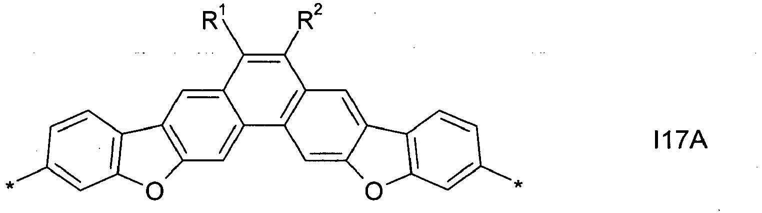 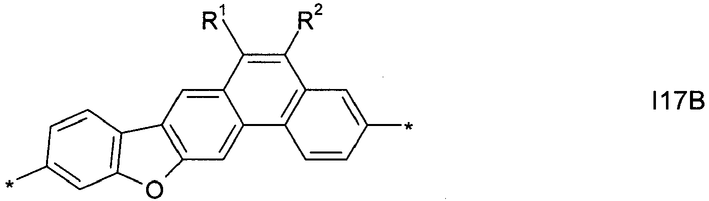 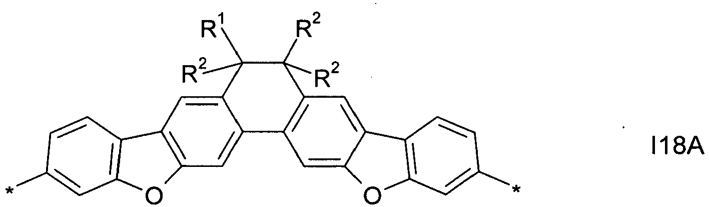 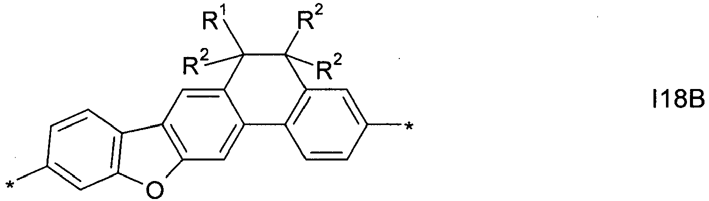 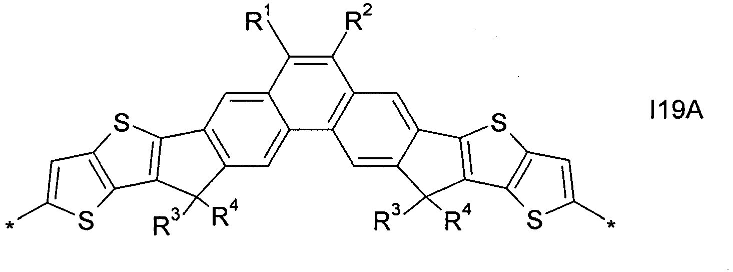 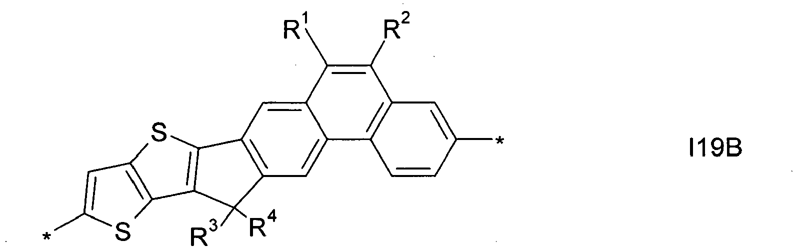 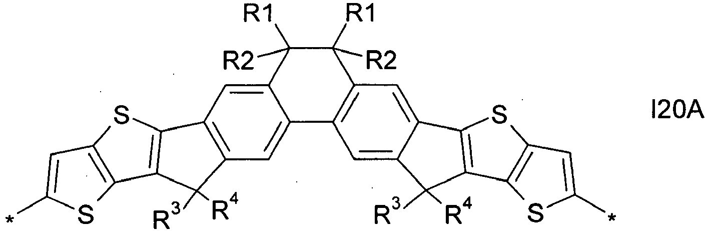 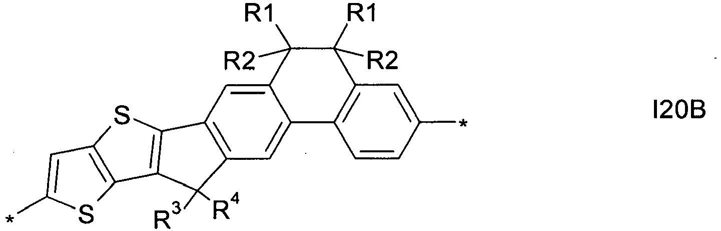 wherein R^{1,2,3,4} have the meanings given in formula I or the preferred meanings as disclosed above and below, and wherein the outer benzene rings can also be substituted by one or two groups R¹.

In the units of formula I and its subformulae, R¹⁻⁴ are independently of each other selected from straight-chain, branched or cyclic alkyl or alkoxy with 1 to 30 C atoms which is unsubstituted or substituted by one or more F atoms, or aryl or heteroaryl each of which is unsubstituted or substituted and has 4 to 30, ring atoms.

If one of R¹⁻⁴ denotes substituted aryl or heteroaryl, it is preferably substituted by one or more groups L, wherein L is selected from F, -CN, - NO₂, NR³, -NCO, -NCS, -OCN, -SCN, -C(=O)NR⁰R⁰⁰, -C(=O)X⁰, C(=O)H,CR⁰=CR⁰R⁰⁰, -C(=O)R⁰, -NR⁰R⁰⁰, optionally substituted aryl or heteroaryl having 4 to 20 ring atoms, or straight chain, branched or cyclic alkyl with 1 to 20, preferably 1 to 12 C atoms wherein one or more non-adjacent CH₂ groups are optionally replaced, in each case independently from one another, by -O-, -S-, -NR⁰-, -SiR⁰R⁰⁰-, -C(=O)-, -C(=O)O-,-CY¹=CY²- or -C≡C- in such a manner that O and/or S atoms are not linked directly to one another and which is unsubstituted or substituted with one or more F, X⁰ is halogen, preferably F, and Y1, Y², R⁰ and R⁰⁰ have the meanings given above and below. It can also be preferred to have siloxane-, perfluoroalkyl- or a combination of both as side-chains in order to modify the rheological properties of the polymers.

In another preferred embodiment, R¹ and R² denote straight-chain, branched or cyclic alkyl or alkoxy with 1 to 30 C atoms which is optionally fluorinated, and R³ and R⁴ denote aryl, aryloxy, heteroaryl or heteroaryloxy, each having from 4 to 30 ring atoms and optionally being fluorinated, alkylated,fluoroalkylated or alkoxylated.

The compounds according to the present invention include monomers and polymers.

Polymers in accordance with the present invention include homopolymers and co-polymers, like statistical or random co-polymers, alternating co-polymers and block co-polymers, as well as combinations thereof.

In a preferred embodiment the polymer comprises, in addition to the units of formula I or its subformulae, one or more co-units having one or more of semiconducting, charge transport, hole or electron transport, hole or electron blocking, electrically conducting, photoconducting, charge generation or light emitting properties.

A preferred embodiment relates to co-polymers, in particular for use in OLED devices, that preferably comprise, in addition to the units of formula I or its subformulae, one or more structurally different co-units which are selected from the following groups 1-8. These units are disclosed and listed extensively in WO 02/077060 A1 and in WO 2005/014689 A2.
- group 1:: units which influence the hole-injection and/or hole-transport properties of the polymers;
- group 2:: units which influence the electron-injection and/or electron-transport properties of the polymers;
- group 3:: units which have combinations of individual units from group 1 and group 2;
- group 4:: units which modify the emission characteristics to such an extent that electrophosphorescence can be obtained instead of electrofluorescence;
- group 5:: units which improve transfer from the singlet state to the triplet state;
- group 6:: units which influence the emission colour of the resultant polymers;
- group 7:: units which are typically used as polymer backbone;
- group 8:: units which influence the film morphology and/or rheology of the resultant polymers.

Structural units from group 1 which have hole-injection and/or hole-transport properties are, for example, triarylamine, benzidine, tetraaryl-para-phenylenediamine, triarylphosphine, phenothiazine, phenoxazine, dihydrophenazine, thianthrene, dibenzo-para-dioxin, phenoxathiyne, carbazole, azulene, thiophene, pyrrole and furan derivatives and further O-, S- or N-containing heterocycles having a high HOMO (HOMO = highest occupied molecular orbital). These arylamines and heterocycles preferably result in an HOMO in the polymer of greater than -5.8 eV (against vacuum level), particularly preferably greater than -5.5 eV.

Structural units from group 2 which have electron-injection and/or electron-transport properties are, for example, pyridine, pyrimidine, pyridazine, pyrazine, oxadiazole, quinoline, quinoxaline, anthracene, benzanthracene, pyrene, perylene, benzimidazole, triazine, ketone, phosphine oxide and phenazine derivatives, but also triarylboranes and further O-, S- or N-containing heterocycles having a low LUMO (LUMO = lowest unoccupied molecular orbital). These units in the polymer preferably result in an LUMO of less than -1.5 eV (against vacuum level), particularly preferably less than -2.0 eV.

It may be preferred for the polymers according to the invention to comprise units from group 3 in which structures which increase the hole mobility and structures which increase the electron mobility (i.e. units from groups 1 and 2) are bonded directly to one another or structures which increase both the hole mobility and the electron mobility. Some of these units can serve as emitters and shift the emission colour into the green, yellow or red. Their use is thus suitable, for example, for the generation of other emission colours from originally blue-emitting polymers.

Structural units from group 4, so-called triplet emitter units, are those which are able to emit light from the triplet state with high efficiency, even at room temperature, i.e. exhibit electrophosphorescence instead of electrofluorescence, which frequently causes an increase in the energy efficiency. A triplet emitter unit in the sense of the present invention is taken to mean a compound which comprises a triplet emitter. Triplet emitter in the sense of the present invention is taken to mean all compounds which are capable of emitting light in the visible or NIR region by transfer from a triplet state into an energetically lower state. The term phosphorescence is also used here. Suitable for this purpose are firstly compounds which contain heavy atoms having an atomic number of greater than 36. Preference is given to compounds which contain d- or f-transition metals which satisfy the above-mentioned condition. Particular preference is given here to corresponding structural units which contain elements from groups 8 to 10 of the Periodic Table (Ru, Os, Rh, Ir, Pd, Pt). Suitable structural units for the polymers according to the invention here are, for example, various complexes, as described, for example, in WO 02/068435 A1, WO 02/081488 A1 and EP 1239526 A2. Corresponding monomers are described in WO 02/068435 A1 and in WO 2005/042548 A1. It is preferred in accordance with the invention to employ triplet emitters which emit in the visible spectral region (red, green or blue). The triplet emitter may be part of the backbone of the polymer (i.e. in the main chain of the polymer) or it may be located in a side chain of the polymer.

Structural units from group 5 are those which improve transfer from the singlet state to the triplet state and which, employed in support of the above-mentioned triplet emitter units, improve the phosphorescence properties of these structural elements. Suitable for this purpose are, in particular, carbazole and bridged carbazole dimer units, as described, for example, in WO 2004/070772 A2 and WO 2004/113468 A1. Also suitable for this purpose are ketones, phosphine oxides, sulfoxides, sulfones, silane derivatives and similar compounds, as described, for example, in WO 2005/040302 A1.

Structural units from group 6, besides those mentioned above, are those which have at least one further aromatic structure or another conjugated structure which does not fall under the above-mentioned groups, i.e. which have only little influence on the charge-carrier mobilities, are not organometallic complexes or do not influence singlet-triplet transfer. Structural elements of this type can influence the emission colour of the resultant polymers. Depending on the unit, they can therefore also be employed as emitters. Preference is given here to aromatic structures having 6 to 40 C atoms and also tolan, stilbene or bisstyrylarylene derivatives, each of which may be substituted by one or more radicals R³. Particular preference is given here to the incorporation of 1,4-phenylene, 1,4-naphthylene, 1,4- or 9,10-anthrylene, 1,6-, 2,7- or 4,9-pyrenylene, 3,9- or 3,10-perylenylene, 4,4'-biphenylylene, 4,4"-terphenylylene, 4,4'-bi-1,1'-naphthylylene, 4,4'-tolanylene, 4,4'-stilbenylene, 4,4"-bisstyrylarylene, benzothiadiazole and corresponding oxygen derivatives, quinoxaline, phenothiazine, phenoxazine, dihydrophenazine, bis(thiophenyl)arylene, oligo(thiophenylene), phenazine, rubrene, pentacene or perylene derivatives, which are preferably substituted, or preferably conjugated push-pull systems (systems which are substituted by donor and acceptor substituents) or systems such as squarines or quinacridones, which are preferably substituted. Further examples of units that are suitable as emitters can be found in WO03/020790.

Structural units from group 7 are units which contain aromatic structures having 6 to 40 C atoms, which are typically used as polymer backbone. These are, for example, 4,5-dihydropyrene derivatives, 4,5,9,10-tetra-hydropyrene derivatives, fluorene derivatives, 9,9'-spirobifluorene derivatives, phenanthrene derivatives, 9,10-dihydrophenanthrene derivatives, 5,7-dihydrodibenzoxepine derivatives and cis- and trans-indenofluorene derivatives, but basically also all similar structures which would, after polymerisation, result in a conjugated, bridged or unbridged polyphenylene or polyphenylene-vinylene homopolymer. Here too, the said aromatic structure may contain heteroatoms, such as O, S or N, in the backbone or a side chain. For some applications, especially electrophosphorescent polymers, it can be useful that the conjugation is intentially disrupted with conjugation breaking co-monomers. These can make up a substantial number of monomer units in the polymer backbone. Suitable co-monomers of that category are found in WO2006/061181, WO2010/136110 and WO2010/136111.

Structural units from group 8 are those which influence the film morphology and/or rheology of the polymers, such as, for example, siloxanes, long alkyl chains or fluorinated groups, but also particularly rigid or flexible units, such as, for example, liquid crystal-forming units or crosslinkable groups.

Preferred polymers according to the invention are those in which at least one structural unit has charge-transport properties, i.e. polymers which comprise, inter alia, at least one unit selected from groups 1 and 2.

Preferred compounds according to the invention are polymers which, besides structural units of the formula (I), at the same time additionally comprise one or more units selected from groups 1 to 8. It may furthermore be preferred for more than one structural unit from one group to be present at the same time.
It is likewise preferred for the polymers according to the invention to comprise units which improve charge transport and/or charge injection, i.e. units from group 1 and/or 2; a proportion of 0.5 to 50 mol% of these units is particularly preferred; a proportion of 1 to 15 mol% of these units is very particularly preferred.
It is furthermore particularly preferred for the polymers according to the invention to comprise structural units from group 7 and units from group 1 and/or 2. It is particularly preferred for the sum of structural units of the formula (I), of units from group 7 and units from group 1 and/or 2 of the polymer to be at least 50 mol%, based on all units of the polymer, where 0.5 to 30 mol% are preferably units from group 1 and/or 2.
The way in which the above-mentioned copolymers having block-like structures can be obtained and which further structural elements are particularly preferred for this purpose is described in detail, for example, in WO 2005/014688 A2. It should likewise be emphasised at this point that the polymer may also have dendritic structures. The synthesis of the above-described units from groups 1 to 8 and of the further emitting units is known to the person skilled in the art and is described in the literature, for example in WO 2005/014689 A2, WO 2005/030827 A1 and WO 2005/030828 A1.

For use in OFET devices, the polymers according to the present invention preferably comprise, in addition to the units of formula I or its subformulae, one or more co-units selected from known conjugated backbone moieties including but not limited to fluorene, spirobifluorene, phenanthrene, indenofluorene, or phenyl-, naphthyl- or anthracene units all of which are optionally substituted by one or more groups R¹ through R⁴ as defined above and below. These co-units essentially lead to polyphenylene polymers with different conjugation lengths, molecular geometry, film forming properties, bandgap and disorder in the film. By chosing the right co-backbone-monomer, the polymer can be tailored for the intended application.

For use in OPV devices, the polymers according to the present invention preferably comprise, in addition to the units of formula I or its subformulae, one or more co-units having sensitizing properties. Alternatively, the conjugated polymers may be used in mixture or blend comprising one or more small molecules as sensitizers.

Another preferred embodiment relates to polymers, in particular for use in OFET, OPV or OPD devices, which comprise, in addition to the units of formula I or its subformulae, one or more co-units selected from the group consisting of formulae D1-D114 and /or A1-A68 as shown below.

Preferably the proportion of the units of formula I and its subformulae in the conjugated part of a co-polymer in accordance with the present invention is at least 50 mol%. For use in OFETs, for example, a higher contents of at least 60%, especially at least 75% are further preferred. For this application it can also be preferred that the monomer containing formula I or one of its subformulae is extended by further co-units prior to polymerization as shown below. In this case, the contents of at least 50%, more preferably 60% and especially preferred 75% refers to the amount of monomer used in the polymerization.

In another preferred embodiment the conjugated polymer in accordance with the present invention is a block co-polymer comprising at least one block having charge transport properties, wherein the proportion of units of formula I or its subformulae in said block is at least 50%.

Block co-polymers can also be made in accordance with DE 10337077. In this case the polymers according to this invention contain at least one unit according to formula I or its subformulae in one block of the polymer in combination with units from the aforementioned groups 1 through 8 or the formulae D1-D114 and/or A1-A68 as shown below.

Compounds and polymers containing an extended version of formula I are therefore also a subject-matter of this invention. These polymers have the following general formula II:

*-[(Ar¹)ₐ-(U)_{b}-(Ar²)_{c}-(Ar³)_{d}]ₙ-* II

wherein
- U: is a unit of formula I, IA, IB or their subformulae,
- Ar¹, Ar², Ar³: on each occurrence identical or different, and independently of each other, are selected from one of the aforementioned groups 1 through 8, or denote aryl or heteroaryl that is different from U, preferably has 5 to 30 ring atoms, and is optionally substituted by one or more groups R¹ through R⁴, and is preferably seelcted from the units D1-D114 and A1-A68 as shown below,
- a, c, d: are on each occurrence identical or different 0 or an integer from 1 to 10,
- b: is 0 or an integer from 1 to 10, preferably an integer from 1 to 10,
- n: is an integer >1,
wherein the polymer comprises at least one repeating unit [(Ar¹)ₐ-(U)_{b}-(Ar²)_{c}-(Ar³)_{d}] wherein b is at least 1,
and wherein the *indicates the bonds that link the unit either with a polymer or functional groups.

Preferred polymers according to the present invention have the following formula III

R⁵-[(Ar¹)ₐ-(U)_{b}-(Ar²)_{c}-(Ar³)_{d}]ₙ-R⁶ III

wherein Ar¹, Ar², Ar³, U, a, b, c, d and n are as defined in formula II, R⁵ and R⁶ denote independently of each other, H, F, Br, Cl, I, -CH₂Cl, -CHO,-CR'=CR"₂, -SiR'R"R''', -SiR'X'X", -SiR'R"X', -SnR'R"R''', -BR'R",-B(OR')(OR"), -B(OH)₂, -O-SO₂-R', -C≡CH, -OC≡SiR'₃, -ZnX' or an endcap group, X' and X" denote halogen, R', R" and R''' have independently of each other one of the meanings of R⁰ given in formula I, and two of R', R" and R''' may also form a ring together with the hetero atom to which they are attached.

Preferred endcap groups R⁵ and R⁶ are H, C₁₋₂₀ alkyl, or optionally substituted C₆₋₁₂ aryl or C₂₋₁₀ heteroaryl, very preferably H or phenyl.

Small molecule compounds and oligomers according to the present invention can be made by endcapping the units according to formula IA or IB or its subformulae, or formula II wherein n is for example an integer form 2 to 10, respectively, with endcap groups. The preferred endcap groups (above and below also referred to as "endcappers") in this case are substituted or unsubstituted phenyl, biphenyl, or straight or branched alkyl.

If the small molecules are, as it is preferred, to be processed from solution, solubility enhancing groups as shown in EP2010/005648 are most preferred. These units have no further optoelectronic function. It is, however, possible to use monofunctional molecules selected from groups 1 through 8, and/or from formulae D1-D114 or A1-A68 below, and attach them to units according to formula IA, IB or II to obtain functional small molecules. These small molecules are also a subject of this invention.

The invention further relates to monomers of formula IV

R⁷-(Ar¹)ₐ-U-(Ar²)_{c}-R⁸ IV

wherein U, Ar¹, Ar², a and c have the meanings of formula II, or one of the preferred meanings as described above and below, and R⁷ and R⁸ are, preferably independently of each other, selected from the group consisting of Cl, Br, I, O-tosylate, O-triflate, O-mesylate, O-nonaflate, -SiMe₂F,-SiMeF₂, -O-SO₂Z¹, -B(OZ²)₂, -CZ³=C(Z³)₂, -C≡CH, - C≡CSi(Z¹)₃, -ZnX⁰ and -Sn(Z⁴)₃, wherein X⁰ is halogen, preferably Cl, Br or I, Z¹⁻⁴ are selected from the group consisting of alkyl and aryl, each being optionally substituted, and two groups Z² may also together form a cyclic group.

The advantage of polymers or compounds made from a preformed subunit is that the sequence of repeat units is predetermined by the choice and sequence of the units U and Ar¹-Ar³ in formula II. Regioregular co-polymers can be obtained this way with obvious advanges in their transport behaviour along the chain and morphological advantages in film formation (lower spatial and energetic disorder). Also, functionalities that interfere with each other, like for example electron donor moieties and electron acceptor moieties, can be separated in order to keep charges from being trapped, or put next to each other in order to facilitate charge generation. Polymers and smaller compounds with these properties are thus a very preferred subject of this invention.

Examples for donor moieties that can be used together with the units of formula I are listed below. These, as well as the aforementioned monomers according to group 1 through 8, are especially preferred co-monomers in combination with formula I, IA or IB, or for extended monomer units according to formula II and III (i.e. Ar¹ through Ar³). 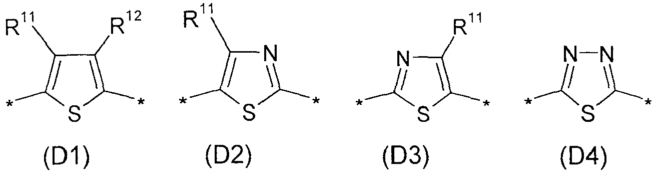 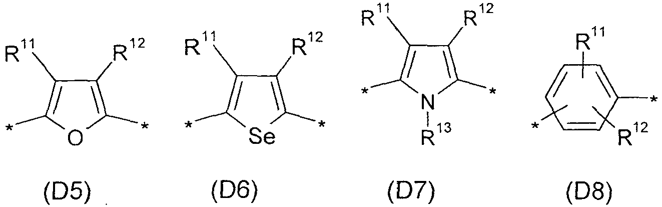 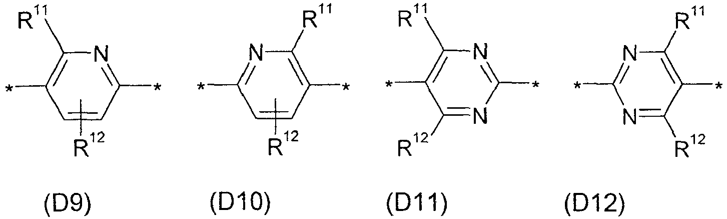 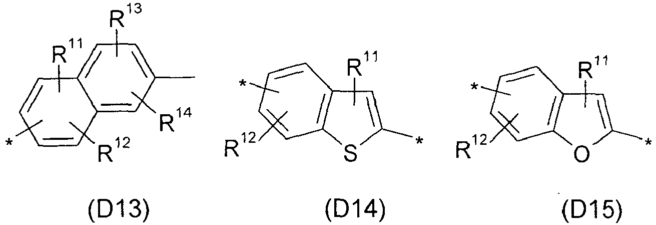 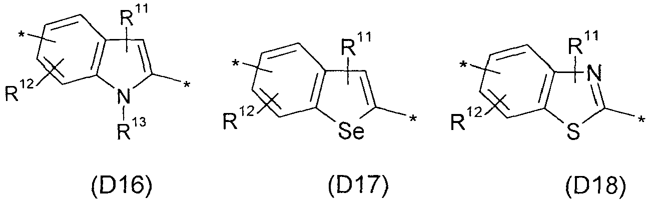 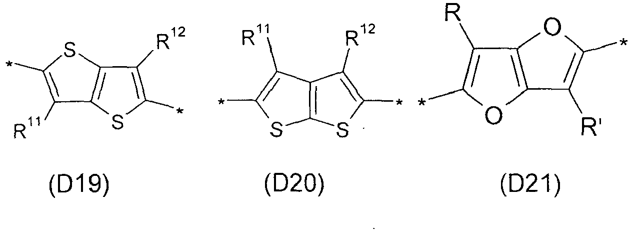 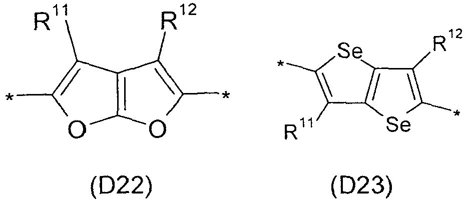 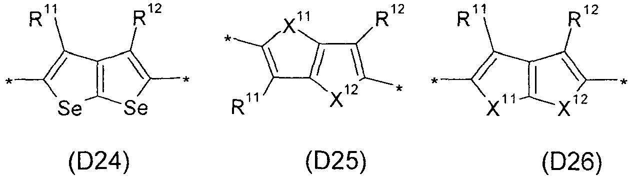 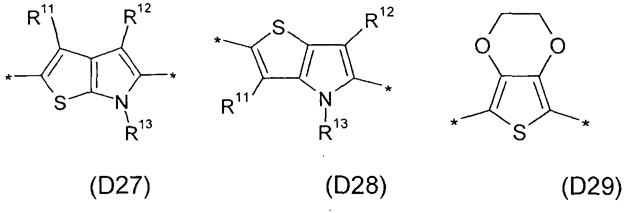 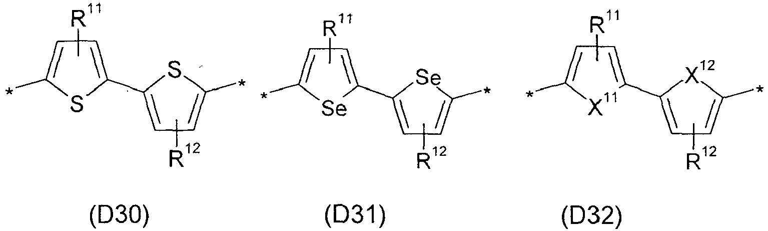 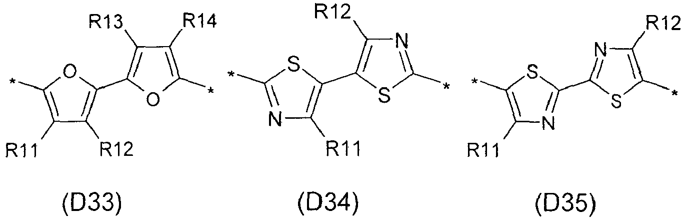 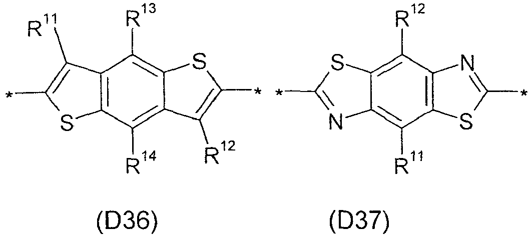 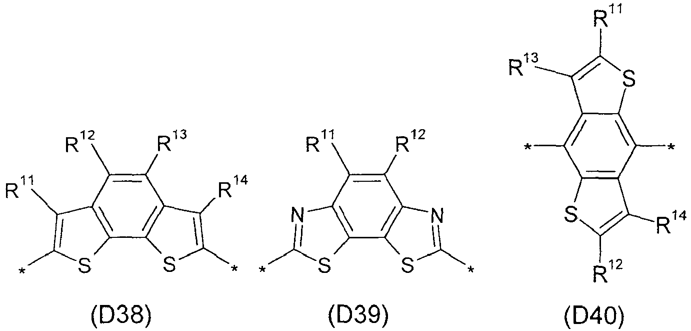 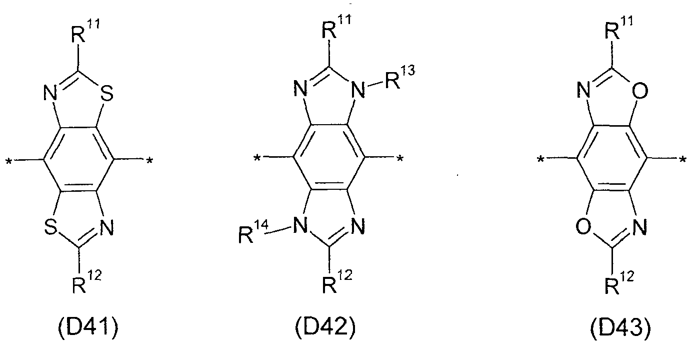 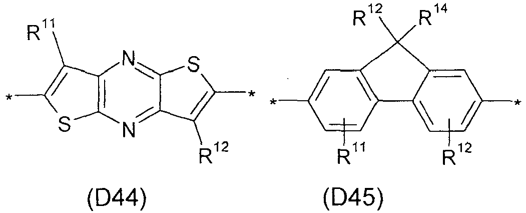 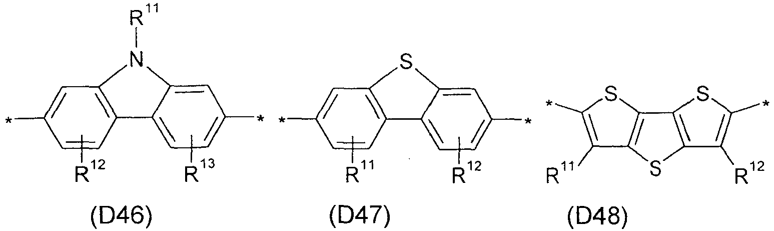 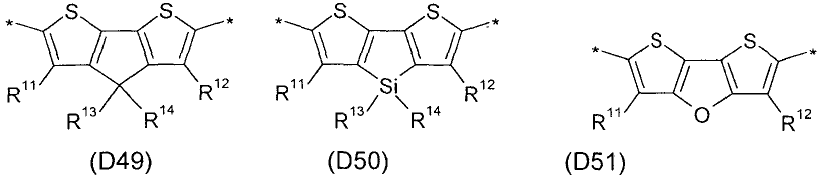 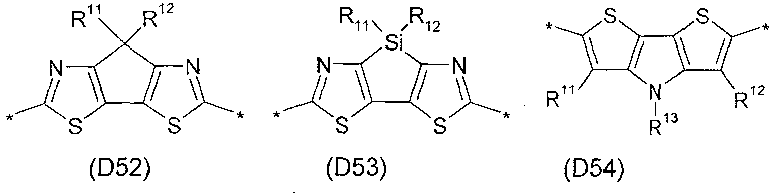 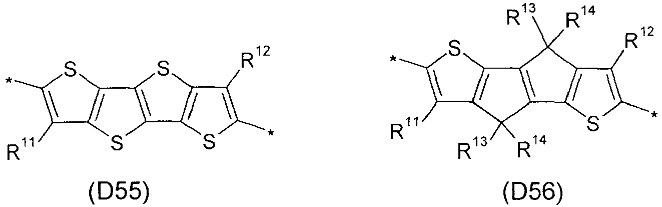 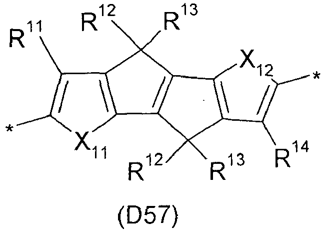 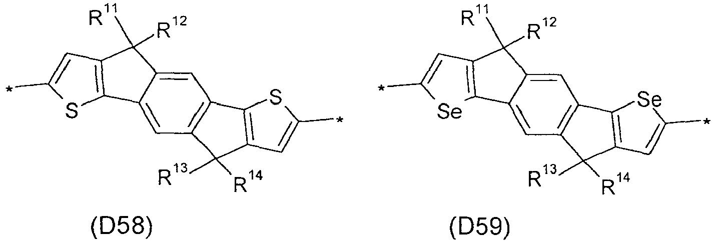 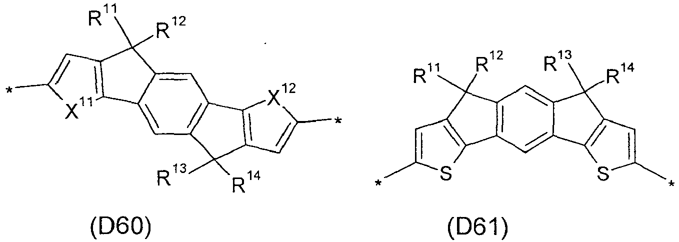 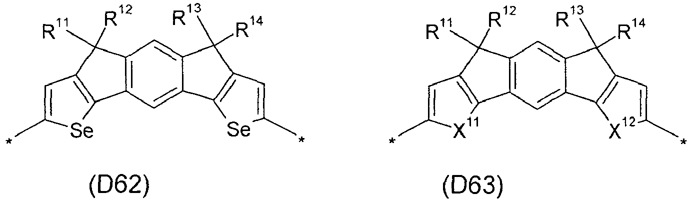 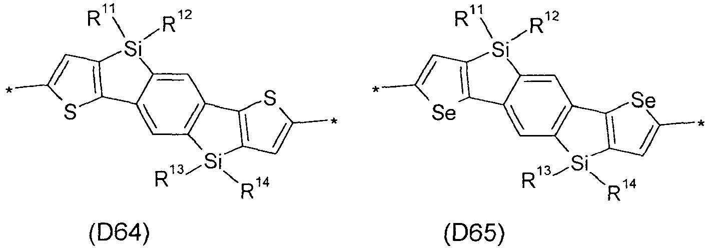 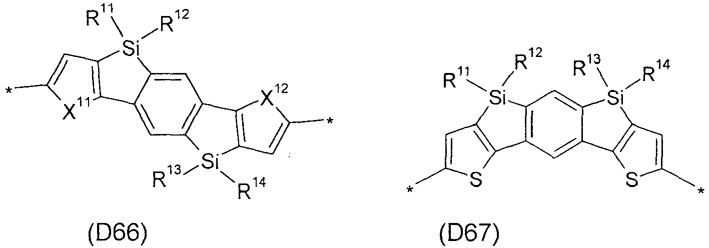 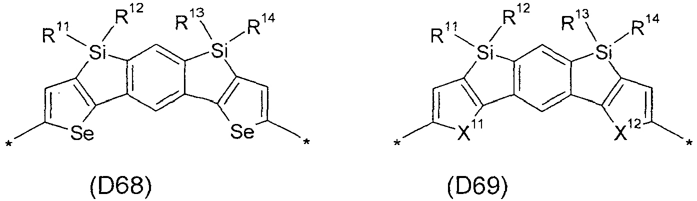 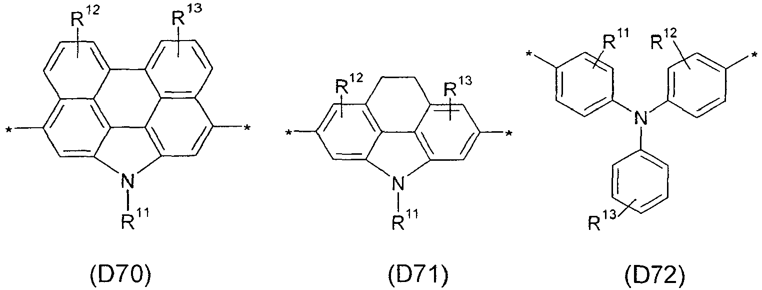 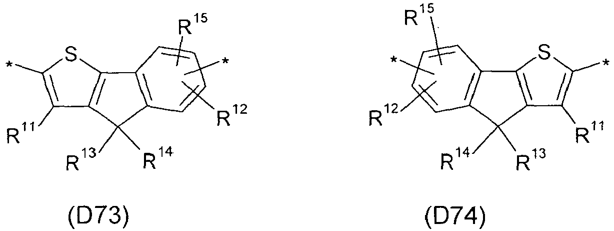 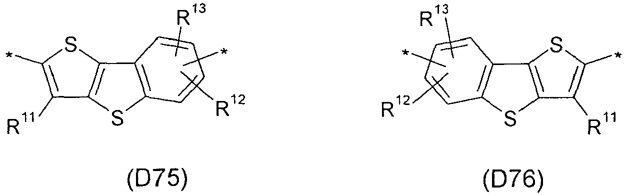 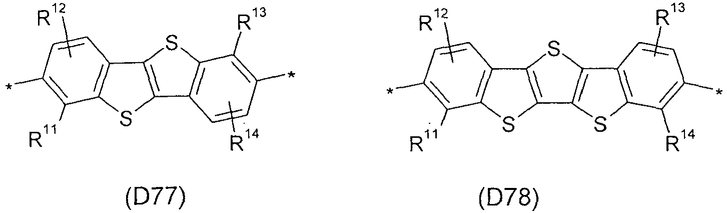 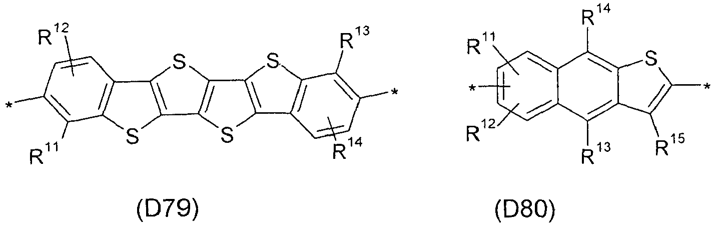 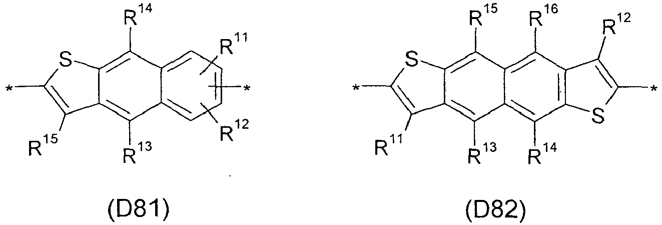 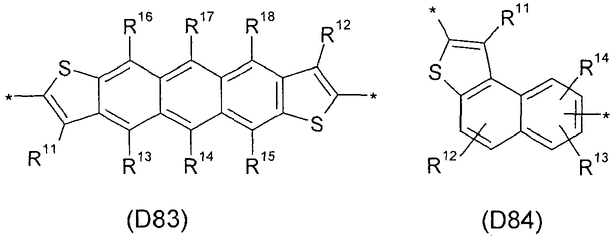 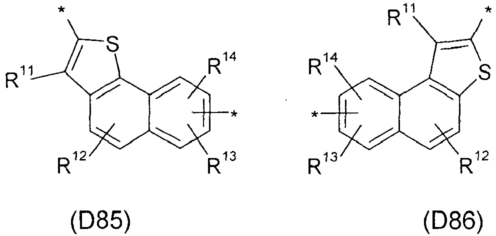 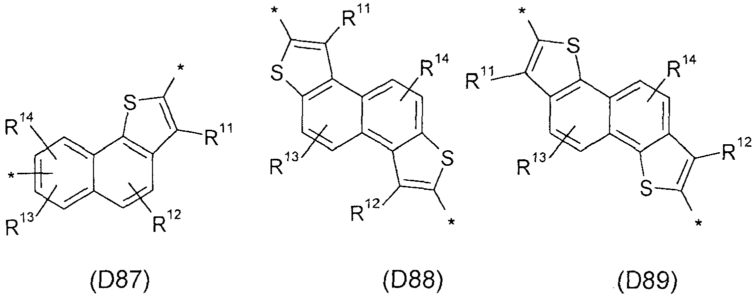 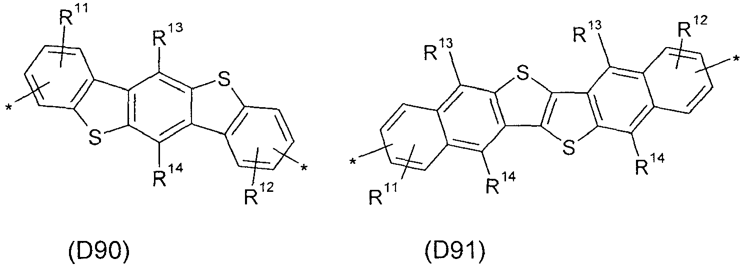 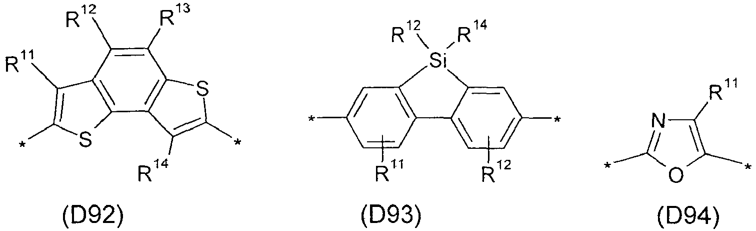 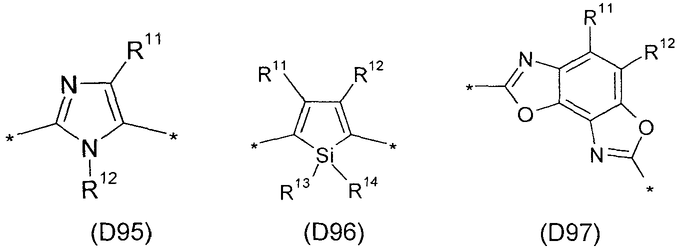 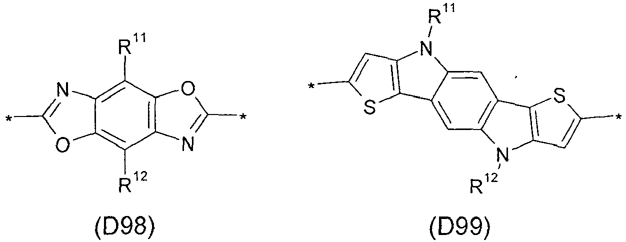 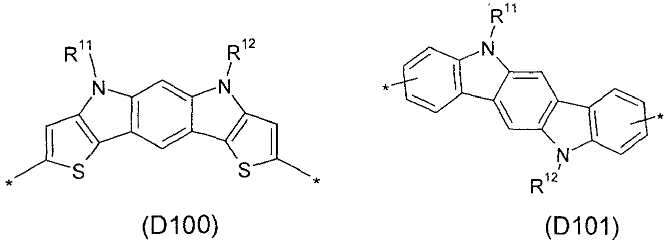 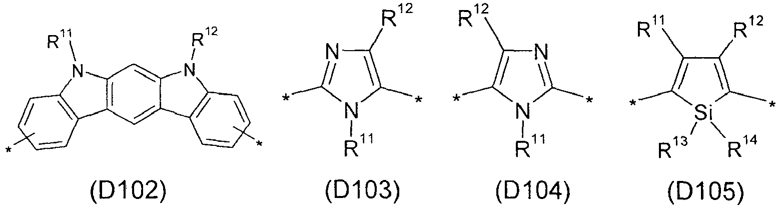 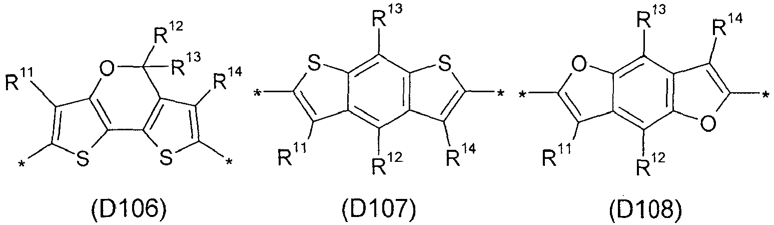 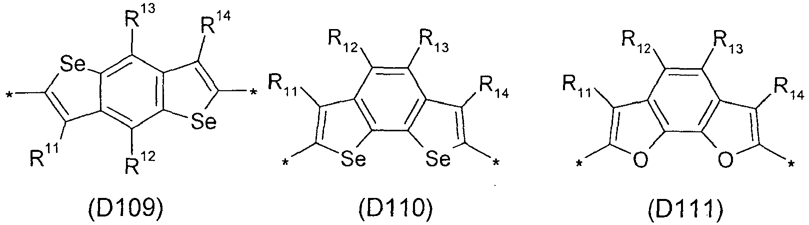 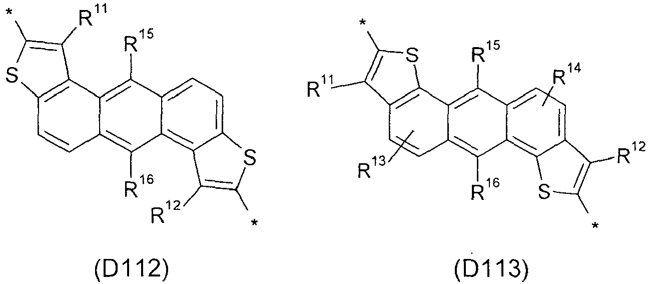 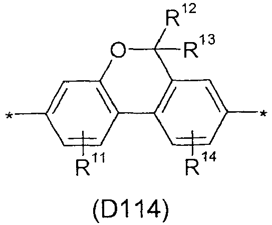 wherein one of X¹¹ and X¹² is S and the other is Se, and R¹¹, R¹², R¹³, R¹⁴, R¹⁵, R¹⁶ independently of each other denote H or have one of the meanings of R¹ to R⁴ as defined above and below.

Examples for acceptor moieties that can be used together with the units of formula I are listed below. These, as well as the aforementioned monomers according to group 1 through 8 and the aforementjoend donor moieties of formula D1-D114, are especially preferred co-monomers in combination with formula I, IA or IB, or for extended monomer units according to formula II and III (i.e. Ar¹ through Ar³). 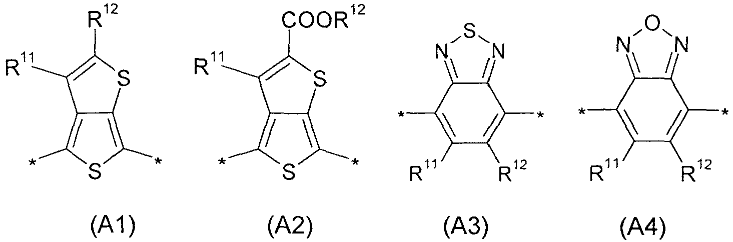 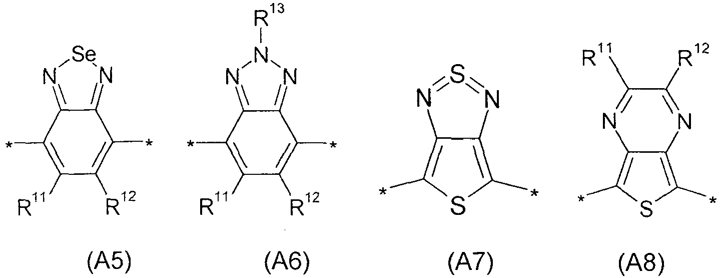 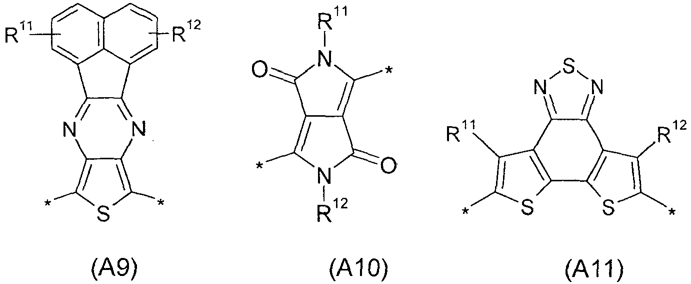 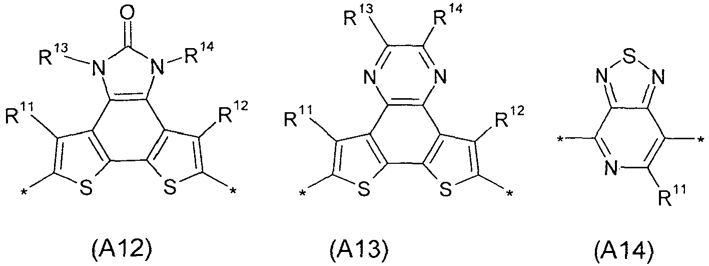 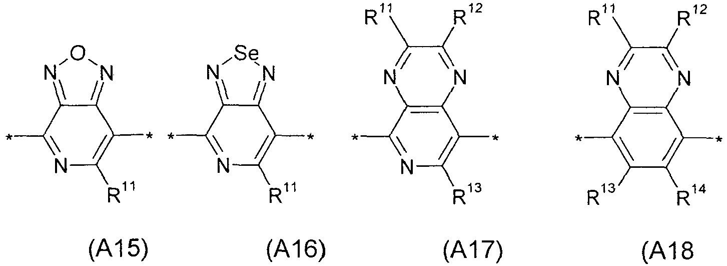 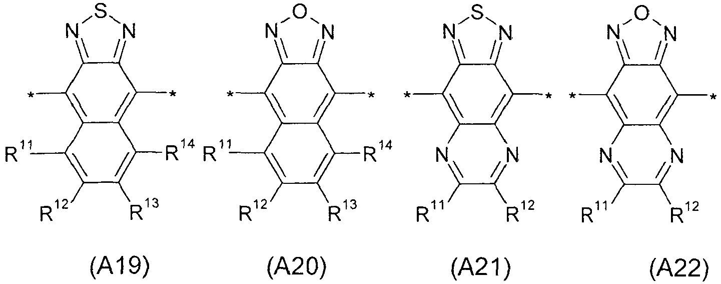 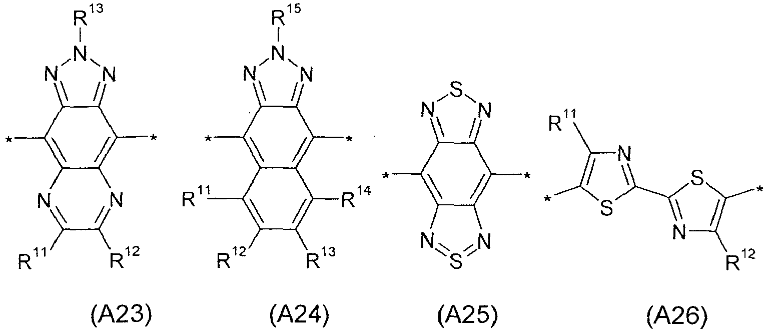 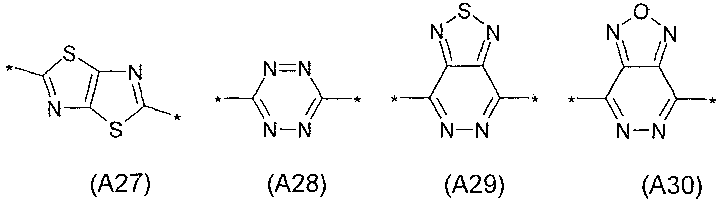 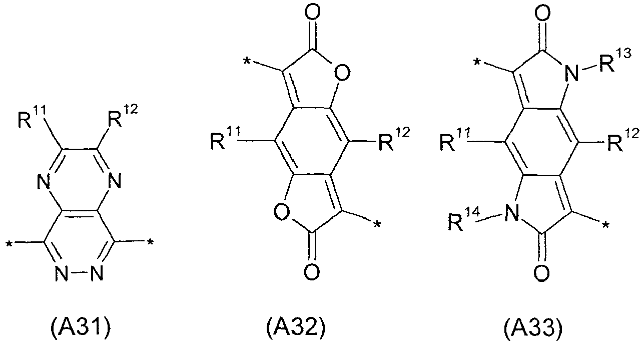 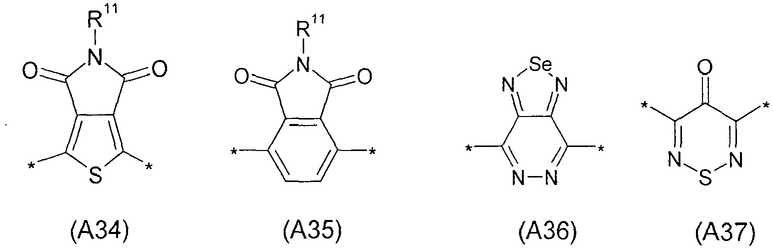 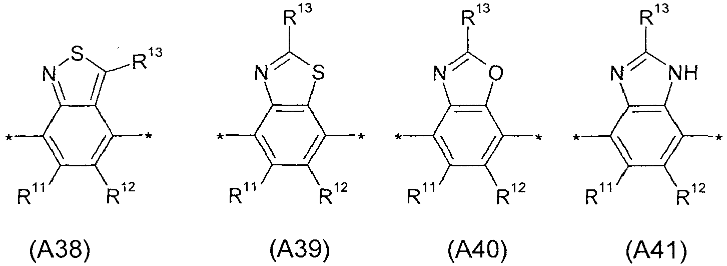 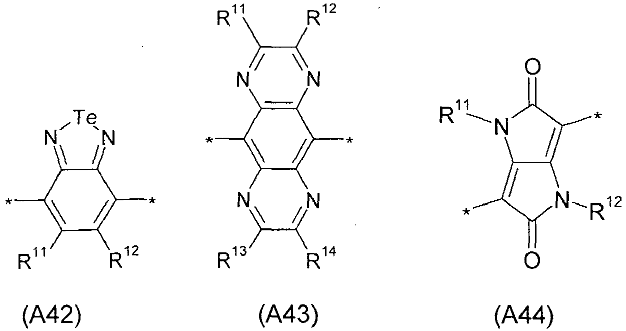 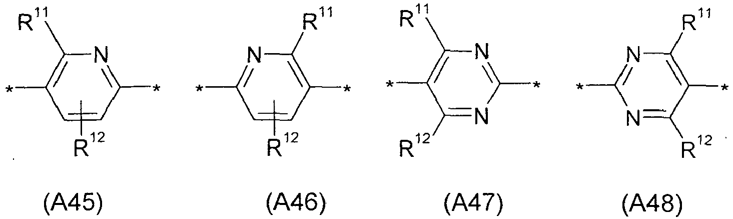 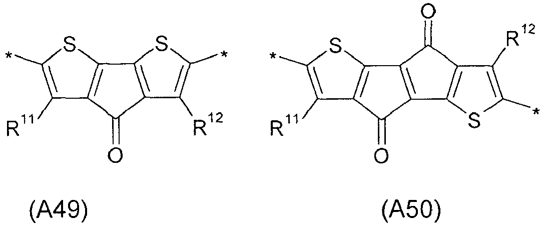 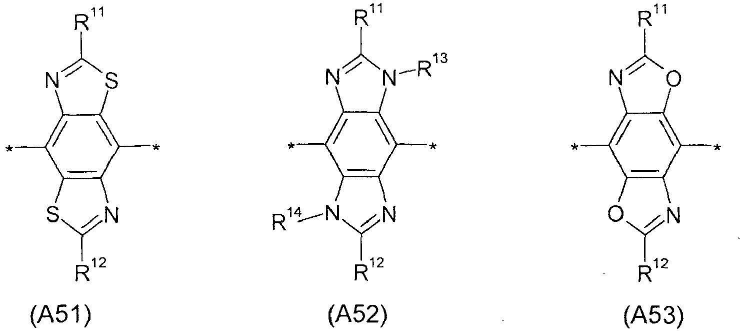 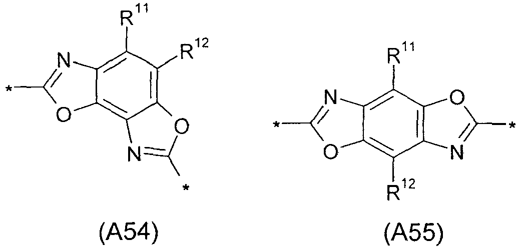 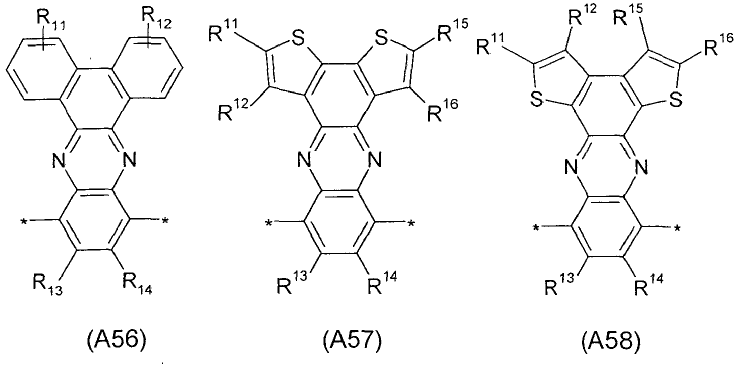 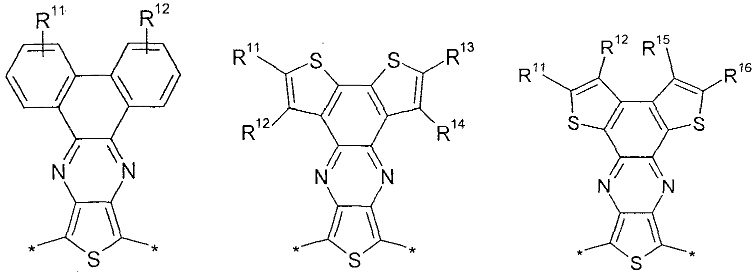 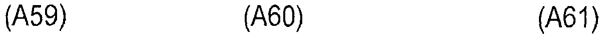 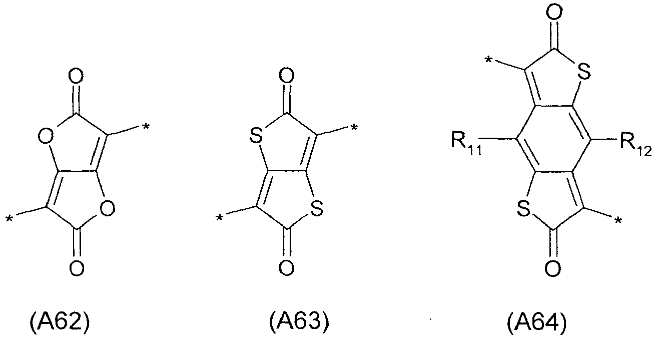 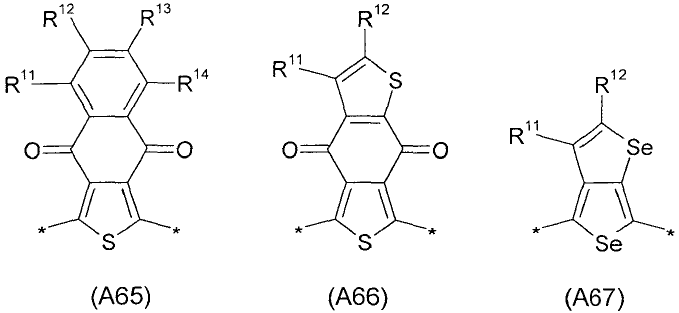 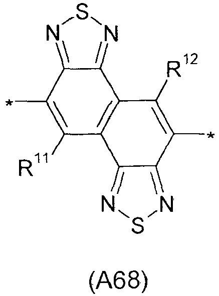 wherein R¹¹, R¹², R¹³, R¹⁴, R¹⁵ and R¹⁶ independently of each other denote H or have one of the meanings of R¹ - R⁴ as defined above and below.

In the polymers according to the present invention, the total number n of repeating units ranges from 2 to 10,000. The total number of repeating units is preferably 5 ≤ n ≤ 1000, very preferably 10 ≤ n ≤ 800, and most preferably 50 ≤ n ≤ 500.

The compounds of the present invention can be synthesized according to or in analogy to methods that are known to the skilled person and are described in the literature. Other methods of preparation can be taken from the examples. For example, polymers can be suitably prepared by aryl-aryl coupling reactions, such as Yamamoto coupling, Suzuki coupling, Stille coupling, Sonogashira coupling, Heck coupling or Buchwald coupling. Suzuki coupling, Buchwald coupling and Yamamoto coupling are especially preferred. The synthesis of the monomers according to the present invention is outlined in the reaction schemes below and specifically shown in the examples.

Preferred aryl-aryl coupling and polymerisation methods used in the processes described above and below are Yamamoto coupling, Negishi coupling, Suzuki coupling, Stille coupling, Sonogashira coupling, Heck coupling, C-H activation coupling, Ullmann coupling or Buchwald coupling. Especially preferred are Suzuki coupling, Negishi coupling, Stille coupling and Buchwald coupling. Suzuki coupling is described for example in WO 00/53656 A1. Negishi coupling is described for example in J. Chem. Soc., Chem. Commun., 1977, 683-684. Yamamoto coupling is described in for example in T. Yamamoto et al., Prog. Polym. Sci., 1993, 17, 1153-1205, or WO 2004/022626 A1, and Stille coupling is described for example in Z. Bao et al., J. Am. Chem. Soc., 1995, 117, 12426-12435. For example, when using Yamamoto coupling, monomers having two reactive halide groups are preferably used. When using Suzuki coupling, compounds of formula II having two reactive boronic acid or boronic acid ester groups or two reactive halide groups are preferably used. When using Stille coupling, monomers having two reactive stannane groups or two reactive halide groups are preferably used. When using Negishi coupling, monomers having two reactive organozinc groups or two reactive halide groups are preferably used.

Preferred catalysts, especially for Suzuki, Negishi or Stille coupling, are selected from Pd(0) complexes or Pd(II) salts. Preferred Pd(0) complexes are those bearing at least one phosphine ligand such as Pd(Ph₃P)₄. Another preferred phosphine ligand is tris(*ortho*-tolyl)phosphine, i.e. Pd(o-Tol₃P)₄. Preferred Pd(II) salts include palladium acetate, i.e. Pd(OAc)₂. Alternatively the Pd(0) complex can be prepared by mixing a Pd(0) dibenzylideneacetone complex, for example tris(dibenzyl-ideneacetone)dipalladium(0), bis(dibenzylideneacetone)palladium(0), or Pd(II) salts *e.g.* palladium acetate, with a phosphine ligand, for example triphenylphosphine, tris(*ortho-*tolyl)phosphine or tri(tert-butyl)phosphine. Suzuki polymerisation is performed in the presence of a base, for example sodium carbonate, potassium carbonate, lithium hydroxide, potassium phosphate or an organic base such as tetraethylammonium carbonate or tetraethylammonium hydroxide. Yamamoto polymerisation employs a Ni(0) complex, for example bis(1,5-cyclooctadienyl) nickel(0).

Suzuki, Stille and Buchwald polymerisation may be used to prepare homopolymers as well as statistical, alternating and block random copolymers. Statistical or block copolymers can be prepared for example from monomers based directly on formula I with all the mentioned co-monomers or the above monomers of formula II, wherein one of the reactive groups is halogen and the other reactive group is a boronic acid, boronic acid derivative group or and alkylstannane. The synthesis of statistical, alternating and block copolymers is described in detail for example in WO 03/048225 A2 or WO 2005/014688 A2.

As alternatives to halogens as described above, well known leaving groups such as tosylate, mesylate and triflate can be used.

For example, a polymer according to the present invention can be prepared by a process of coupling one or more monomers according to formula IV, wherein R⁷ and R⁸ are selected from Cl, Br, I, -B(OZ²)₂and-Sn(Z⁴)₃, with each other and/or with one or more monomers selected from the following formulae

R⁷-(Ar¹)ₐ-(Ar)²_{c}-(Ar³)_{d}-R⁸ V

R⁷-Ar¹-R⁸ VI

R⁷-Ar²-R⁸ VII

R⁷-Ar³-R⁸ VIII

wherein Ar¹, Ar², Ar³, a, c and d have one of the meanings of formula II or one of the meanings given above and below, with a+c+d≥2, and R⁷ and R⁸ are selected from Cl, Br, I, -B(OZ²)₂ and -Sn(Z⁴)₃, in an aryl-aryl coupling reaction.

Especially suitable and preferred synthesis methods of the units and monomers of formulae I, IA, IB and IIa are illustrated in the synthesis schemes shown below, wherein R¹ to R⁴ have the meaning as mentioned above and Ar has one of the meanings of Ar¹⁻³.

The synthesis of a typical symmetric dibromide monomer of formula IA is exemplarily illustrated in Scheme 1 below.

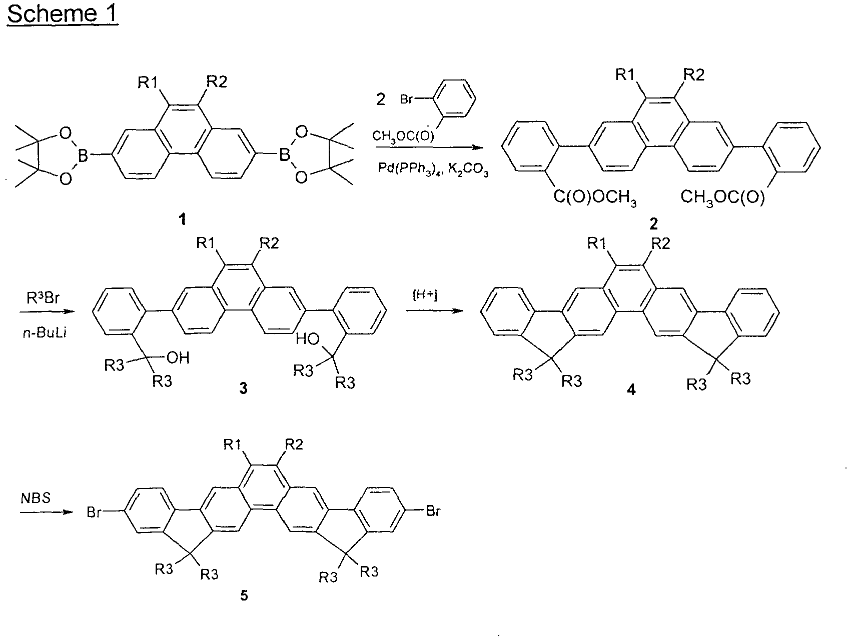

The synthesis of a typical symmetric dibromide monomer of formula IB is exemplarily illustrated in Scheme 2 below.

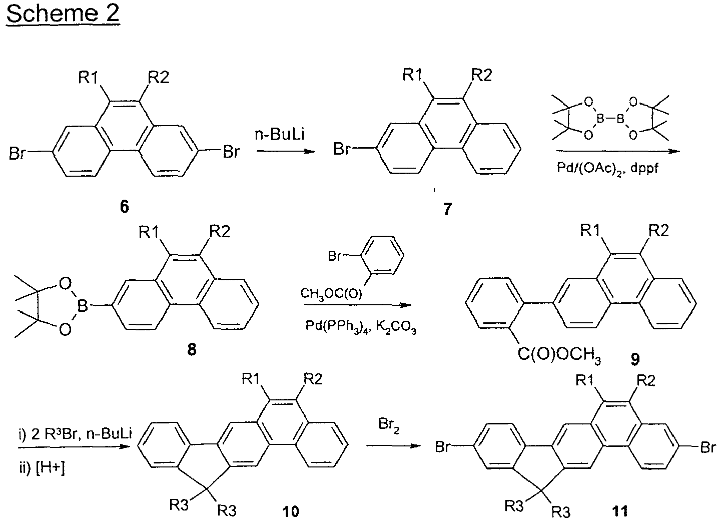

The synthesis of a typical monomer of formula IB with two different reactive groups is exemplarily illustrated in Scheme 3 below. These asymmetric monomers are suitable for preparing polymers of high regioregularity.

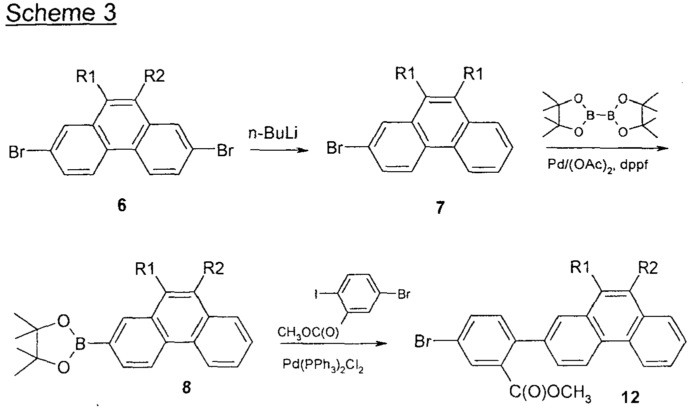

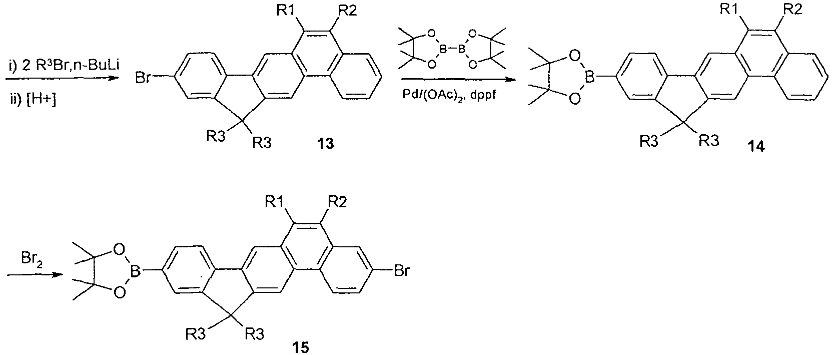

The conversion of dibromide monomers to diboronic ester monomers is exemplarily illustrated in Scheme 4.

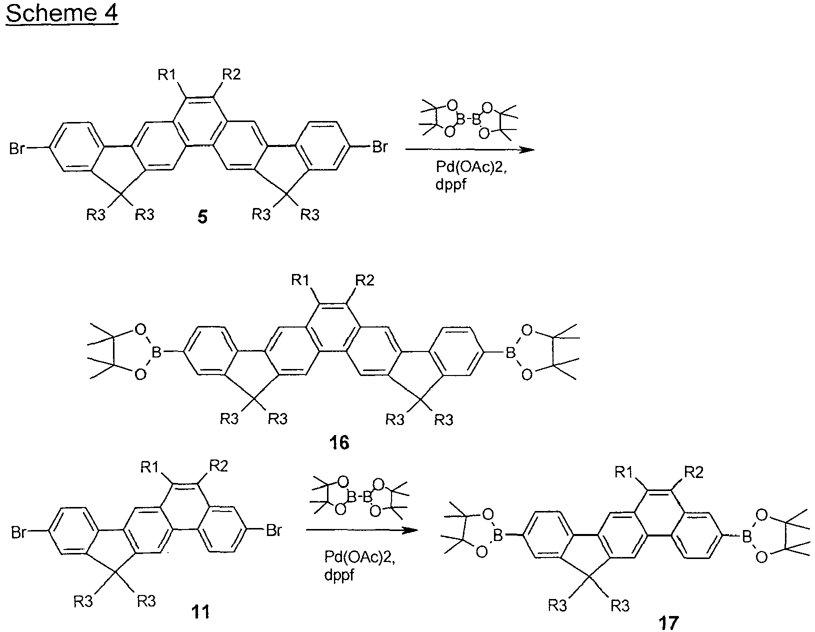

The homo- and copolymerisation is exemplarily illustrated in Scheme 5.

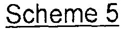

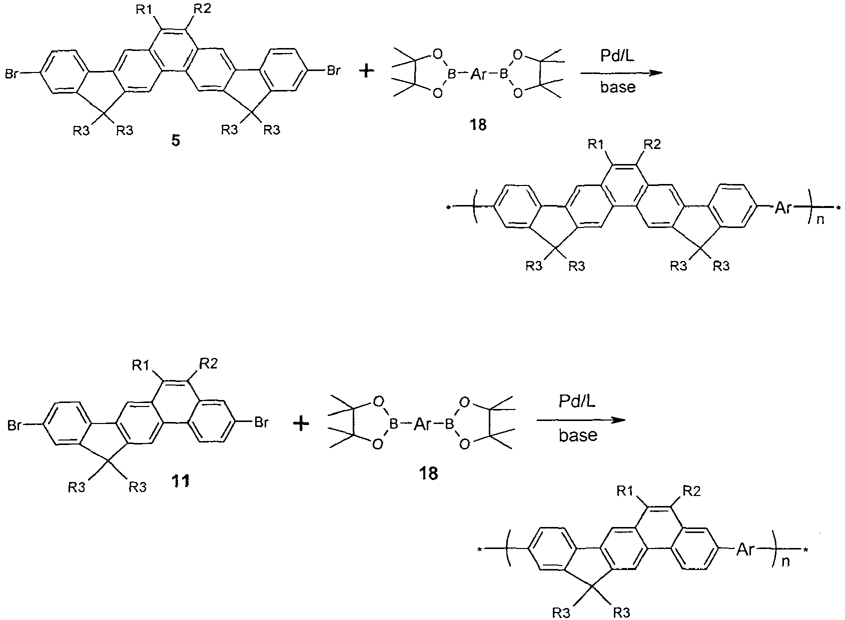

The novel methods of preparing monomers and polymers as described above and below are another aspect of the invention.

The compounds and polymers according to the present invention can also be used in mixtures or polymer blends, for example together with small molecules, monomeric compounds or together with other polymers having charge-transport, semiconducting, electrically conducting, photoconducting and/or light emitting semiconducting properties. Thus, another aspect of the invention relates to a polymer blend comprising one or more polymers according to the present invention and one or more further polymers having one or more of the above-mentioned properties. These blends can be prepared by conventional methods that are described in prior art and known to the skilled person. Typically the polymers are mixed with each other or dissolved in suitable solvents and the solutions combined.

Another aspect of the invention relates to a formulation comprising one or more small molecules, polymers, mixtures or polymer blends as described above and below and one or more organic solvents.

Preferred solvents are aliphatic hydrocarbons, chlorinated hydrocarbons, aromatic hydrocarbons, ketones, ethers and mixtures thereof. Additional solvents which can be used include 1,2,4-trimethylbenzene, 1,2,3,4-tetramethyl benzene, pentylbenzene, mesitylene, cumene, cymene, cyclohexylbenzene, diethylbenzene, tetralin, decalin, 2,6-lutidine, 2-fluoro-m-xylene, 3-fluoro-o-xylene, 2-chlorobenzotrifluoride, dimethylformamide, 2-chloro-6fluorotoluene, 2-fluoroanisole, anisole, 2,3-dimethylpyrazine, 4-fluoroanisole, 3-fluoroanisole, 3-trifluoro-methylanisole, 2-methylanisole, phenetol, 4-methylanisole, 3-methylanisole, 4-fluoro-3-methylanisole, 2-fluorobenzonitrile, 4-fluoroveratrol, 2,6-dimethylanisole, 3-fluorobenzo-nitrile, 2,5-dimethylanisole, 2,4-dimethylanisole, benzonitrile, 3,5-dimethylanisole, N,N-dimethylaniline, ethyl benzoate, 1-fluoro-3,5-dimethoxybenzene, 1-methylnaphthalene, N-methylpyrrolidinone, 3-fluorobenzotrifluoride, benzotrifluoride, benzotrifluoride, diosane, trifluoromethoxy-benzene, 4-fluorobenzotrifluoride, 3-fluoropyridine, toluene, 2-fluorotoluene, 2-fluorobenzotrifluoride, 3-fluorotoluene, 4-isopropylbiphenyl, phenyl ether, pyridine, 4-fluorotoluene, 2,5-difluorotoluene, 1-chloro-2,4-difluorobenzene, 2-fluoropyridine, 3-chlorofluorobenzene, 3-chlorofluorobenzene, 1-chloro-2,5-difluorobenzene, 4-chlorofluorobenzene, chlorobenzene, o-dichlorobenzene, 2-chlorofluorobenzene, p-xylene, m-xylene, o-xylene or mixture of o-, m-, and p-isomers. Solvents with relatively low polarity are generally preferred. For inkjet printing solvents and solvent mixtures with high boiling temperatures are preferred. For spin coating alkylated benzenes like xylene and toluene are preferred.

Examples of especially preferred solvents include, without limitation, dichloromethane, trichloromethane, monochlorobenzene, o-dichlorobenzene, tetrahydrofuran, anisole, morpholine, toluene, o-xylene, m-xylene, p-xylene, 1,4-dioxane, acetone, methylethylketone, 1,2-dichloroethane, 1,1,1-trichloroethane, 1,1,2,2-tetrachloroethane, ethyl acetate, n-butyl acetate, dimethylformamide, dimethylacetamide, dimethylsulfoxide, tetraline, decaline, indane, methyl benzoate, ethyl benzoate, mesitylene and/or mixtures thereof.

The concentration of polymers in the solution is preferably 0.1 to 2% by weight, more preferably 0.2 to 1.5% by weight. Lower molecular weight compounds have a wider preferable concentration range since, depending on their function in the final device, they can be present in a small percentage or as the main compound. Preferably the concentration thus ranges from 0.01 to 2.5%, more preferably from 0.02 to 1.6% by weight. Optionally, the solution also comprises one or more binders to adjust the rheological properties, as described for example in WO 2005/055248 A1.

After the appropriate mixing and ageing, solutions are evaluated as one of the following categories: complete solution, borderline solution or insoluble. The contour line is drawn to outline the solubility parameter-hydrogen bonding limits dividing solubility and insolubility. 'Complete' solvents falling within the solubility area can be chosen from literature values such as published in "Crowley, J.D., Teague, G.S. Jr and Lowe, J.W. Jr., Journal of Paint Technology, 1966, 38 (496), 296 ". Solvent blends may also be used and can be identified as described in "Solvents, W.H.Ellis, Federation of Societies for Coatings Technology, p9-10, 1986". Such a procedure may lead to a blend of 'non' solvents that will dissolve both the polymers of the present invention, although it is desirable to have at least one true solvent in a blend.

The compounds and polymers according to the present invention can also be used in patterned OSC layers in the devices as described above and below. For applications in modern microelectronics it is generally desirable to generate small structures or patterns to reduce cost (more devices/unit area), and power consumption. Patterning of thin layers comprising a polymer according to the present invention can be carried out for example by photolithography, electron beam lithography or laser patterning.

For use as thin layers in electronic or electrooptical devices the compounds, polymers, polymer blends or formulations of the present invention may be deposited by any suitable method. Liquid coating of devices is more desirable than vacuum deposition techniques. Solution deposition methods are especially preferred. The formulations of the present invention enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, dip coating, curtain coating, brush coating, slot dye coating or pad printing.

Inkjet printing is particularly preferred when high resolution layers and devices need to be prepared.Selected formulations of the present invention may be applied to prefabricated device substrates by ink jet printing or microdispensing. Preferably industrial piezoelectric print heads such as but not limited to those supplied by Aprion, Hitachi-Koki, InkJet Technology, On Target Technology, Picojet, Spectra, Trident, Xaar may be used to apply the organic semiconductor layer to a substrate. Additionally semi-industrial heads such as those manufactured by Brother, Epson, Konica, Seiko Instruments Toshiba TEC or single nozzle microdispensers such as those produced by Microdrop and Microfab may be used.

In order to be applied by inkjet printing or microdispensing, the compounds or polymers should be first dissolved in a suitable solvent. Solvents must fulfil the requirements stated above and must not have any detrimental effect on the chosen print head. Additionally, solvents should have boiling points >100°C, preferably >140°C and more preferably >150°C in order to prevent operability problems caused by the solution drying out inside the print head. Apart from the solvents methoned above, suitable solvents include substituted and non-substituted xylene derivatives, di-C₁₋₂-alkyl formamide, substituted and non-substituted anisoles and other phenol-ether derivatives, substituted heterocycles such as substituted pyridines, pyrazines, pyrimidines, pyrrolidinones, substituted and non-substituted *N*,*N*-di-C₁₋₂-alkylanilines and other fluorinated or chlorinated aromatics.

A preferred solvent for depositing a compound or polymer according to the present invention by inkjet printing comprises a benzene derivative which has a benzene ring substituted by one or more substituents wherein the total number of carbon atoms among the one or more substituents is at least three. For example, the benzene derivative may be substituted with a propyl group or three methyl groups, in either case there being at least three carbon atoms in total. Such a solvent enables an ink jet fluid to be formed comprising the solvent with the compound or polymer, which reduces or prevents clogging of the jets and separation of the components during spraying. The solvent(s) may include those selected from the following list of examples: dodecylbenzene, 1-methyl-4-tert-butylbenzene, terpineol limonene, isodurene, terpinolene, cymene, diethylbenzene. The solvent may be a solvent mixture, that is a combination of two or more solvents, each solvent preferably having a boiling point >100°C, more preferably >140°C. Such solvent(s) also enhance film formation in the layer deposited and reduce defects in the layer.

The inkjet fluid (that is mixture of solvent, binder and semiconducting compound) preferably has a viscosity at 20°C of 1-100 mPa·s, more preferably 1-50 mPas and most preferably 1-30 mPa·s.

The polymer blends and formulations according to the present invention can additionally comprise one or more further components or additives selected for example from surface-active compounds, lubricating agents, wetting agents, dispersing agents, hydrophobing agents, adhesive agents, flow improvers, defoaming agents, deaerators, diluents which may be reactive or non-reactive, auxiliaries, colourants, dyes or pigments, sensitizers, stabilizers, nanoparticles or inhibitors.

The compounds and polymers to the present invention are useful as charge transport, semiconducting, electrically conducting, photoconducting or light emitting materials in optical, electrooptical, electronic, electroluminescent or photoluminescent components or devices. In these devices, the polymers and compounds of the present invention are typically applied as thin layers or films.

Thus, the present invention also provides the use of the semiconducting compound, polymer, polymers blend, formulation or layer in an electronic device. The formulation may be used as a high mobility semiconducting material in various devices and apparatus. The formulation may be used, for example, in the form of a semiconducting layer or film. Accordingly, in another aspect, the present invention provides a semiconducting layer for use in an electronic device, the layer comprising a compound, polymer, polymer blend or formulation according to the invention. The layer or film may be less than about 30 microns. For various electronic device applications, the thickness may be less than about 1 micron thick. The layer may be deposited, for example on a part of an electronic device, by any of the aforementioned solution coating or printing techniques.

The invention additionally provides an electronic device comprising a compound, polymer, polymer blend, formulation or organic semiconducting layer according to the present invention. Especially preferred devices are OFETs, TFTs, ICs, logic circuits, capacitors, RFID tags, OLEDs, OLETs, OPEDs, OPVs, solar cells, laser diodes, photoconductors, photodetectors, electrophotographic devices, electrophotographic recording devices, organic memory devices, sensor devices, charge injection layers, Schottky diodes, planarising layers, antistatic films, conducting substrates and conducting patterns.

Especially preferred electronic devices are OFETs, OLEDs and OPV devices, in particular bulk heterojunction (BHJ) OPV devices. In an OFET, for example, the active semiconductor channel between the drain and source may comprise the layer of the invention. As another example, in an OLED device, the charge (hole or electron) injection or transport layer may comprise the layer of the invention.

For use in OPV or OPD devices the polymer according to the present invention is preferably used in a formulation that comprises or contains, more preferably consists essentially of, very preferably exclusively of, a p-type (electron donor) semiconductor and an n-type (electron acceptor) semiconductor. The p-type semiconductor is constituted by a polymer according to the present invention. The n-type semiconductor can be an inorganic material such as zinc oxide (ZnOₓ), zinc tin oxide (ZTO), titan oxide (TiOₓ), molybdenum oxide (MoOₓ), nickel oxide (NiOₓ), or cadmium selenide (CdSe), or an organic material such as graphene or a fullerene or substituted fullerene, for example an indene-C₆₀-fullerene bisaduct like ICBA, or a (6,6)-phenyl-butyric acid methyl ester derivatized methano C₆₀ fullerene, also known as "PCBM-C₆₀" or "C₆₀PCBM", as disclosed for example in G. Yu, J. Gao, J.C. Hummelen, F. Wudl, A.J. Heeger, Science 1995, Vol. 270, p. 1789 ff and having the structure shown below, or structural analogous compounds with e.g. a C₆₁ fullerene group, a C₇₀ fullerene group, or a C₇₁ fullerene group, or an organic polymer (see for example Coakley, K. M. and McGehee, M. D. Chem. Mater. 2004, 16, 4533).

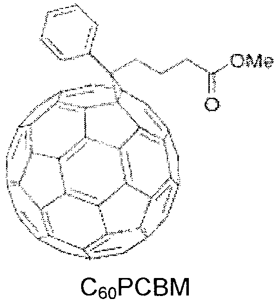

Preferably the polymer according to the present invention is blended with an n-type semiconductor such as a fullerene or substituted fullerene, like for example PCBM-C₆₀, PCBM-C₇₀, PCBM-C₆₁, PCBM-C₇₁, bis-PCBM-C₆₁, bis-PCBM-C₇₁, ICBA (1',1",4',4"- tetrahydro-di[1,4]methanonaphthaleno [1,2:2',3';56,60:2",3"][5,6]fullerene-C60-lh), graphene, or a metal oxide, like for example, ZnOₓ, TiOₓ, ZTO, MoOₓ, NiOₓ, to form the active layer in an OPV or OPD device. The device preferably further comprises a first transparent or semi-transparent electrode on a transparent or semi-transparent substrate on one side of the active layer, and a second metallic or semi-transparent electrode on the other side of the active layer.

Further preferably the OPV or OPD device comprises, between the active layer and the first or second electrode, one or more additional buffer layers acting as hole transporting layer and/or electron blocking layer, which comprise a material such as metal oxide, like for example, ZTO, MoOₓ, NiOₓ, a conjugated polymer electrolyte, like for example PEDOT:PSS, a conjugated polymer, like for example polytriarylamine (PTAA), an organic compound, like for example N,N'-diphenyl-N,N'-bis(1-naphthyl)(1,1'-biphenyl)-4,4'diamine (NPB), N,N'-diphenyl-N,N'-(3-methylphenyl)-1,1'-biphenyl-4,4'-diamine (TPD), or alternatively as hole blocking layer and/or electron transporting layer, which comprises a material such as a metal oxide, like for example, ZnOₓ, TiOₓ, a salt, like for example LiF, NaF, CsF, a conjugated polymer electrolyte, like for example poly[3-(6-trimethylammoniumhexyl)thiophene], poly(9,9-bis(2-ethylhexyl)-fluorene]-*b*-poly[3-(6-trimethylammoniumhexyl)thiophene], or poly [(9,9-bis(3'-(N,N-dimethylamino)propyl)-2,7-fluorene)-alt-2,7-(9,9-dioctylfluorene)] or an organic compound, like for example tris(8-quinolinolato)-aluminium(III) (Alq₃), 4,7-diphenyl-1,10-phenanthroline.

In a blend or mixture of a polymer according to the present invention with a fullerene or modified fullerene, the ratio polymer:fullerene is preferably from 5:1 to 1:5 by weight, more preferably from 1:1 to 1:3 by weight, most preferably 1:1 to 1:2 by weight. A polymeric binder may also be included, from 5 to 95% by weight. Examples of binder include polystyrene(PS), polypropylene (PP), polycarbonate (PC), Polyvinylbutyral (PVB) and polymethylmethacrylate (PMMA).

To produce thin layers in BHJ OPV devices the compounds, polymers, polymer blends or formulations of the present invention may be deposited by any suitable method. Liquid coating of devices is more desirable than vacuum deposition techniques. Solution deposition methods are especially preferred. The formulations of the present invention enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, dip coating, curtain coating, brush coating, slot dye coating or pad printing. For the fabrication of OPV devices and modules area printing method compatible with flexible substrates are preferred, for example slot dye coating, spray coating and the like.

Suitable solutions or formulations containing the blend or mixture of a polymer according to the present invention with a C₆₀ or C₇₀ fullerene or modified fullerene like PCBM must be prepared. In the preparation of formulations, suitable solvents must be selected to ensure full dissolution of both components, p-type and n-type, and take into accound the boundary conditions (for example rheological properties) introduced by the chosen printing method.

Organic solvents are generally used for this purpose. Typical solvents can be aromatic solvents, halogenated solvents or chlorinated solvents, including chlorinated aromatic solvents. Examples include, but are not limited to chlorobenzene, 1,2-dichlorobenzene, chloroform, 1,2-dichloroethane, dichloromethane, carbon tetrachloride, toluene, cyclohexanone, ethylacetate, tetrahydrofuran, anisole, morpholine, o-xylene, m-xylene, p-xylene, 1,4-dioxane, acetone, methylethylketone, 1,2-dichloroethane, 1,1,1-trichloroethane, 1,1,2,2-tetrachloroethane, ethyl acetate, n-butyl acetate, dimethylformamide, dimethylacetamide, dimethylsulfoxide, tetraline, decaline, indane, methyl benzoate, ethyl benzoate, mesitylene and combinations thereof.

The OPV device can for example be of any type known from the literature (see e.g. Waldauf et al., Appl. Phys. Lett., 2006, 89, 233517).

A first preferred OPV device according to the invention comprises the following layers (in the sequence from bottom to top):
- optionally a substrate,
- a high work function electrode, preferably comprising a metal oxide, like for example ITO, serving as anode,
- an optional conducting polymer layer or hole transport layer, preferably comprising an organic poymer or polymer blend, for example of PEDOT:PSS (poly(3,4-ethylenedioxythiophene): poly(styrene-sulfonate), or TBD (N,N'-dyphenyl-N-N'-bis(3-methylphenyl)-1,1'biphenyl-4,4'-diamine) or NBD (N,N'-dyphenyl-N-N'-bis(1-napthylphenyl)-1,1'biphenyl-4,4'-diamine),
- a layer, also referred to as "active layer", comprising a p-type and an n-type organic semiconductor, which can exist for example as a p-type/n-type bilayer or as distinct p-type and n-type layers, or as blend or p-type and n-type semiconductor, forming a BHJ,
- optionally a layer having electron transport properties, for example comprising LiF,
- a low work function electrode, preferably comprising a metal like for example aluminum, serving as cathode,
   wherein at least one of the electrodes, preferably the anode, is transparent to visible light, and
   wherein the p-type semiconductor is a polymer according to the present invention.

A second preferred OPV device according to the invention is an inverted OPV device and comprises the following layers (in the sequence from bottom to top):
- optionally a substrate,
- a high work function metal or metal oxide electrode, comprising for example ITO, serving as cathode,
- a layer having hole blocking properties, preferably comprising a metal oxide like TiOₓ or Znₓ,
- an active layer comprising a p-type and an n-type organic semiconductor, situated between the electrodes, which can exist for example as a p-type/n-type bilayer or as distinct p-type and n-type layers, or as blend or p-type and n-type semiconductor, forming a BHJ,
- an optional conducting polymer layer or hole transport layer, preferably comprising an organic poymer or polymer blend, for example of PEDOT:PSS or TBD or NBD,
- an electrode comprising a high work function metal like for example silver, serving as anode,
   wherein at least one of the electrodes, preferably the cathode, is transparent to visible light, and
   wherein the p-type semiconductor is a polymer according to the present invention.

In the OPV devices of the present invent invention the p-type and n-type semiconductor materials are preferably selected from the materials, like the polymer/fullerene systems, as described above

When the active layer is deposited on the substrate, it forms a BHJ that phase separates at nanoscale level. For discussion on nanoscale phase separation see Dennler et al, Proceedings of the IEEE, 2005, 93 (8), 1429 or Hoppe et al, Adv. Func. Mater, 2004, 14(10), 1005. An optional annealing step may be then necessary to optimize blend morpohology and consequently OPV device performance.

Another method to optimize device performance is to prepare formulations for the fabrication of OPV(BHJ) devices that may include high boiling point additives to promote phase separation in the right way. 1,8-octanedithiol, 1,8-diiodooctane, nitrobenzene, chloronaphthalene, and other additives have been used to obtain high-efficiency solar cells. Examples are disclosed in J. Peet, et al, Nat. Mater., 2007, 6, 497 or Fréchet et al. J. Am. Chem. Soc., 2010, 132, 7595-7597.

The compounds, polymers, formulations and layers of the present invention are also suitable for use in an OFET as the semiconducting channel. Accordingly, the invention also provides an OFET comprising a gate electrode, an insulating (or gate insulator) layer, a source electrode, a drain electrode and an organic semiconducting channel connecting the source and drain electrodes, wherein the organic semiconducting channel comprises a compound, polymer, polymer blend, formulation or organic semiconducting layer according to the present invention. Other features of the OFET are well known to those skilled in the art.

OFETs where an OSC material is arranged as a thin film between a gate dielectric and a drain and a source electrode, are generally known, and are described for example in US 5,892,244, US 5,998,804, US 6,723,394 and in the references cited in the background section. Due to the advantages, like low cost production using the solubility properties of the compounds according to the invention and thus the processibility of large surfaces, preferred applications of these FETs are such as integrated circuitry, TFT displays and security applications.

The gate, source and drain electrodes and the insulating and semiconducting layer in the OFET device may be arranged in any sequence, provided that the source and drain electrode are separated from the gate electrode by the insulating layer, the gate electrode and the semiconductor layer both contact the insulating layer, and the source electrode and the drain electrode both contact the semiconducting layer.

An OFET device according to the present invention preferably comprises:
- a source electrode,
- a drain electrode,
- a gate electrode,
- a semiconducting layer,
- one or more gate insulator layers,
- optionally a substrate.
wherein the semiconductor layer preferably comprises a compound, polymer, polymer blend or formulation as described above and below.

The OFET device can be a top gate device or a bottom gate device. Suitable structures and manufacturing methods of an OFET device are known to the skilled in the art and are described in the literature, for example in US 2007/0102696 A1.

The gate insulator layer preferably comprises a fluoropolymer, like e.g. the commercially available Cytop 809M® or Cytop 107M® (from Asahi Glass). Preferably the gate insulator layer is deposited, e.g. by spin-coating, doctor blading, wire bar coating, spray or dip coating or other known methods, from a formulation comprising an insulator material and one or more solvents with one or more fluoro atoms (fluorosolvents), preferably a perfluorosolvent. A suitable perfluorosolvent is e.g. FC75® (available from Acros, catalogue number 12380). Other suitable fluoropolymers and fluorosolvents are known in prior art, like for example the perfluoropolymers Teflon AF® 1600 or 2400 (from DuPont) or Fluoropel® (from Cytonix) or the perfluorosolvent FC 43® (Acros, No. 12377). Especially preferred are organic dielectric materials having a low permittivity (or dielectric contant) from 1.0 to 5.0, very preferably from 1.8 to 4.0 ("low k materials"), as disclosed for example in US 2007/0102696 A1 or US 7,095,044.

In security applications, OFETs and other devices with semiconducting materials according to the present invention, like transistors or diodes, can be used for RFID tags or security markings to authenticate and prevent counterfeiting of documents of value like banknotes, credit cards or ID cards, national ID documents, licenses or any product with monetry value, like stamps, tickets, shares, cheques etc.

Alternatively, the materials according to the invention can be used in OLEDs, e.g. as the active display material in flat panel display applications, or as backlight of a flat panel display like e.g. a liquid crystal display. Common OLEDs are realized using multilayer structures. An emission layer is generally sandwiched between one or more electron-transport and / or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/ or in the emission layer, corresponding to their electrical and /or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see, e.g., Muller et al, Synth. Metals, 2000, 111-112, 31-34, Alcala, J. Appl. Phys., 2000, 88, 7124-7128 and the literature cited therein.

According to another use, the materials according to this invention, especially those showing photoluminescent properties, may be employed as materials of light sources, e.g. in display devices, as described in EP 0 889 350 A1 or by C. Weder et al., Science, 1998, 279, 835-837.

A further aspect of the invention relates to both the oxidised and reduced form of the compounds according to this invention. Either loss or gain of electrons results in formation of a highly delocalised ionic form, which is of high conductivity. This can occur on exposure to common dopants.

Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g., I₂, Cl₂, Br₂, ICl, ICl₃, IBr and IF), Lewis acids (e.g., PF₅, AsF₅, SbF₅, BF₃, BCl₃, SbCl₅, BBr₃ and SO₃), protonic acids, organic acids, or amino acids (e.g., HF, HCI, HNO₃, H₂SO₄, HClO₄, FSO₃H and ClSO₃H), transition metal compounds (e.g., FeCl₃, FeOCI, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g., Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g., H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃ 6H₂O, FSO₂OOSO₂F, Eu, acetylcholine, R₄N⁺, (R is an alkyl group), R₄P⁺ (R is an alkyl group), RₑAs⁺ (R is an alkyl group), and R₃S⁺ (R is an alkyl group).

The conducting form of the compounds of the present invention can be used as an organic "metal" in applications including, but not limited to, charge injection layers and ITO planarising layers in OLED applications, films for flat panel displays and touch screens, antistatic films, printed conductive substrates, patterns or tracts in electronic applications such as printed circuit boards and condensers.

The compounds and formulations according to the present invention amy also be suitable for use in organic plasmon-emitting diodes (OPEDs), as described for example in Koller et a/., Nat. Photonics, 2008, 2, 684.

According to another use, the materials according to the present invention can be used alone or together with other materials in or as alignment layers in LCD or OLED devices, as described for example in US 2003/0021913. The use of charge transport compounds according to the present invention can increase the electrical conductivity of the alignment layer. When used in an LCD, this increased electrical conductivity can reduce adverse residual dc effects in the switchable LCD cell and suppress image sticking or, for example in ferroelectric LCDs, reduce the residual charge produced by the switching of the spontaneous polarisation charge of the ferroelectric LCs. When used in an OLED device comprising a light emitting material provided onto the alignment layer, this increased electrical conductivity can enhance the electroluminescence of the light emitting material. The compounds or materials according to the present invention having mesogenic or liquid crystalline properties can form oriented anisotropic films as described above, which are especially useful as alignment layers to induce or enhance alignment in a liquid crystal medium provided onto said anisotropic film. The materials according to the present invention may also be combined with photoisomerisable compounds and/or chromophores for use in or as photoalignment layers, as described in US 2003/0021913 A1.

According to another use the materials according to the present invention, especially their water-soluble derivatives (for example with polar or ionic side groups) or ionically doped forms, can be employed as chemical sensors or materials for detecting and discriminating DNA sequences. Such uses are described for example in L. Chen, D. W. McBranch, H. Wang, R. Helgeson, F. Wudl and D. G. Whitten, Proc. Natl. Acad. Sci. U.S.A., 1999, 96, 12287; D. Wang, X. Gong, P. S. Heeger, F. Rininsland, G. C. Bazan and A. J. Heeger, Proc. Natl. Acad. Sci. U.S.A., 2002, 99, 49; N. DiCesare, M. R. Pinot, K. S. Schanze and J. R. Lakowicz, Langmuir, 2002, 18, 7785; D. T. McQuade, A. E. Pullen, T. M. Swager, Chem. Rev., 2000, 100, 2537.

Unless the context clearly indicates otherwise, as used herein plural forms of the terms herein are to be construed as including the singular form and vice versa. Above and below, unless stated otherwise percentages are per cent by weight and temperatures are given in degrees Celsius.

Throughout the description and claims of this specification, the words "comprise" and "contain" and variations of the words, for example "comprising" and "comprises", mean "including but not limited to", and are not intended to (and do not) exclude other components.

It will be appreciated that variations to the foregoing embodiments of the invention can be made within the scope of the invention.

All of the features disclosed in this specification may be combined in any combination, except combinations where at least some of such features and/or steps are mutually exclusive. In particular, the preferred features of the invention are applicable to all aspects of the invention and may be used in any combination. Likewise, features described in non-essential combinations may be used separately (not in combination).
The invention will now be described in more detail by reference to the following examples, which are illustrative only and do not limit the scope of the invention.

### Example 1

### Procedures for the synthesis of monomer I

The synthetic sequence for monomer **I** is as follows:

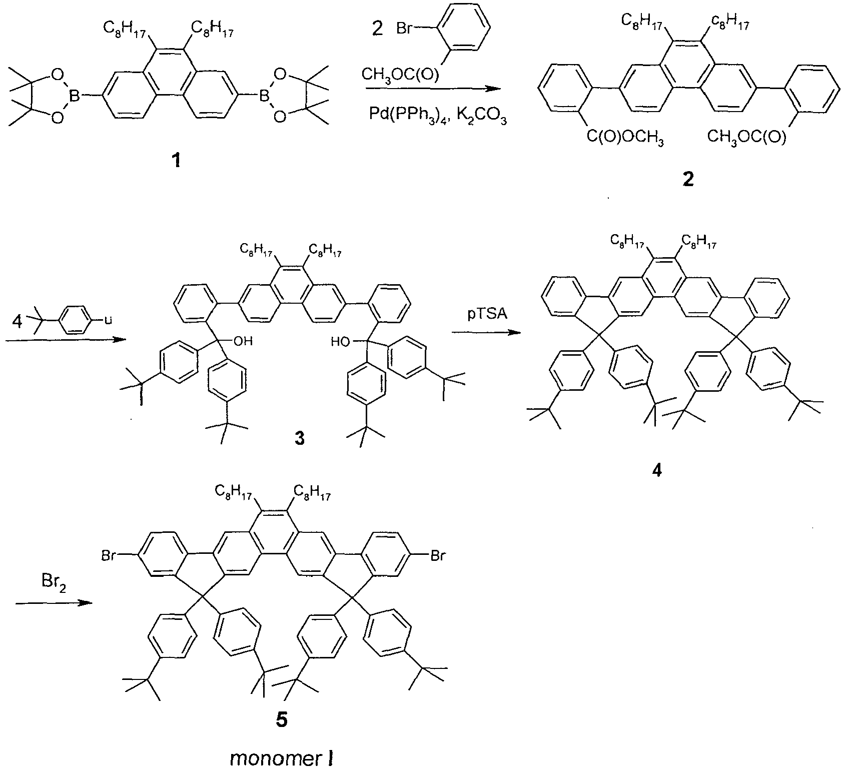

The synthesis of starting material **1** is described in WO2005/104264 A1.

The individual steps in the above synthetic sequence are described below.

### Preparation of 2 via Suzuki coupling of 1 with methyl 2-bromobenzoate

A 250 ml three-neck flask, equipped with magnetic stirrer, reflux condenser and argon inlet/exhaust, is charged with 9,10-dioctylphenanthrene diboronic ester **1** (20.00 g, 30.4 mmol, 1.0 eq.), methyl 2-bromobenzoate (14.70 g, 68.4 mmol, 2.25 eq.), potassium carbonate (23.09 g, 167.0 mmol, 5.5 eq.), 60 ml toluene and 60 ml water. The resulting suspension is degassed at room temperature by purging with argon for 30 min. The solution of catalyst tetrakis(triphenylphosphine)palladium(0) (69.1 mg, 0.061 mmol, 0.002 eq.) is added, and the mixture heated to 95°C (oil bath) with rapid stirring.

After 24h, the reaction mixture is cooled down to room temperature. The aqueous phase is extracted with toluene. The combined toluene phases are filtered over celite to remove palladium black. The filtrate is dried over magnesium sulphate, and then concentrated to dryness *in vacuo.* The crude product is recrystallized in dichloromethane/ethanol (v:v 1:1) solvent mixture to obtain the product (16.0 g, yield: 78%). NMR(¹H, 500 MHz, CDCl₃): δ 8.74 (d, 2H, ArH); δ 8.04 (s, 2H, ArH); δ 7.90 (d, 2H, ArH); δ 7.61 (t, 2H, ArH); δ 7.57 (d, 2H, ArH); δ 7.53 (d, 2H, ArH); δ 7.48 (d, 2H, ArH); δ 3.14 (t, 4H, -CH₂-); δ 1.72 (m, 4H, -CH₂-); δ 1.54 (m, 4H, -CH₂-); δ 1.42-1.29 (m, 16H, -CH₂-); δ 0.88 (t, 6H, -CH₃).

### Arylation of 2 to obtain diol 3

A 500 ml three-neck flask, equipped with magnetic stirrer and argon inlet/exhaust, is charged with 1-bromo-4-tertbutylbenzene (12.68 g, 59.5 mmol, 5.0 eq.) and 100 ml anhydrous THF. The resulting solution is cooled to -78°C. n-Butyllithium (2.5M in hexanes) (23.8 ml, 59.5 mmol, 5.0 eq.) is added dropwise via syringe. The reaction mixture is allowed to stir at <-65°C for 1 hour. The solution of the methyl ester **2** (8.00 g, 11.9 mmol, 1.0 eq.) in 100 ml anhydrous THF is added dropwise, with the internal temperature remaining below -65°C. After complete addition of **2,** the solution is allowed to warm up slowly to room temperature over 3 hours.

The mixture is extracted with ethyl acetate (3 x 60ml). The combined organic phase is washed with water (100 ml), dried over magnesium sulfate, and then dried in vacuum to obtain **3** as a slightly yellow solid (13.0 g, yield: 96%). The product is used for the next step without further purification.

### Ring closure of 3 to 4

Compound **3** (13.0 g, 11.4 mmol, 1.0 eq.) from the previous step is dissolved in 120 ml toluene in a 250 ml flask. To this solution *p-*Toluenesulfonic acid monohydrate (0.23 g, 1.2 mmol, 0.1 eq.) is added.

The mixture is heated to 95°C (oil bath). After 3 hours, TLC indicates complete conversion of **3.**

The reaction mixture is allowed to cool down to room temperature. The solution is filtered through a plug of silica. The filtrate is dried in vacuum and recrystallized in toluene/heptane (1:10) to obtain intermediate 4 (9.38 g, yield: 72%). NMR(¹H, 500 MHz, CDCl₃): δ 8.59 (s, 2H, ArH); δ 8.41 (s, 2H, ArH); δ 7.92 (d, 2H, ArH); δ 7.45 (d, 2H, ArH); δ 7.40 (t, 2H, ArH); δ 7.22-7.17 (q, 16H, ArH); δ 3.23 (t, 4H, -CH₂-); δ 1.77 (m, 4H, -CH₂-); δ 1.64 (m, 4H, -CH₂-); δ 1.50 (m, 4H, -CH₂-); δ 1.42-1.35 (m, 12H, -CH₂-); δ 1.24 (s, 36H, -CH₃); δ 0.92 (t, 6H, -CH₃).

### Bromination of 4 to 5

Compound **4** (9.0 g, 8.1 mmol, 1.0 eq.) from the previous step is dissolved in 150 ml dry dichloromethane in a 250 ml flask. The solution is cooled down to 0°C, to which a solution of bromine (1.2 ml, 24.3 mmol, 3.0 eq.) in 5 ml dry dichloromethane is added dropwise. The reaction mixture is stirred at 0°C for 4 hours. TLC indicates complete conversion of **4.**

The reaction is quenched by addition of 55 ml 10 wt.% aqueous Na₂SO₃ solution. The aqueous phase is extracted with dichloromethane (3 x 50 ml). The combined organic phase is washed with water (100 ml), dried over magnesium sulfate, and concentrated to dryness *in vacuo.* The crude product is purified by repeated recrystallization in a toluene/heptane mixture to render the final product **5** (monomer **I**) as a slightly yellow solid (5.8 g, yield: 57%).

### Example 2

### Procedures for the synthesis of monomer II

The synthetic sequence for monomer **II** is as follows:

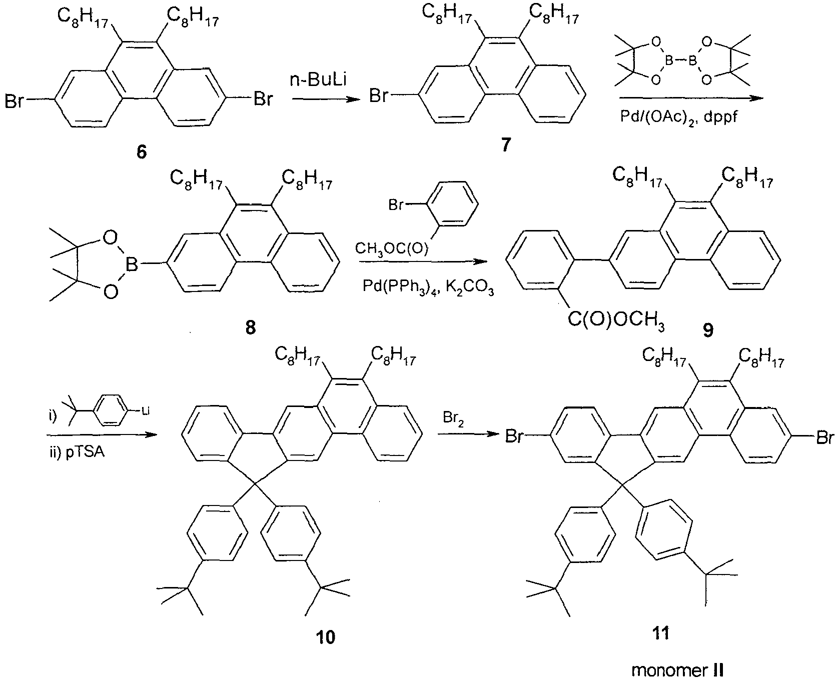

The synthesis of starting material **6** is described in WO2005/104264 A1.

The individual steps in the above synthetic sequence are described below.

### Monodebromination of 6 to 7

A 100 ml three-neck flask, equipped with magnetic stirrer and argon inlet/exhaust, is charged with phenanthrene dibromide **6** (5.62 g, 10.0 mmol, 1.0 eq.) and 50 ml anhydrous THF. The resulting solution is cooled to -78°C. n-Butyllithium (2.5 M in hexane) (4.4 ml, 11.0 mmol, 1.1 eq.) is added dropwise via syringe. The reaction mixture is allowed to stir at <-65°C for 1 hour. The reaction is then quenched by dropwise addition of 0.4 ml aqueous solution of NH₄Cl.

After warming up to room temperature, the mixture is extracted with ethyl acetate (3 x 20ml). The combined organic phase is washed with water (50 ml), dried over magnesium sulfate, and then concentrated to dryness *in vacuo* to obtain **7** as solid (4.4 g, yield: 92%).

### Preparation of monoboronic ester 8 from 7

A 250 ml three-neck flask, equipped with magnetic stirrer, reflux condenser and argon inlet/exhaust, is charged with phenanthrene monobromide **7** (3.86 g, 8.0 mmol, 1.0 eq.), bis(pinacolato)diborane (2.45 g, 9.6 mmol, 1.2 eq.), potassium acetate (2.20 g, 24.0 mmol, 3.0 eq.) and 100 ml anhydrous THF. The resulting suspension is degassed at room temperature by purging argon for 30 min. The catalyst [1,1'-bis(diphenylphosphino)ferrocene]dichloropalladium(II) (117.2 mg, 0.16 mmol, 0.02 eq.) is added as solid. The mixture is heated to reflux under rapid stirring. After 16h, TLC indicates the complete conversion of **7.**

The mixture is allowed to cool down to room temperature, and then filtered over Celite, and washed with THF (2 x 20 ml). The filtrate is concentrated *in vacuo.* The obtained dark brown oil is purified by column chromatography over silica with toluene as elute (3.3 g, yield: 77%).

### Preparation of 9 via Suzuki coupling of 8 with methyl 2-bromobenzoate

A 100 ml three-neck flask, equipped with magnetic stirrer, reflux condenser and argon inlet/exhaust, is charged with phenanthrene monoboronic ester **8** (3.17 g, 6.0 mmol, 1.0 eq.), methyl 2-bromobenzoate (1.46 g, 6.6 mmol, 1.1 eq.), potassium carbonate (2.49 g, 18.0 mmol, 3.0 eq.), 20 ml toluene and 20 ml water. The resulting suspension is degassed at room temperature by purging argon for 30 min. The solution of catalyst tetrakis(triphenylphosphine)palladium(0) (14.9 mg, 0.012 mmol, 0.002 eq.) is added, and the mixture is heated to 95°C (oil bath) under rapid stirring. After 36h, TLC indicates the complete conversion of 8.

After cooling down to room temperature, the aqueous phase is extracted with 2 x 20 ml toluene. The combined toluene phase is passed through a plug of Celite. The filtrate is dried over magnesium sulphate, and then concentrated to dryness *in vacuo.* The crude product is recrystallized in a dichloromethane/ethanol (2:1) mixture to obtain the product (2.8 g, 86%).

### Arylation of 9 and further ring closure to obtain 10

A 100 ml three-neck flask, equipped with magnetic stirrer and argon inlet/exhaust, is charged with 1-bromo-4-tertbutylbenzene (2.66 g, 12.5 mmol, 2.5 eq.) and 20 ml anhydrous THF. The resulting solution is cooled to -78°C. n-Butyllithium (2.5 M in hexane) (5.2 ml, 13.0 mmol, 2.6 eq.) is added dropwise via syringe. The reaction mixture is allowed to stir at <-65°C for 1 hour. The solution of the methyl ester **9** (2.68 g, 5.0 mmol, 1.0 eq.) in 20 ml anhydrous THF (20 ml) is added dropwise, with the internal temperature remaining below -65°C. Afterwards, the solution is allowed to warm up to room temperature over 3 hours.

The mixture is extracted with ethyl acetate (3 x 20ml). The combined organic phase is washed with water (50 ml), dried over magnesium sulfate, and then concentrated to dryness *in vacuo* to obtain the crude product (3.62 g, yield: 93%). The product is used for the next step without further purification.

All of the crude product from the previous step (3.62 g, 4.65 mmol, 1.0 eq.) is dissolved in 50 ml toluene in a 100 ml flask. To this solution *p-*toluenesulfonic acid monohydrate (0.088 g, 0.465 mmol, 0.1 eq.) is added. The mixture is heated to 95°C (oil bath) for 3 hours.

After the solution has cooled down to room temperature, the solution is filtered through a plug of silica. The filtrate is concentrated to dryness *in vacuo.* The crude product is recrystallized in a toluene/heptane (1:2) mixture to obtain intermediate **10** (2.52 g, yield: 72%).

### Bromination of 10 to 11

Compound **10** (2.5 g, 3.3 mmol, 1.0 eq.) from the previous step is dissolved in 60 ml dry dichloromethane in a 100 ml flask. A solution of bromine (0.48 ml, 9.9 mmol, 3.0 eq.) in 2 ml dry dichloromethane is added dropwise at 0°C. The reaction mixture is stirred at 0°C for 6 hours. TLC indicates complete conversion of **10.**

The reaction is quenched by addition of 20 ml 10 wt.% aqueous Na₂SO₃ solution. The aqueous phase is extracted with dichloromethane (3 x 20 ml). The combined organic phase is washed with water (30 ml), dried over magnesium sulfate, and concentrated to dryness *in vacuo.* The crude product is purified by repeated recrystallization in a toluene/heptane solvent mixture to render the final product **11** (monomer **II**) as a slightly yellow solid (1.8 g, yield: 60%).

### Example 3

### Procedures for the synthesis of monomer III

The synthetic sequence for monomer **III** is in analogy to the synthesis of monomer **I** as follows:

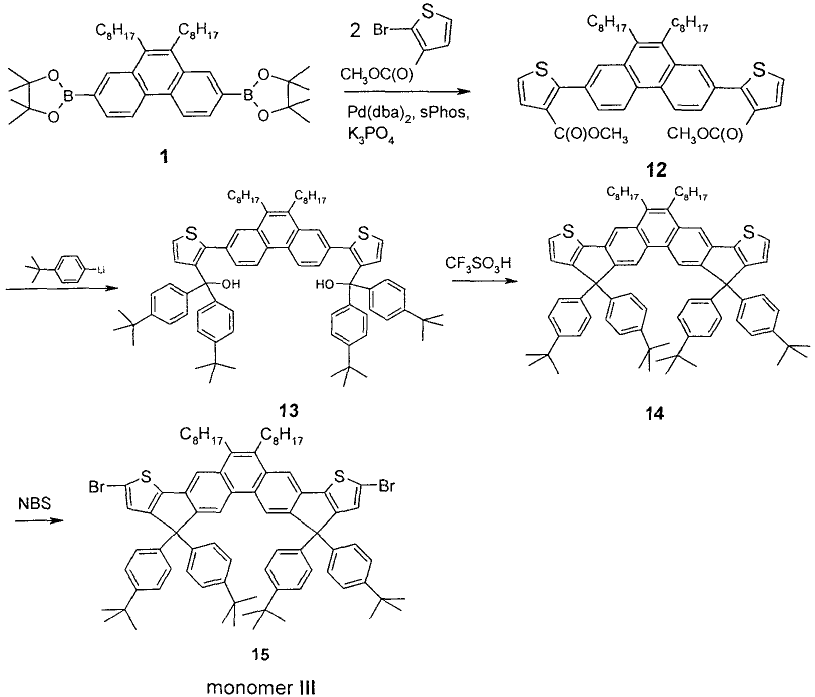

The synthesis of starting material **1** is described in WO2005/104264 A1.

The individual steps in the above synthetic sequence are described below.

### Preparation of 12 via Suzuki coupling of 1 with methyl 2-bromothiophene-3-carboxylate

A 250 ml three-neck flask, equipped with magnetic stirrer, reflux condenser and argon inlet/exhaust, is charged with phenanthrene diboronic ester **1** (13.16 g, 20.0 mmol, 1.0 eq.), methyl 2-bromothiophene-3-carboxylate (13.26 g, 60.0 mmol, 3.0 eq.), potassium phosphate monohydrate (13.80 g, 60.0 mmol, 3.0 eq.), 50 ml toluene and 60 ml water. The resulting suspension is degassed at room temperature by purging argon for 30 min. The solution of catalyst bis(dibenzylideneacetone) palladium(0) (230 mg, 0.4 mmol, 0.02 eq.) and ligand 2-dicyclohexylphosphino-2',6'-dimethoxybiphenyl (sPhos) (328 mg, 0.8 mmol, 0.04 eq.) in 10 ml degassed toluene were added in one go. The reaction mixture is heated to 100°C (oil bath) under rapid stirring. After 18h, TLC indicates complete conversion of 1.

After the reaction mixture has cooled down to room temperature, the aqueous phase is extracted with 2 x 30 ml toluene. The combined toluene phase is passed through a plug of Celite. The filtrate is dried over magnesium sulphate, and then concentrated to ca. 10 ml *in vacuo.* 150 ml methanol is added slowly under stirring. The resulting suspension is stirred at room temperature for 30 min. The precipitated product is filtered, washed with methanol, and dried in vacuo at room temperature to obtain intermediate 12 as a yellow powder (12.4 g, 91%). The product is used in the next step without further purification.

### Arylation of 12

A 500 ml three-neck flask, equipped with magnetic stirrer, dropping funnel and argon inlet/exhaust, is charged with 1-bromo-4-tertbutylbenzene (26.36g, 120 mmol, 12 eq.) and 200 ml anhydrous THF. The resulting solution is cooled to -78°C. n-Butyllithium (2.5 M in hexanes) (52.0 ml, 130 mmol, 13 eq.) is added dropwise via dropping funnel. The reaction mixture is allowed to stir at -50°C for 1 hour. Methyl ester **12** (6.83 g, 10.0 mmol, 1.0 eq.) is added as solid at once. The resulting suspension is stirred at - 50°C for 1h before allowed to warm up slowly to room temperature. After 3 hours, TLC indicates complete conversion of **12.**

The mixture is extracted with ethyl acetate (3 x 20ml). The combined organic phase is washed with water (50 ml), dried over magnesium sulfate, and then concentrated to dryness *in vacuo.* The crude product is purified by column chromatography over silica with heptane:THF (15:1) as elute to render the product 13 as a colorless oil (7.3 g, yield: 63%)

### Ring closure of 13 to obtain 14

The product **13** (6.94 g, 6.0 mmol, 1.0 eq.) is dissolved in 100 ml dry toluene in a 250 ml flask and degassed with nitrogen for 30 minutes. Trifluoromethane sulfonic acid (8.0 ml, 60 mmol, 10.0 eq) is added drop wise over a syringe. The reaction mixture immediately turns to dark green. This mixture was stirred at 80°C for 17 h. TLC indicates complete conversion of **13.**

The reaction mixture is allowed to cool down to room temperature and is then poured into 100 ml water and extracted with toluene (3 x 100 ml). The combined organic phases are dried over magnesium sulphate and the solvent is evaporated under reduced pressure. The crude product is purified by column chromatography in a heptane/ethyl acetate (10:1) mixture to obtain product **14** (4.10 g, yield: 60%).
¹H NMR (500 MHz, Chloroform-d) δ 8.52 (s, 1H), 8.06 (s, 1H), 7.36 (d, *J* = 4.9 Hz, 1H), 7.26 - 7.16 (m, 8H), 7.08 (d, *J* = 4.9 Hz, 1H), 3.17 (dd, *J* = 9.8, 6.7 Hz, 2H), 1.77 - 1.68 (m, 2H), 1.61 (p, *J* = 7.1 Hz, 2H), 1.51 - 1.42 (m, 2H), 1.41 - 1.26 (m, 24H), 0.94 - 0.89 (m, 3H).

### Bromination of 14 to 15

The product **14** (2.5 g, 2.2 mmol, 1.0 eq.) from the previous step is dissolved in 100 ml dry dichloromethane in a 250 ml flask. NBS (0.85 g, 4.8 mmol, 2.2 eq) is added at room temperature in one portion. The solution is stirred for 20 h in the dark. TLC indicates complete conversion of **14.**

The reaction mixture is poured into 100 ml water and extracted with dichloromethane (3 x 150 ml). The combined organic phases are dried over magnesium sulphate and the solvent is evaporated under reduced pressure. The crude product is purified by column chromatography in a heptane/ethyl acetate (20:1) mixture to obtain product **15** (1.70 g, yield: 59%).
¹H NMR (500 MHz, Chloroform-d) δ 8.48 (s, 1H), 7.99 (s, 1H), 7.25 - 7.18 (m, 8H), 7.08 (s, 1H), 3.18 - 3.13 (m, 2H), 1.75 - 1.67 (m, 2H), 1.64 - 1.56 (m, 2H), 1.45 (dt, *J* = 8.8, 6.5 Hz, 2H), 1.41 - 1.27 (m, 24H), 0.94 - 0.91 (m, 3H).

### Example 4

### Procedure for the synthesis of monomer IV: Conversion of dibromide 5 to diboronic ester 16

The following reaction is also a general example how dibromides can be converted into the diboronic esters. This is especially important for the preparation of homopolymers via Suzuki polymerization.

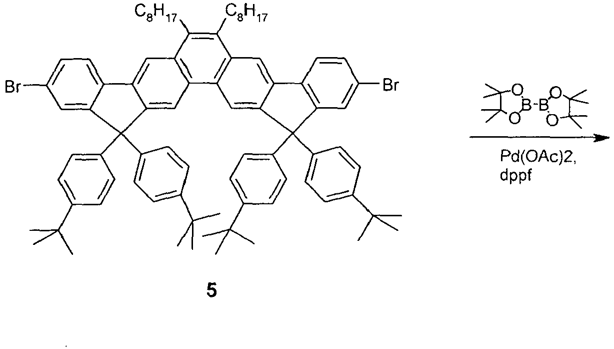

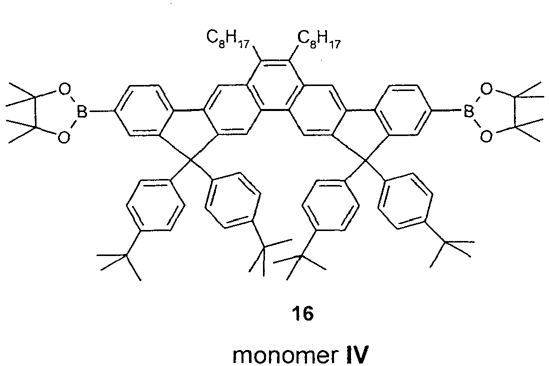

A 100 ml three-neck flask, equipped with magnetic stirrer, reflux condenser and argon inlet/exhaust, is charged with bisindenophenanthrene dibromide **5** (2.53 g, 2.0 mmol, 1.0 eq.), bis(pinacolato)diborane (1.52 g, 6.0 mmol, 3.0 eq.), potassium acetate (1.09 g, 11.0 mmol, 5.5 eq.) and 20 ml anhydrous THF. The resulting suspension is degassed at room temperature by purging argon for 30 min. The catalyst [1,1'-bis(diphenylphosphino)ferrocene]dichloropalladium(II) (29.3 mg, 0.04 mmol, 0.02 eq.) is added. The mixture is heated to reflux under rapid stirring. After 48h, TLC indicates complete conversion of **5.**

The mixture is allowed to cool down to room temperature, filtered through a plug of Celite, and then washed with THF (2 x 20 ml). The filtrate is concentrated to *in vacuo.* The obtained dark brown oil is purified by column chromatography over silica with toluene as elute. To achieve the required purity, the crude product is purified by repeated recrystallization in a toluene/heptane solvent mixture to obtain the final product **16** (monomer **IV**) as off-white solid (1.6 g, yield: 58%).

### Polymer examples

Conjugated copolymers are prepared from the monomers as described above and / or other desired co-monomers by Suzuki polymerization in accordance with the procedure disclosed in WO 2003/048225.

### Example 5

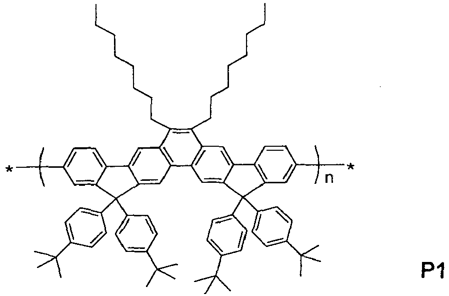

Polymer **P1** is prepared from monomer **I** (50 mol.%) and monomer **IV** (50 mol.%) by Suzuki polymerization according to the following procedure:
A 100 ml three-neck flask, equipped with mechanic stirrer, reflux condenser and argon inlet/exhaust, is charged with monomer **I** (1.265 g, 1.0 mmol, 1.0 eq.), monomer **IV** (1.360 g, 1.0 mmol, 1.0 eq.), potassium phosphate monohydrate (1.012 g, 4.4 mmol, 4.4 eq.), 10 ml of dioxane, 10 ml toluene and 10 ml H₂O. The resulting solution is degassed by purging with Argon for 30 min. The solution is heated to gentle reflux and the polymerization is started by adding 1 ml solution of palladiumacetate (0.45 mg, 2 µmol, 0.002 eq.) and tris-o-tolylphosphine palladiumacetate (3.65 mg, 12 µmol, 0.012 eq.) in dioxane/toluene. The reaction mixture is refluxed for 2 hours until the target viscosity is reached. 0.06 ml bromobenzene is then added and refluxed for another hour to stop the polymerization. The reaction mixture is cooled down to 65°C. 20 ml toluene and a 10 ml 10% sodium diethyldithiocarbaminate aqueous solution are added, and the mixture is stirred at 65°C for 3 hours.
The reaction mixture is cooled down to room temperature. The toluene phase is washed with water. The polymer is precipitated in methanol and filtered. The polymer is purified by repeated precipitation from its solution in toluene into methanol. The purified polymer is filtered and dried in vacuo.

### Example 6

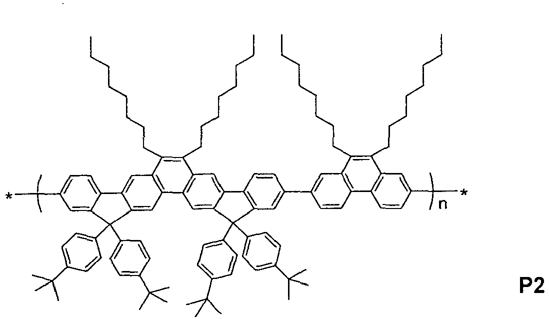

Polymer **P2** is prepared from monomers **I** (50 mol.%) and 9,10-dioctyl-phenanthrene diboronic ester monomer (50 mol.%) in analogy to the method described in Example 5. 9,10-dioctyl-phenanthrene diboronic ester monomer can be prepared as described in WO2005/104264 A1. The molecular weight of the obtained polymer is determined by gel permeation chromatography (GPC), M_{w} = 366,000 g/mol, Mₙ = 140,000 g/mol.

### Example 7

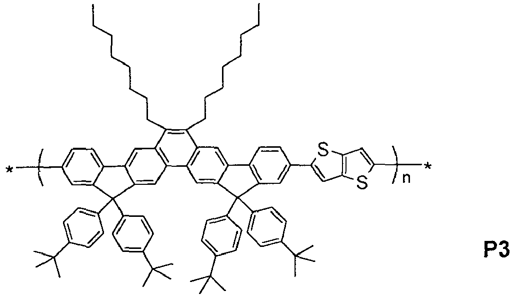

Polymer **P3** is prepared from monomers **I** (50 mol.%) and 2,5-bis(trimethylstannyl)thienothiophene monomer (50 mol.%) by Stille polymerization according to the procedure described below. 2,5-bis(trimethylstannyl)thienothiophene monomer can be prepared as described in the literature.

A microwave vial is charged with monomer **I** (379.6 mg, 0.3 mmol, 1.0 eq.), 2,5-bis(trimethylstannyl)thienothiophene (139.7m g, 0.3 mmol, 1.0 eq.), bis(dibenzylideneacetone) palladium(0) (3.5 mg, 0.006 mmol, 0.02 eq.), tri-o-tolylphosphine (3.65 mg, 0.024 mmol, 0.08 eq.), 7.5 ml toluene and 1.5 ml DMF. The resulting solution is degassed by purging with Ar for 30 min. The solution is heated to 110°C in a microwave reactor for 60 mins.

The reaction mixture is allowed to cool down to room temperature. 10 ml toluene is added, and the solution is transferred into a three-neck flask and degassed by purging with Ar for 30 min. 92 mg bromobenzene, bis(dibenzylideneacetone) palladium(0) (3.5 mg, 0.006 mmol, 0.02 eq.) and tri-o-tolylphosphine (3.65 mg, 0.024 mmol, 0.08 eq.) is then added and refluxed at 110°C (oil bath) for one hour. 323 mg tributylstannyl benzene is added and refluxed at 110°C (oil bath) for another hour. The reaction mixture is cooled down to 65°C. 50 ml 10% sodium diethyldithiocarbaminate aqueous solution are added and the mixture is stirred at 65°C for 3 hours.

The reaction mixture is cooled down to room temperature. The toluene phase is washed with water. The polymer is precipitated in methanol and filtered. The polymer is purified by repeated precipitation from its solution in toluene into methanol. The purified polymer is filtered and dried in vacuo. Molecular weight of the obtained polymer is determined by GPC, M_{w} = 200,000 g/mol, Mₙ = 69,000 g/mol.

### Example 8

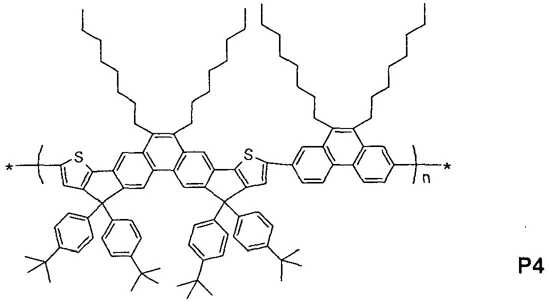

Polymer **P4** is prepared from monomers **III** (50 mol.%) and 9,10-dioctyl-phenanthrene diboronic ester monomer (50 mol.%) in analogy to the method described in Example 5. 9,10-dioctyl-phenanthrene diboronic ester monomer can be prepared as described in WO2005/104264 A1.

### Example 9

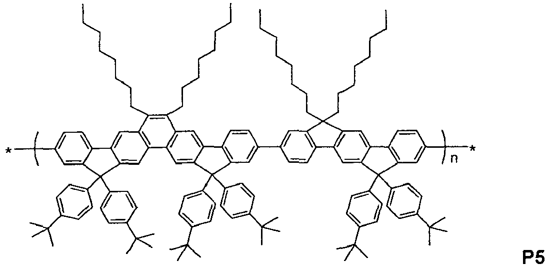

Polymer **P5** is prepared from monomers **I** (50 mol.%) and 6,6-bis-(4-tert-butyl-phenyl)-12,12-dioctyl-indenofluorene diboronic ester monomer (50 mol.%) in analogy to the method described in Example 5. 6,6-bis-(4-tert-butyl-phenyl)-12,12-dioctyl-indenofluorene diboronic ester onomer can be prepared as described in WO2003/048225 A1. The molecular weight of the obtained polymer is determined by GPC, M_{w} = 170,000 g/mol, Mₙ = 68,000 g/mol.

### Example 10: FET Device fabrication and characterization

Top-gate thin-film organic field-effect transistors (OFETs) are fabricated as follows. High quality transistorgrade glass is cleaned in Decon90 at T > 65 °C for 30min. The glass is then sonicated repeatatively in water and methanol water at T > 65 °C. The substrated is then spin dried with methanol for 1 min. To this clean substrate gold source drain electrodes with thickness of ca. 35 nm are thermally evaporated through a shadow mask which has corresponding gaps for devices with desired channel length of 50 µm and width of 1000 µm, respectively. Directly after the electrode deposition a self assempled monolayer (SAM) treatment is casted to enable ohmic contact between the electrode surface and the polymer. Directly after SAM treatment, the organic semiconductor layer is deposited by spin-coating ca. 1 wt. % solution of polymer in a suitable organic solvent and subsequently annealed at 100 °C for 5 mins. Subsequently the dielectric material D139 is spin-coated on top of the organic semiconductor. Finally a gold gate electrode with a thickness of ca. 35 nm is deposited.

The electrical characterization of the transistor devices is carried out in ambient air atmosphere. Field-effect mobilities are extracted in the linear regime from the slope of the source-drain current in the linear regime versus the gate valtage, and in the saturation regime are calculated from the linear fit of the source-drain current in the saturation regime versus the gate voltage. The results are summarized in Table 1 below.

**Table 1 Transistor characterization**

| polymer example | lin. mobility (cm²/Vs) | sat. mobility (cm²/Vs) |
|---|---|---|
| P2 | 0.005 | |
| P3 | 0.009 | 0.013 |
| P4 | | |
| P5 | 0.033 | 0.081 |

## Claims

1. A compound, **characterized in that** it is a polymer with a conjugated backbone comprising more than one units of formula I 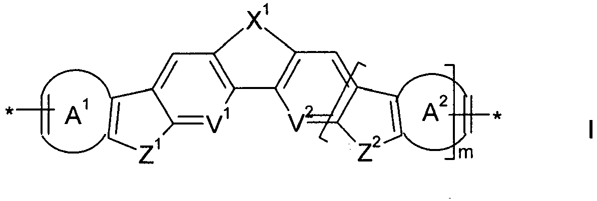 wherein
X¹ is CR¹=CR² or CR¹R²-CR¹R²,
V¹ and V² denote CH,
Z¹ and Z² independently of each other denote S, O or CR³R⁴,
A¹ and A² independently of each other denote a mono- or bicyclic aromatic or heteroaromatic group that is selected from formulae (2a), (3a), (6a), (7a), (10a) and (13a), 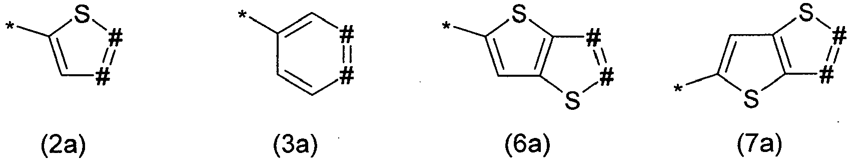 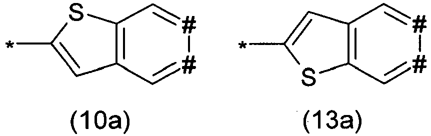 wherein the individual aromatic and heteroaromatic rings can also be substituted by one or more groups R¹,
R¹, R², R³ and R⁴ are independently of each other selected from straight-chain, branched or cyclic alkyl or alkoxy with 1 to 30 C atoms which is unsubstituted or substituted by one or more F atoms, or aryl or heteroaryl, each of which is unsubstituted or substituted and has 4 to 30 ring atoms,
m is 0 or 1,
the * indicates the bonds that link the unit either with a polymer or other optoelectronically functional groups and the sign # in a ring denotes a C atom that is fused to the adjacent five ring in formula I.

2. The compound according to claim 1, **characterized in that** in the units of formula I are selected from the following formulae 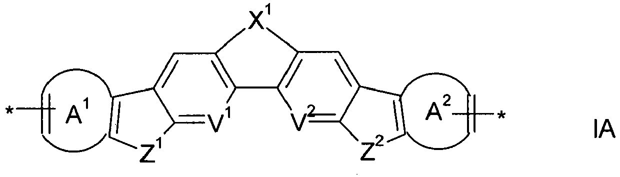 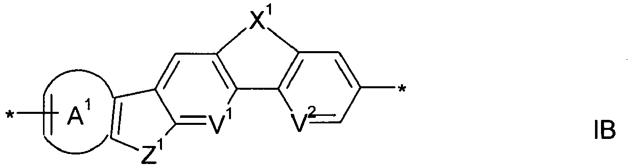 wherein X¹, A¹, A², V¹, V², Z¹ and Z² are as defined in claim 1.

3. The compound according to claim 1 or 2, **characterized in that** the unit of formula I is selected from the following subformulae: 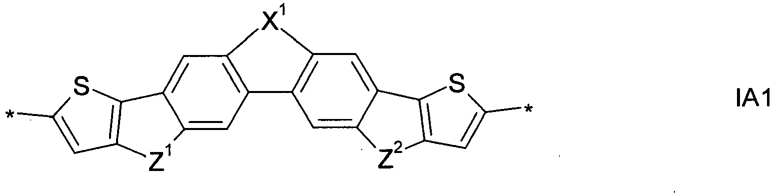 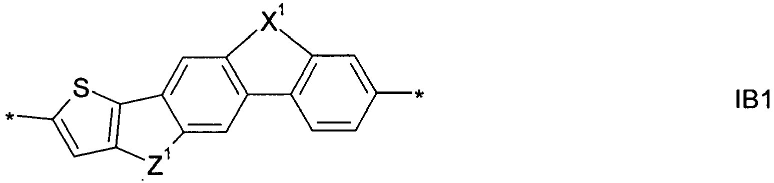 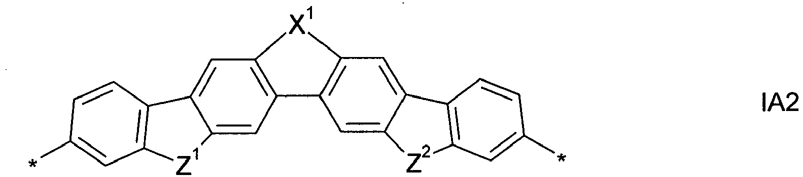 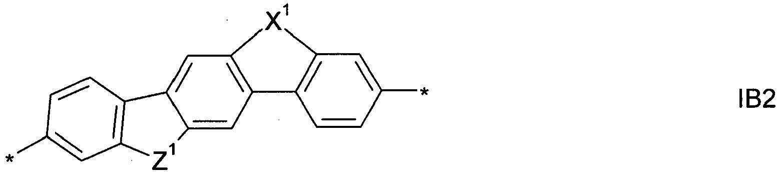 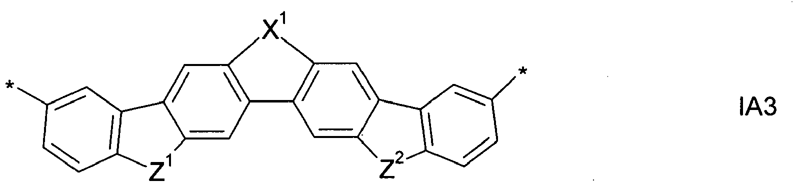 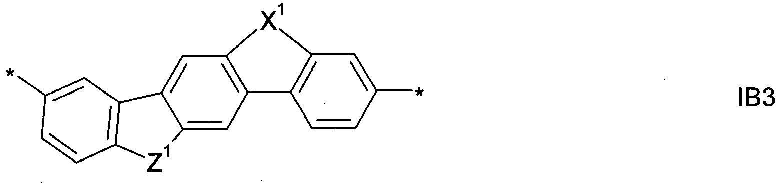 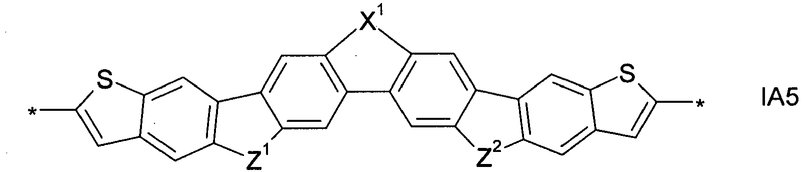 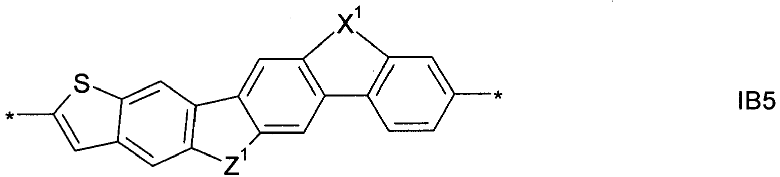 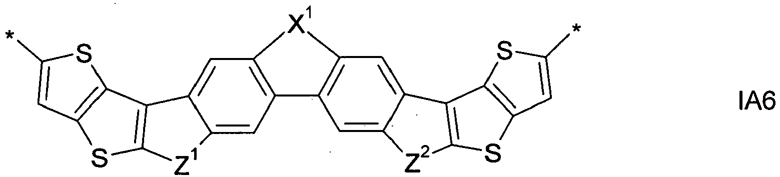 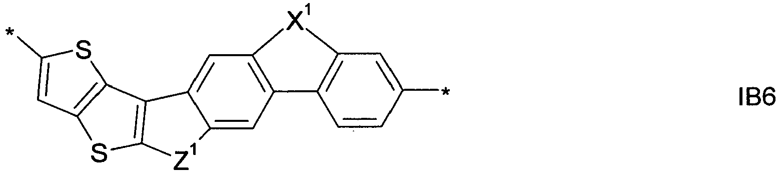 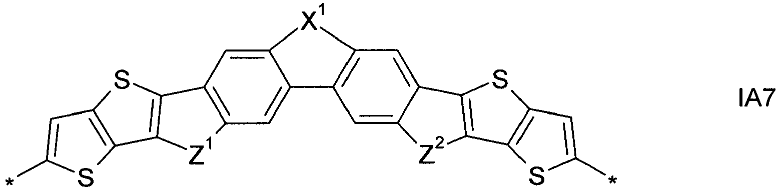 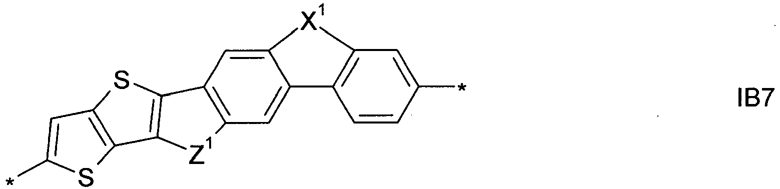 wherein X¹, Z and Z² are as defined in claim 1.

4. The compound according to one or more of claims 1 to 3, **characterized in that** the unit of formula I is selected from the following subformulae: 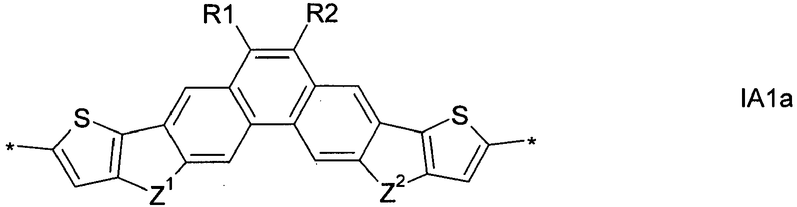 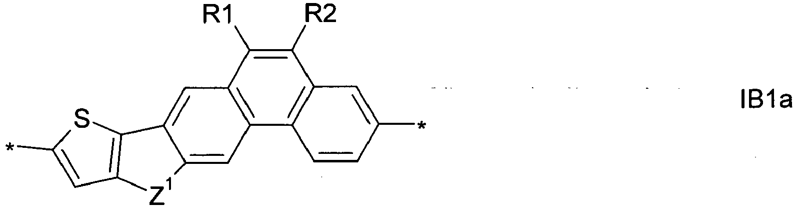 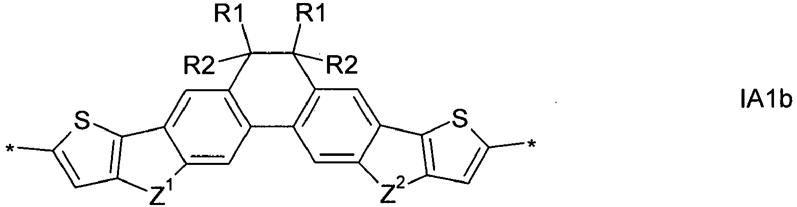 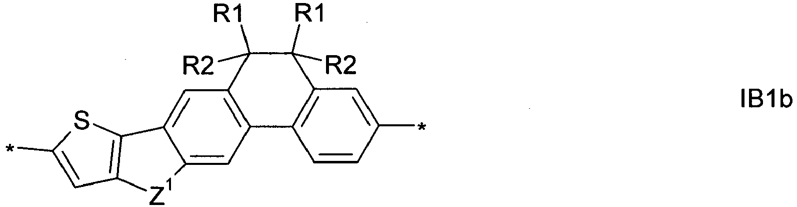 wherein R¹, R², Z¹ and Z² are as defined in claim 1.

5. The compound according to one or more of claims 1 to 4, **characterized in that** the unit of formula I is selected from the following subformulae: 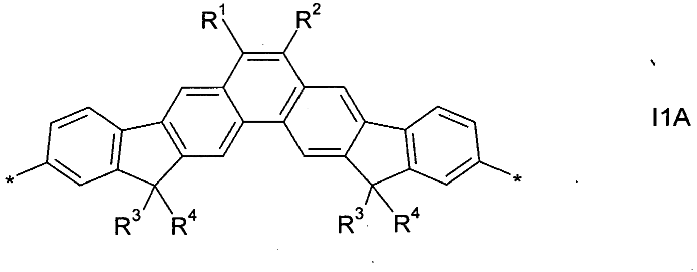 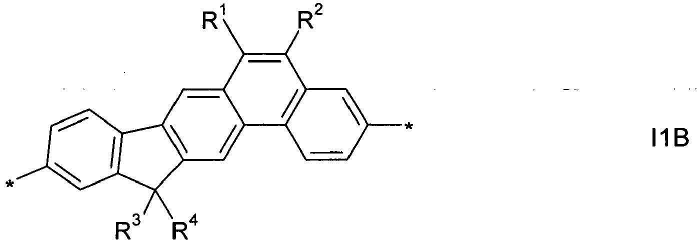 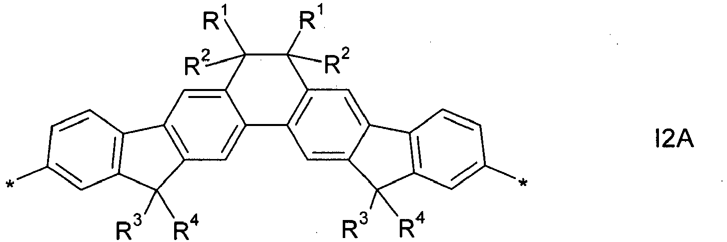 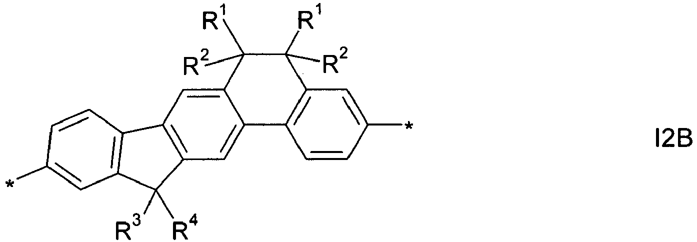 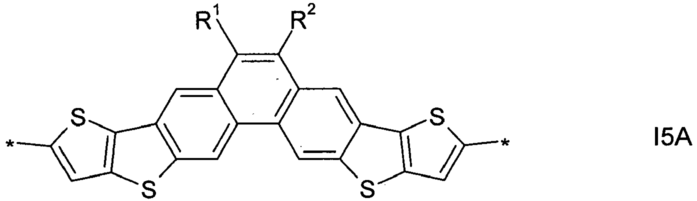 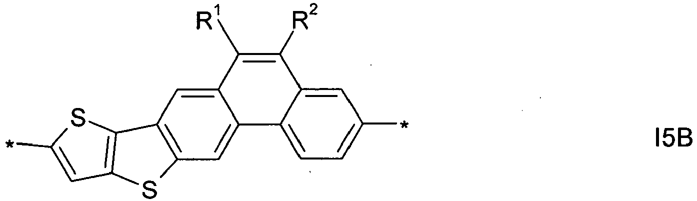 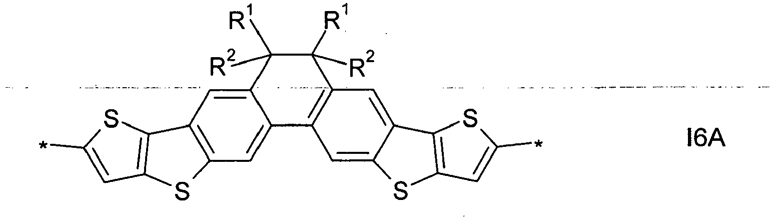 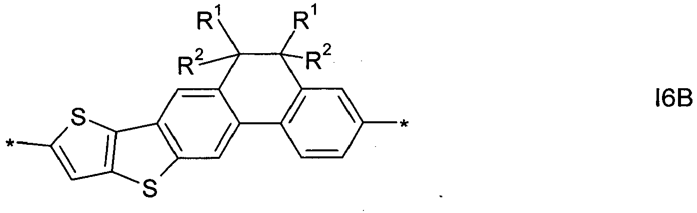 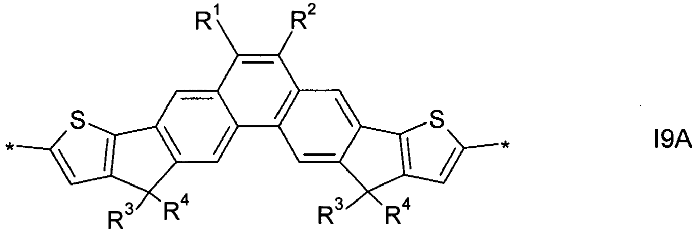 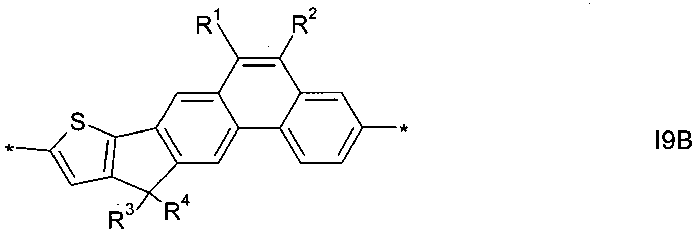 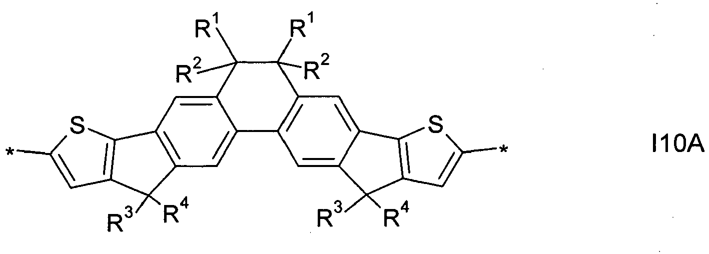 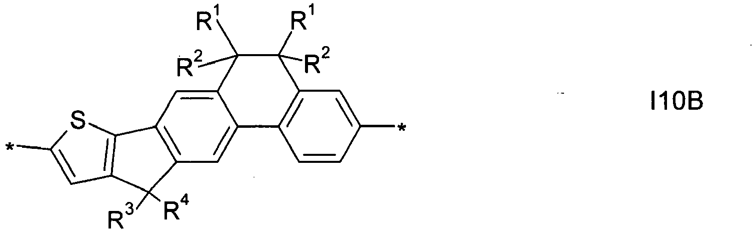 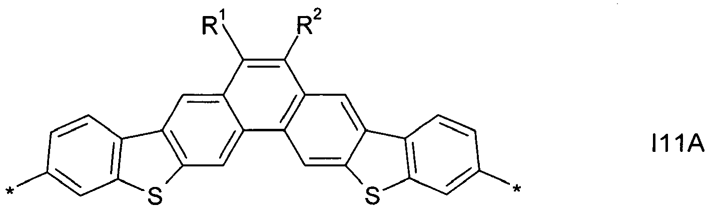 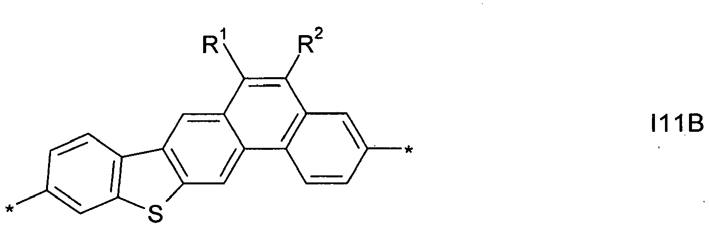 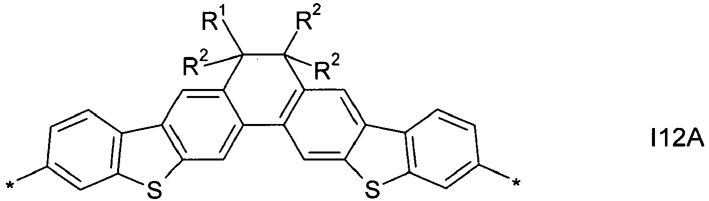 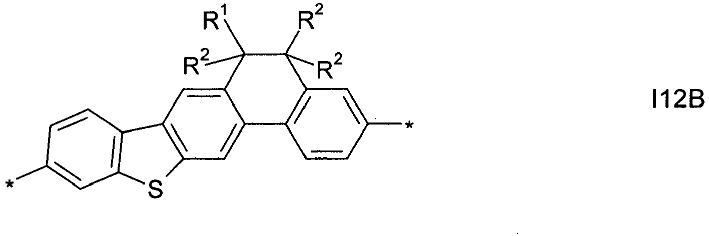 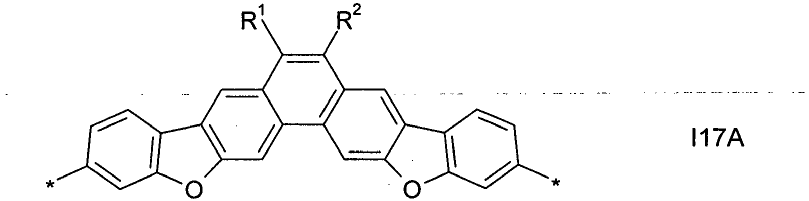 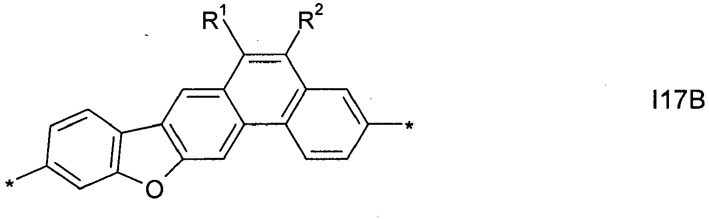 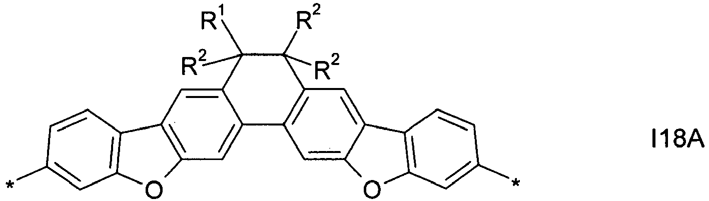 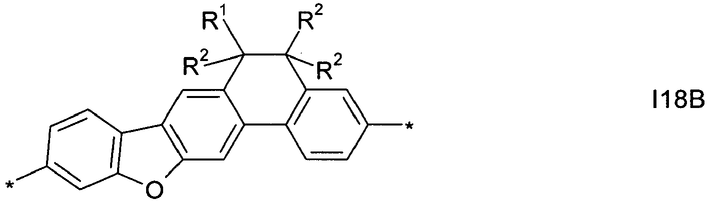 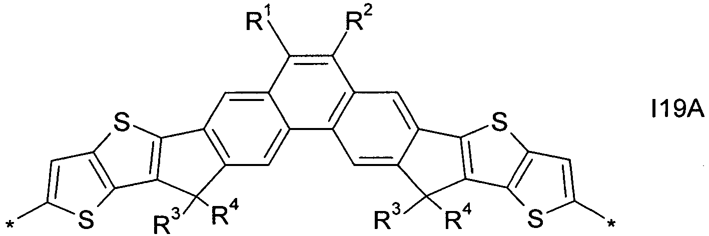 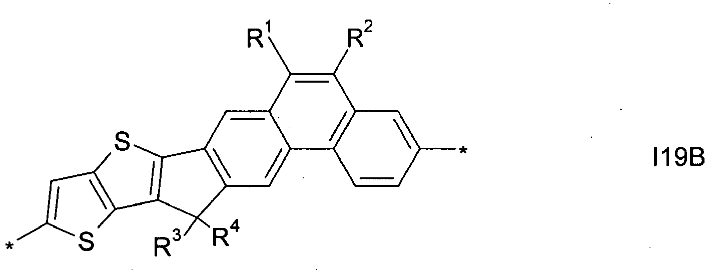 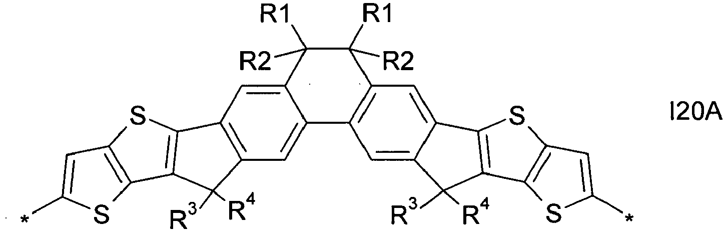 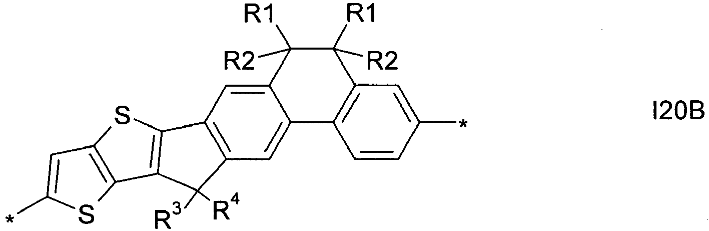 wherein R^{1,2,3,4} have the meanings given in claim 1, and wherein the outer benzene rings can also be substituted by one or two groups R¹.

6. The compound according to one or more of claims 1 to 5, **characterized in that** R¹ and R² denote straight-chain, branched or cyclic alkyl or alkoxy with 1 to 30 C atoms which is optionally fluorinated, and R³ and R⁴ denote aryl, aryloxy, heteroaryl or heteroaryloxy, each having from 4 to 30 ring atoms and optionally being fluorinated, alkylated,fluoroalkylated or alkoxylated.

7. The compound according to one or more of claims 1 to 6, **characterized in that** it is a conjugated polymer comprising one or more units of formula I as defined in one or more of claims 1 to 6, and further comprising one or more aryl or heteroaryl units that are optionally substituted.

8. The compound according to claim 1 or 7, **characterized in that** it is selected of formula II
*-[(Ar¹)ₐ-(U)_{b}-(Ar²)_{c}-(Ar³)_{d}]ₙ-* II
wherein
U is a unit of formula I, IA, IB or its subformulae as defined in one or more of claims 1 to 6,
Ar¹, Ar², Ar³ on each occurrence identical or different, and independently of each other, denote aryl or heteroaryl that is different from U, preferably has 5 to 30 ring atoms, and is optionally substituted by one or more groups R¹ to R⁴ as defined in claim 1 or 6,
a, c, d are on each occurrence identical or different 0 or an integer from 1 to 10,
b is an integer from 1 to 10,
n is an integeral >1,
wherein the polymer comprises at least one repeating unit -[(Ar¹)ₐ-(U)_{b}-(Ar²)_{c}-(Ar³)_{d}]- wherein b is at least 1,
and wherein the * indicates the bonds that link the unit either with a polymer or functional groups.

9. The compound of claim 8, **characterized in that** it is selected of formula III
R⁵-[(Ar¹)ₐ-(U)_{b}-(Ar²)_{c}-(Ar³)_{d}]ₙ-R⁶ III
wherein Ar¹, Ar², Ar³, U, a, b, c, d and n are as defined in claim 8, R⁵ and R⁶ denote independently of each other, H, F, Br, Cl, I, -CH₂Cl, - CHO, -CR'=CR"₂, -SiR'R"R''', -SiR'X'X", -SiR'R"X', -SnR'R"R''', - BR'R", -B(OR')(OR"), -B(OH)₂, -O-SO₂-R', -C≡CH, -C≡C-SiR'₃, -ZnX' or an endcap group, X' and X" denote halogen, R', R" and R'" have independently of each other one of the meanings of R⁰, and two of R', R" and R''' may also form a ring together with the hetero atom to which they are attached and R⁰ is H, optionally substituted C₁₋₄₀ carbyl or hydrocarbyl, or optionally substituted aryl or heteroaryl, and preferably denote alkyl with 1 to 12 C-atoms, or aryl or heteroaryl with 5 to 12 C-atoms, very preferably methyl, tert-butyl, phenyl, tolyl or tert-butyl-phenyl.

10. A monomer of formula IV
R⁷-(Ar¹)ₐ-U-(Ar²)_{c}-R⁸ IV
wherein U, Ar¹, Ar², a and c have the meanings given in claim 8, and R⁷ and R⁸ are independently of each other selected from the group consisting of CI, Br, I, O-tosylate, O-triflate, O-mesylate, O-nonaflate, -SiMe₂F, -SiMeF₂, -O-SO₂Z¹, -B(OZ²)₂ , -CZ³=C(Z³)₂, -C≡CH, - C≡CSi(Z¹)₃, -ZnX⁰ and -Sn(Z⁴)₃, wherein X⁰ is halogen, preferably Cl, Br or I, Z¹⁻⁴ are selected from the group consisting of alkyl and aryl, each being optionally substituted, and in -B(OZ²)₂ two groups Z² may also together form a cyclic group.

11. A process of preparing a conjugated polymer according to one or more of claims 1 to 9, by coupling one or more monomers according to claim 10, wherein R⁷ and R⁸ are selected from Cl, Br, I, -B(OZ²)₂ and -Sn(Z⁴)₃, with each other and/or with one or more monomers selected from the following formulae
R⁷-(Ar¹)ₐ-(Ar)²_{c}-(Ar³)_{d}-R⁸ V
R⁷-Ar¹-R⁸ VI
R⁷-Ar²-R⁸ VII
R⁷-Ar³-R⁸ VIII
wherein Ar¹, Ar², Ar³, a, c and d have one of the meanings given in claim 8, wherein a+c+d≥2, and R⁷ and R⁸ are selected from Cl, Br, I, -B(OZ²)₂ and -Sn(Z⁴)₃, in an aryl-aryl coupling reaction

12. Use of a compound according to one or more of claims 1 to 9 as electron donor or p-type semiconductor in a semiconducting material, formulation, polymer blend, device or component of a device.

13. Use of a compound according to one or more of claims 1 to 9 in a hole transport, electron transport, electron blocking, hole blocking, charge generation, conducting, semiconducting or light emitting material.

14. A semiconducting material, formulation, mixture, polymer blend, device or component of a device, comprising a compound according to one or more of claims 1 to 9 as electron donor component,

15. A semiconducting material, formulation, mixture, polymer blend, device or component of a device according to claim 14, further comprising one or more compounds having electron acceptor properties.

16. A mixture or polymer blend comprising one or more compounds according to one or more of claims 1 to 9, and further comprising one or more compounds having one or more of semiconducting, charge transport, hole or electron transport, hole or electron blocking, electrically conducting, photoconducting or light emitting properties.

17. A formulation comprising one or more compounds, mixtures or polymer blends according to one or more of claims 1 to 9 and 16, and further comprising one or more solvents selected from organic solvents.

18. An organic semiconducting formulation comprising one or more compounds according to one or more of claims 1 to 9, one or more organic binders or precursors thereof, and optionally one or more solvents.

19. A charge transport, semiconducting, electrically conducting, photoconducting or light emitting material comprising a compound, polymer, formulation, mixture or polymer blend according to one or more of claims 1 to 9 and 16 to 18.

20. Use of a compound, polymer, formulation, mixture or polymer blend according to one or more of claims 1 to 9, and 16 to 18 as charge transport, semiconducting, electrically conducting, photoconducting or light emitting material, or in an optical, electrooptical, electronic, electroluminescent or photoluminescent device, or in a component of such a device or in an assembly comprising such a device or component.

21. An optical, electrooptical, electronic, electroluminescent or photoluminescent device, or a component thereof, or an assembly comprising it, which comprises a compound, polymer, formulation, mixture or polymer blend according to to one or more of claims 1 to 9 and 16 to 18.

22. The optical, electrooptical, electronic, electroluminescent or photoluminescent device according to claim 21, which is an organic field effect transistor (OFET), organic thin film transistor (OTFT), organic light emitting diode (OLED), organic light emitting transistor (OLET), organic photovoltaic device (OPV), organic photodetector (OPD), organic solar cell, laser diode, Schottky diode, or organic photoconductor.

23. The component according to claim 21, which is a charge injection layer, charge transport layer, interlayer, planarising layer, antistatic film, polymer electrolyte membrane (PEM), conducting substrate or conducting pattern.

24. The assembly according to claim 21, which is an integrated circuit (IC), radio frequency identification (RFID) tag, security marking, security device, flat panel display, backlight, electrophotographic device, electrophotographic recording device, organic memory device, sensor device, biosensor or biochip.

## Patentansprüche

1. Verbindung, **dadurch gekennzeichnet, dass** es sich um ein Polymer mit einem konjugierten Rückgrat mit mehr als einer Einheiten der Formel I 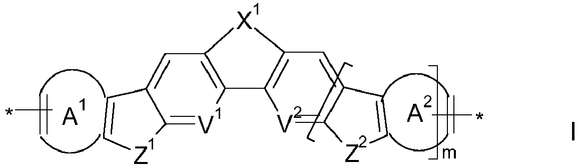 handelt, bei der
X¹ CR¹=CR² oder CR¹R²-CR¹R² ist,
V¹ und V² CH bedeuten,
Z¹ und Z² unabhängig voneinander S, O oder CR³R⁴ bedeuten,
A¹ und A² unabhängig voneinander eine mono- oder bicyclische aromatische oder heteroaromatische Gruppe bedeuten, die aus den Formeln (2a), (3a), (6a), (7a), (10a) und (13a) 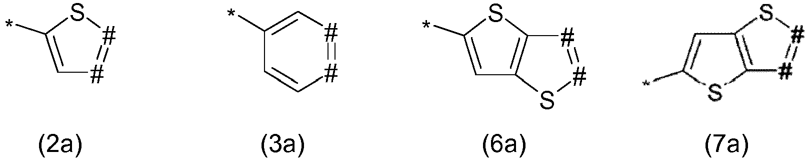 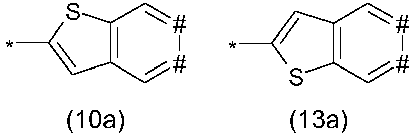 ausgewählt ist, bei denen die einzelnen aromatischen und heteroaromatischen Ringe auch durch eine oder mehrere Gruppen R¹ substituiert sein können,
R¹, R², R³ und R⁴ unabhängig voneinander aus geradkettigem, verzweigtem oder cyclischem Alkyl oder Alkoxy mit 1 bis 30 C-Atomen, das unsubstituiert oder durch ein oder mehrere F-Atome substituiert ist, oder Aryl oder Heteroaryl, das jeweils unsubstituiert oder substituiert ist und 4 bis 30 Ringatome aufweist, ausgewählt sind,
m 0 oder 1 ist,
das * die Bindungen angibt, welche die Einheit entweder mit einem Polymer oder anderen optoelektronisch funktionellen Gruppen verknüpfen, und das #-Zeichen in einem Ring ein C-Atom bedeutet, das mit dem benachbarten Fünfring in Formel I anelliert ist.

2. Verbindung nach Anspruch 1, **dadurch gekennzeichnet, dass** in den Einheiten der Formel I aus den folgenden Formeln ausgewählt sind 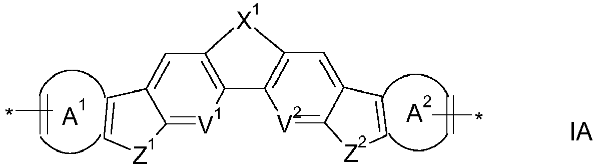 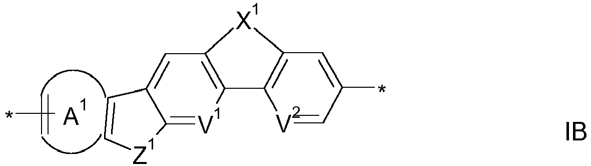 bei denen X¹, A¹, A², V¹, V², Z¹ und Z² wie in Anspruch 1 definiert sind.

3. Verbindung nach Anspruch 1 oder 2, **dadurch gekennzeichnet, dass** die Einheit der Formel I aus den folgenden Unterformeln ausgewählt ist: 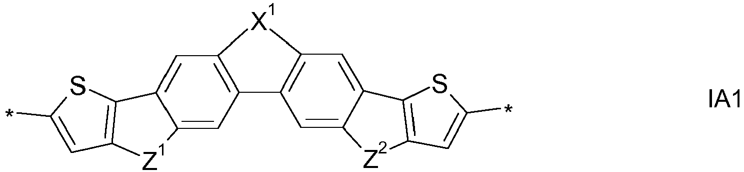 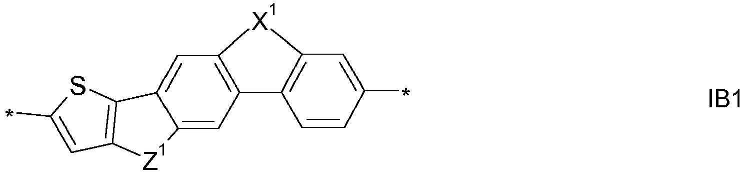 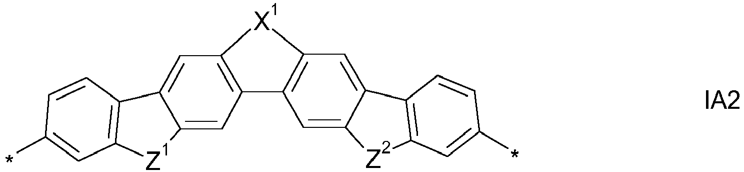 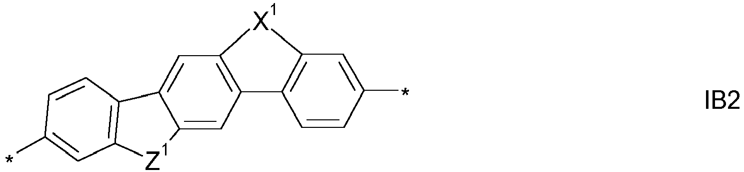 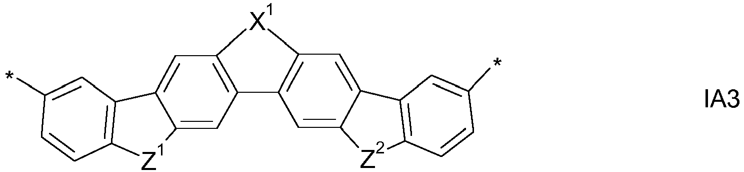 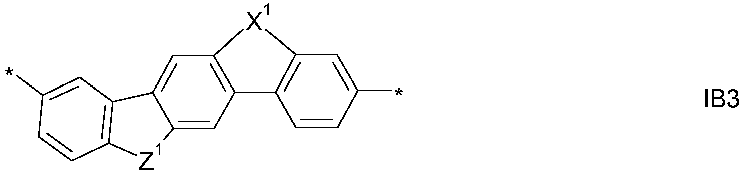 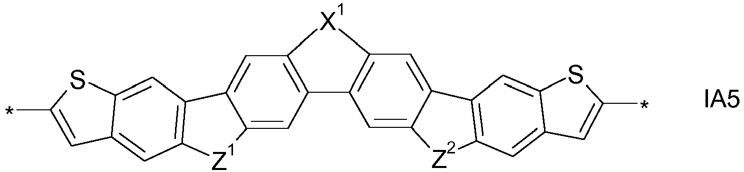 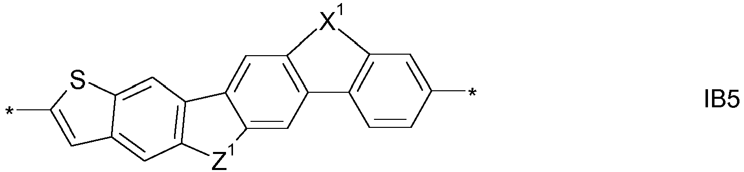 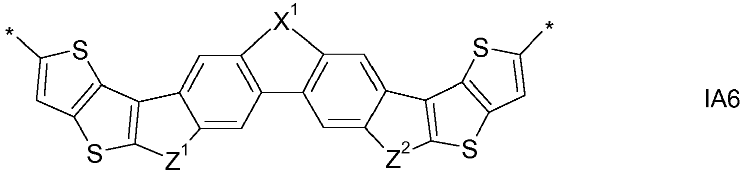 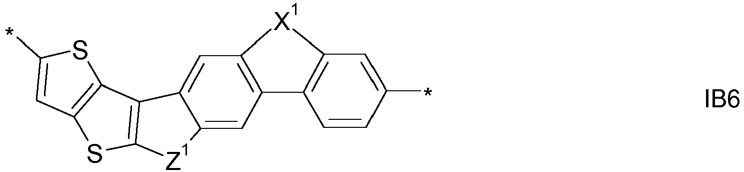 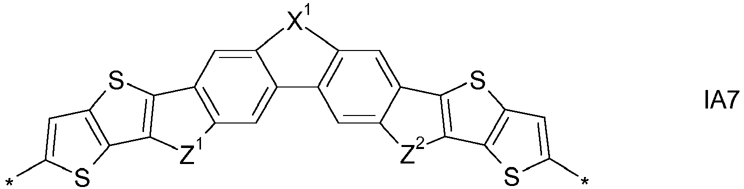 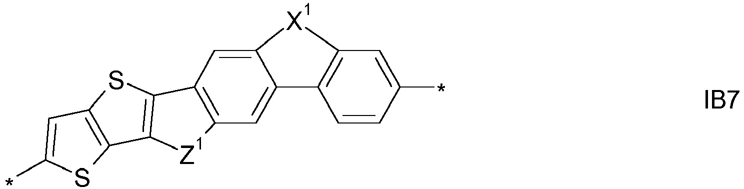 bei denen X¹, Z und Z² wie in Anspruch 1 definiert sind.

4. Verbindung nach einem oder mehreren der Ansprüche 1 bis 3, **dadurch gekennzeichnet, dass** die Einheit der Formel I aus den folgenden Unterformeln ausgewählt ist: 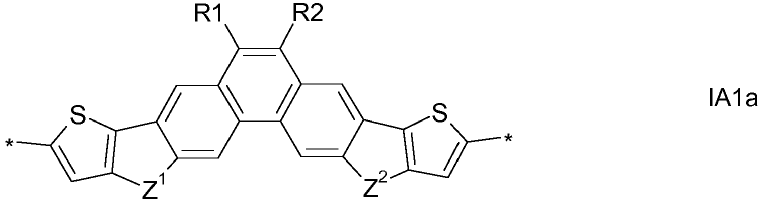 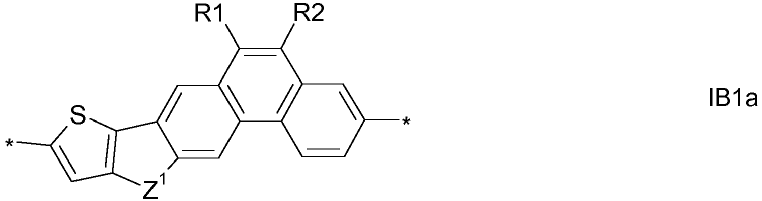 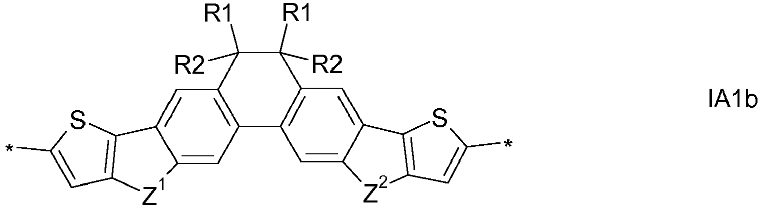 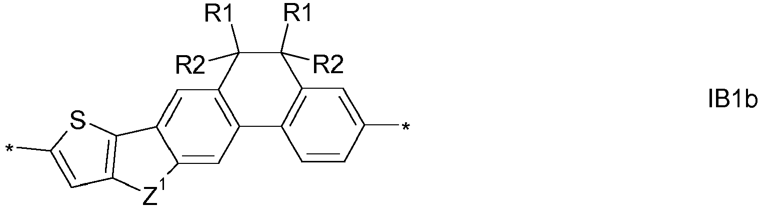 bei denen R¹, R², Z¹ und Z² wie in Anspruch 1 definiert sind.

5. Verbindung nach einem oder mehreren der Ansprüche 1 bis 4, **dadurch gekennzeichnet, dass** die Einheit der Formel I aus den folgenden Unterformeln ausgewählt ist: 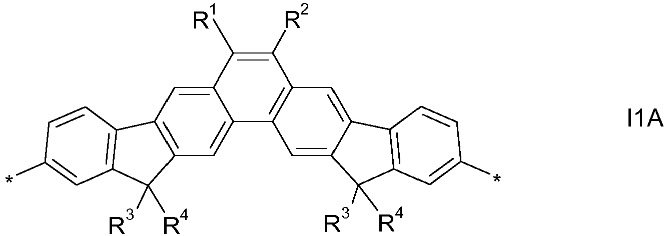 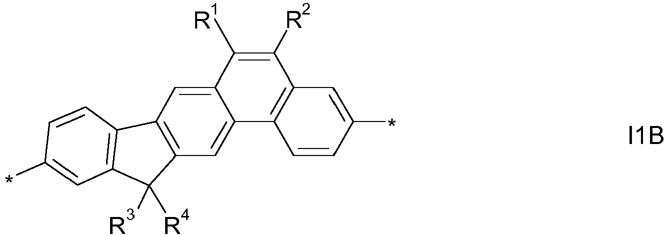 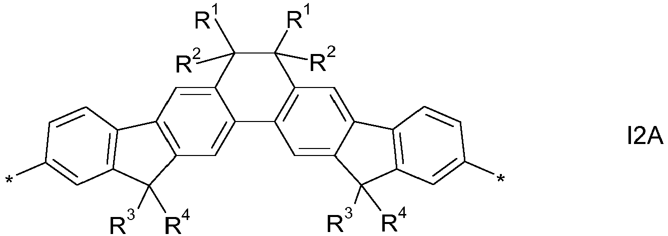 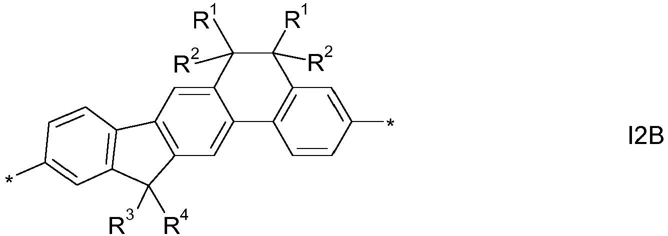 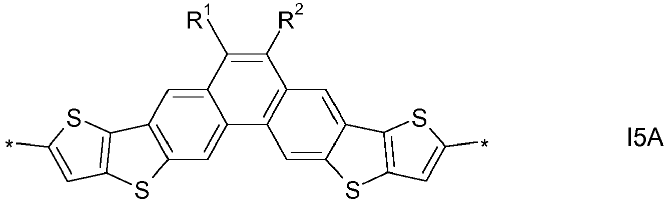 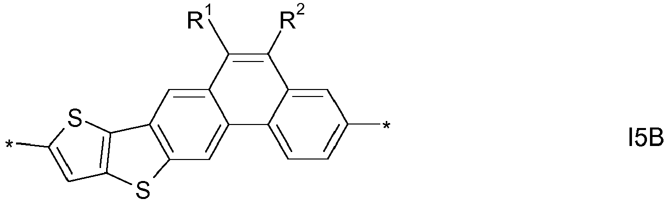 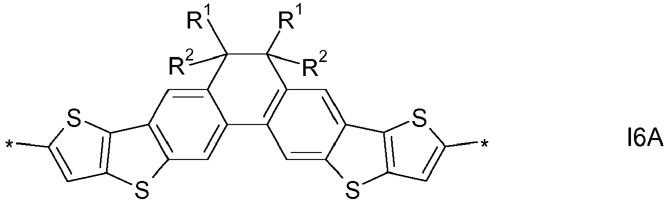 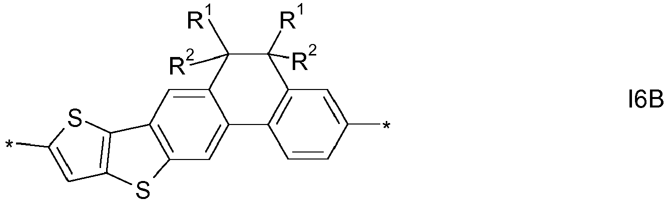 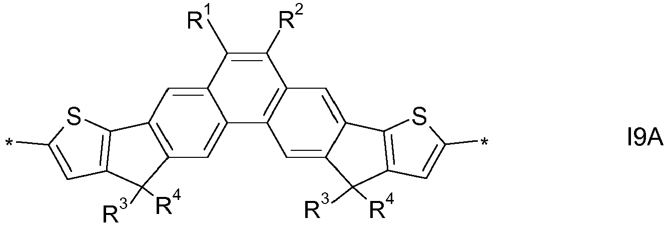 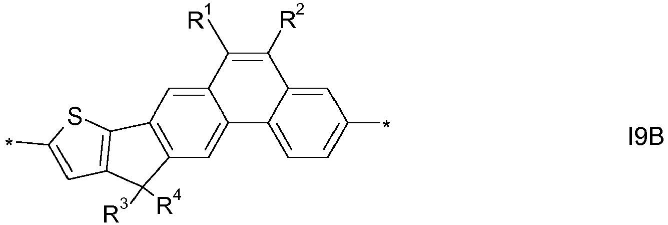 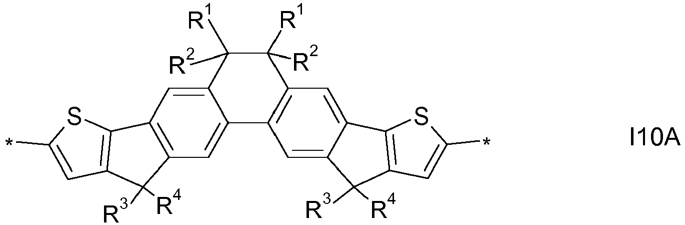 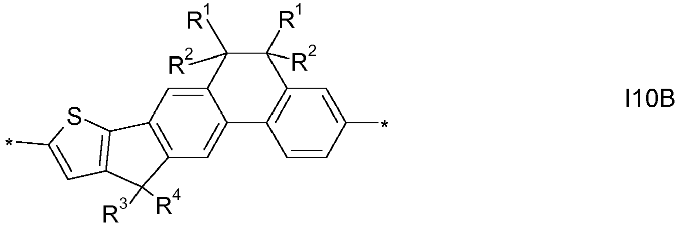 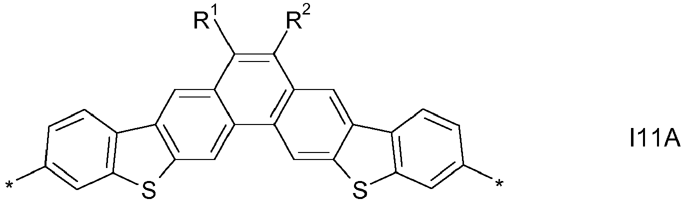 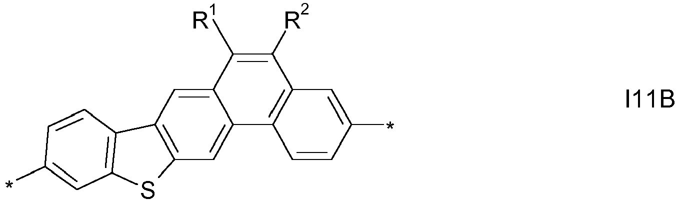 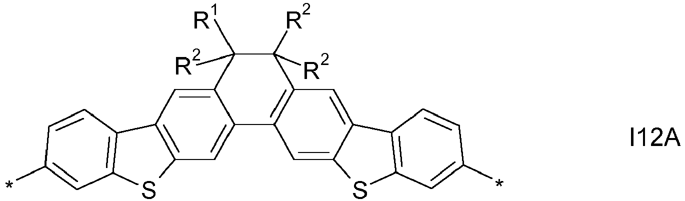 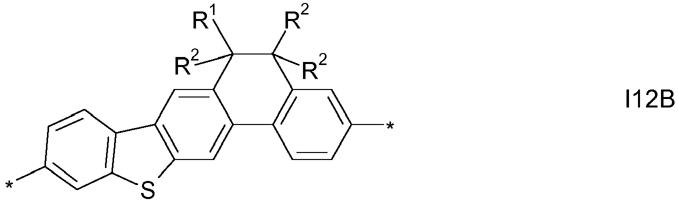 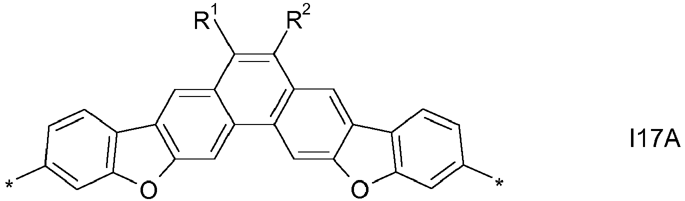 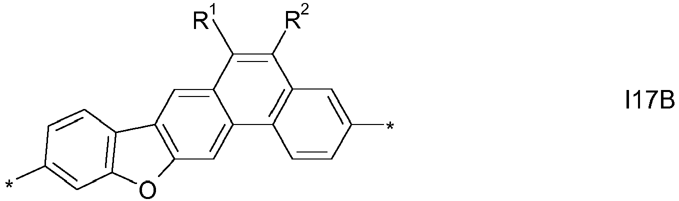 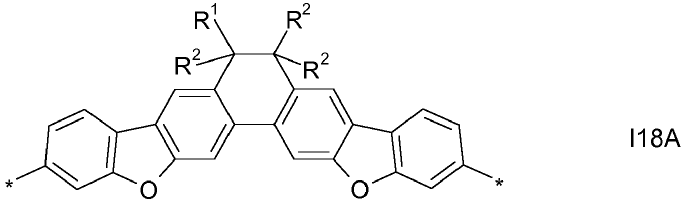 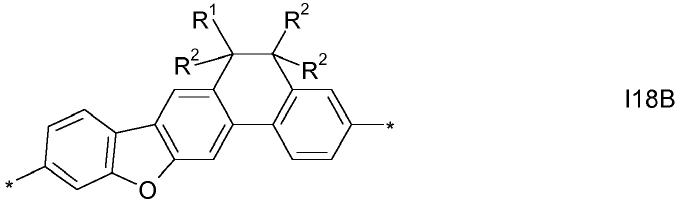 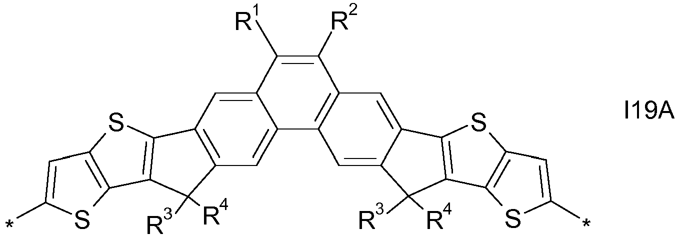 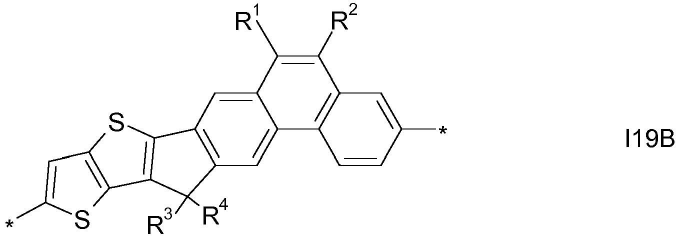 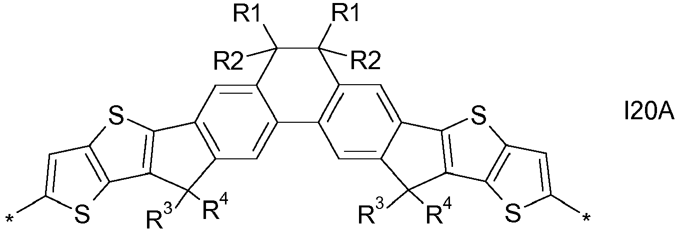 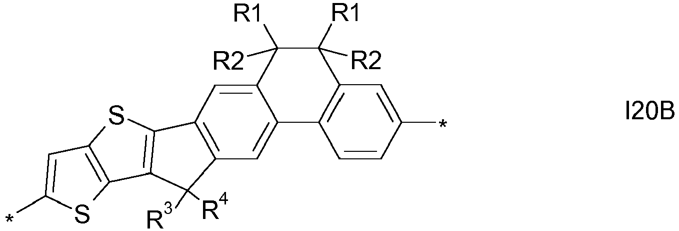 bei denen R^{1,2,3,4} die in Anspruch 1 angegebenen Bedeutungen besitzen und bei denen die äußeren Benzolringe auch durch eine oder zwei Gruppen R¹ substituiert sein können.

6. Verbindung nach einem oder mehreren der Ansprüche 1 bis 5, **dadurch gekennzeichnet, dass** R¹ und R² geradkettiges, verzweigtes oder cyclisches Alkyl oder Alkoxy mit 1 bis 30 C-Atomen bedeuten, das gegebenenfalls fluoriert ist, und R³ und R⁴ Aryl, Aryloxy, Heteroaryl oder Heteroaryloxy bedeuten, die jeweils 4 bis 30 Ringatome aufweisen und gegebenenfalls fluoriert, alkyliert, fluoralkyliert oder alkoxyliert sind.

7. Verbindung nach einem oder mehreren der Ansprüche 1 bis 6, **dadurch gekennzeichnet, dass** es sich um ein konjugiertes Polymer enthaltend eine oder mehrere Einheiten der Formel I wie in einem oder mehreren der Ansprüche 1 bis 6 definiert, und außerdem enthaltend eine oder mehrere Aryl- oder Heteroaryleinheiten, die gegebenenfalls substituiert sind, handelt.

8. Verbindung nach Anspruch 1 oder 7, **dadurch gekennzeichnet, dass** sie aus der Formel II
*-[(Ar¹)ₐ-(U)_{b}-(Ar¹)_{c}-(Ar³)_{d}]ₙ-* II
ausgewählt ist, bei der
U eine Einheit der Formel I, IA, IB oder ihrer Unterformeln wie in einem oder mehreren der Ansprüche 1 bis 6 definiert ist,
Ar¹, Ar², Ar³ bei jedem Auftreten gleich oder verschieden und unabhängig voneinander Aryl oder Heteroaryl bedeuten, das von U verschieden ist, bevorzugt 5 bis 30 Ringatome aufweist und gegebenenfalls durch eine oder mehrere Gruppen R¹ bis R⁴ wie in Anspruch 1 oder 6 definiert substituiert ist,
a, c, d bei jedem Auftreten gleich oder verschieden 0 oder eine ganze Zahl von 1 bis 10 sind,
b eine ganze Zahl von 1 bis 10 ist,
n eine ganze Zahl >1 ist,
bei der das Polymer mindestens eine Wiederholungseinheit -[(Ar¹)ₐ-(U)_{b}-(Ar²)_{c}-(Ar³)_{d}]- enthält, bei der b mindestens 1 ist,
und bei der das * die Bindungen angibt, welche die Einheit entweder mit einem Polymer oder funktionellen Gruppen verknüpfen.

9. Verbindung des Anspruchs 8, **dadurch gekennzeichnet, dass** sie aus der Formel III
R⁵-[(Ar¹)ₐ-(U)_{b}-(Ar²)_{c}-(Ar³)_{d}]ₙ-R⁶ III
ausgewählt ist, bei der Ar¹, Ar², Ar³, U, a, b, c, d und n wie in Anspruch 8 definiert sind, R⁵ und R⁶ unabhängig voneinander H, F, Br, Cl, I, -CH₂Cl, -CHO, -CR'=CR"₂, -SiR'R"R''', -SiR'X'X", - SiR'R"X', -SnR'R"R''', -BR'R", -B(OR')(OR"), -B(OH)₂, -O-SO₂-R', - C≡CH, -C≡C-SiR'₃, -ZnX' oder eine Endverkappungsgruppe bedeuten, X' und X" Halogen bedeuten, R', R" und R'" unabhängig voneinander eine der Bedeutungen von R⁰ besitzen und zwei von R', R" und R'" zusammen mit dem Heteroatom, an das sie gebunden sind, auch einen Ring bilden können, und R⁰ H, gegebenenfalls substituiertes C₁₋₄₀-Carbyl oder -Hydrocarbyl oder gegebenenfalls substituiertes Aryl oder Heteroaryl ist, und bevorzugt Alkyl mit 1 bis 12 C-Atomen oder Aryl oder Heteroaryl mit 5 bis 12 C-Atomen, ganz bevorzugt Methyl, tert-Butyl, Phenyl, Tolyl oder tert-Butyl-phenyl bedeuten.

10. Monomer der Formel IV
R⁷-(Ar¹)ₐ-U-(Ar²)_{c}-R⁸ IV
bei der U, Ar¹, Ar², a und c die in Anspruch 8 angegebenen Bedeutungen besitzen und R⁷ und R⁸ unabhängig voneinander aus der Gruppe bestehend aus Cl, Br, I, O-Tosylat, O-Triflat, O-Mesylat, O-Nonaflat, -SiMe₂F, -SiMeF₂, -O-SO₂Z¹, -B(OZ²)₂, - CZ³=C(Z³)₂, -C≡CH, -C≡CSi(Z¹)₃, -ZnX⁰ und -Sn(Z⁴)₃ ausgewählt sind, bei denen X⁰ Halogen, bevorzugt Cl, Br oder I ist, Z¹⁻⁴ aus der Gruppe bestehend aus Alkyl und Aryl ausgewählt sind, das jeweils gegebenenfalls substituiert ist, und in -B(OZ²)₂ zwei Gruppen Z² zusammen auch eine cyclische Gruppe bilden können.

11. Verfahren zur Herstellung eines konjugierten Polymers nach einem oder mehreren der Ansprüche 1 bis 9 durch Kuppeln eines oder mehrerer Monomere nach Anspruch 10, bei denen R⁷ und R⁸ aus Cl, Br, I, -B(OZ²)₂ und -Sn(Z⁴)₃ ausgewählt sind, miteinander und/oder mit einem oder mehreren Monomeren, die aus den folgenden Formeln ausgewählt sind
R⁷-(Ar¹),-(Ar)²_{c}-(Ar³)_{d}-R⁸ V
R⁷-Ar¹-R⁸ VI
R⁷-Ar²-R⁸ VII
R⁷-Ar³-R⁸ VIII
bei denen Ar¹, Ar², Ar³, a, c und d eine der in Anspruch 8 angegebenen Bedeutungen besitzen, bei denen a+c+d≥2, und R⁷ und R⁸ aus Cl, Br, I, -B(OZ²)₂ und -Sn(Z⁴)₃ ausgewählt sind, in einer Aryl-Aryl-Kupplungsreaktion.

12. Verwendung einer Verbindung nach einem oder mehreren der Ansprüche 1 bis 9 als Elektronendonor oder Halbleiter des p-Typs in einem/r halbleitenden Material, Formulierung, Polymerblend, Vorrichtung oder Komponente einer Vorrichtung.

13. Verwendung einer Verbindung nach einem oder mehreren der Ansprüche 1 bis 9 in Lochtransport-, Elektronentransport-, elektronenblockierendem, lochblockierendem, ladungserzeugendem, leitendem, halbleitendem oder lichtemittierendem Material.

14. Halbleitende(s) Material, Formulierung, Mischung, Polymerblend, Vorrichtung oder Komponente einer Vorrichtung, enthaltend eine Verbindung nach einem oder mehreren der Ansprüche 1 bis 9 als Elektronendonorkomponente.

15. Halbleitende(s) Material, Formulierung, Mischung, Polymerblend, Vorrichtung oder Komponente einer Vorrichtung nach Anspruch 14, außerdem enthaltend eine oder mehrere Verbindungen mit Elektronenakzeptoreigenschaften.

16. Mischung oder Polymerblend enthaltend eine oder mehrere Verbindungen nach einem oder mehreren der Ansprüche 1 bis 9 und außerdem enthaltend eine oder mehrere Verbindungen mit einer oder mehreren von halbleitenden, Ladungstransport-, Loch- oder Elektronentransport-, loch- oder elektronenblockierenden, elektrisch leitenden, photoleitenden oder lichtemittierenden Eigenschaften.

17. Formulierung enthaltend ein(e) oder mehrere Verbindungen, Mischungen oder Polymerblends nach einem oder mehreren der Ansprüche 1 bis 9 und 16 und außerdem enthaltend ein oder mehrere aus organischen Lösungsmitteln ausgewählte Lösungsmittel.

18. Organische halbleitende Formulierung enthaltend eine oder mehrere Verbindungen nach einem oder mehreren der Ansprüche 1 bis 9, ein oder mehrere organische Bindemittel oder Vorläufer davon und gegebenenfalls ein oder mehrere Lösungsmittel.

19. Ladungstransport-, halbleitendes, elektrisch leitendes, photoleitendes oder lichtemittierendes Material enthaltend ein(e) Verbindung, Polymer, Formulierung, Mischung oder Polymerblend nach einem oder mehreren der Ansprüche 1 bis 9 und 16 bis 18.

20. Verwendung einer/s Verbindung, Polymer, Formulierung, Mischung oder Polymerblends nach einem oder mehreren der Ansprüche 1 bis 9 und 16 bis 18 als Ladungstransport-, halbleitendes, elektrisch leitendes, photoleitendes oder lichtemittierendes Material oder in einer optischen, elektrooptischen, elektronischen, Elektrolumineszenz- oder Photolumineszenzvorrichtung oder in einer Komponente einer solchen Vorrichtung oder in einer eine solche Vorrichtung oder Komponente enthaltenden Baugruppe.

21. Optische, elektrooptische, elektronische, Elektrolumineszenz- oder Photolumineszenzvorrichtung oder Komponente davon oder eine diese enthaltende Baugruppe, die ein(e) Verbindung, Polymer, Formulierung, Mischung oder Polymerblend nach einem oder mehreren der Ansprüche 1 bis 9 und 16 bis 18 enthält.

22. Optische, elektrooptische, elektronische, Elektrolumineszenz- oder Photolumineszenzvorrichtung nach Anspruch 21, bei der es sich um eine(n) organischen Feldeffekttransistor (OFET), organischen Dünnschichttransistor (organic thin film transistor - OTFT), organische Leuchtdiode (organic light emitting diode - OLED), organischen lichtemittierenden Transistor (organic light emitting transistor - OLET), organische Photovoltaikvorrichtung (OPV), organischen Photodetektor (OPD), organische Solarzelle, Laserdiode, Schottky-Diode oder organischen Photoleiter handelt.

23. Komponente nach Anspruch 21, bei der es sich um ein(e) Ladungsinjektionsschicht, Ladungstransportschicht, Zwischenschicht, Planarisierungsschicht, Antistatikfolie, Polymerelektrolytmembran (PEM), leitendes Substrat oder leitende Struktur handelt.

24. Baugruppe nach Anspruch 21, bei der es sich um eine(n) integrierte Schaltung (integrated circuit - IC), RFID(radio frequency identification)-Tag, Sicherheitsmarkierung, Sicherheitsvorrichtung, Flachbildschirm, Hintergrundbeleuchtung, elektrophotographische Vorrichtung, elektrophotographische Aufzeichnungsvorrichtung, organische Speichervorrichtung, Sensorvorrichtung, Biosensor oder Biochip handelt.

## Revendications

1. Composé, **caractérisé en ce qu'**il s'agit d'un polymère avec un squelette conjugué comprenant plus d'une unité de la formule I : 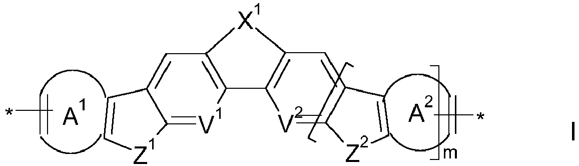 formule dans laquelle :
X¹ est CR¹-CR² ou CR¹R²-CR¹R² ;
V¹ et V² représentent CH ;
Z¹ et Z² représentent, indépendamment l'un de l'autre, S, O ou CR³R⁴ ;
A¹ et A² représentent, indépendamment l'un de l'autre, un groupe aromatique ou hétéroaromatique monocyclique ou bicyclique qui est sélectionné parmi les formules (2a), (3a), (6a), (7a), (10a) et (13a) : 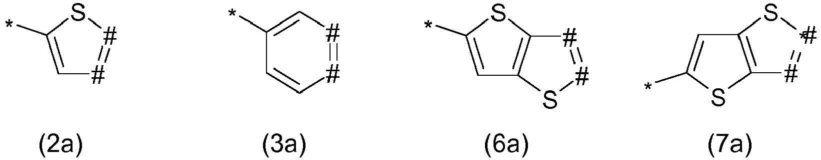 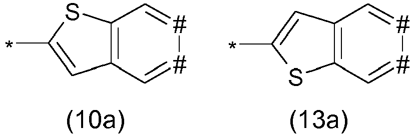 formules dans lesquelles les cycles aromatiques et hétéroaromatiques individuels peuvent également être substitués par un groupe ou par plusieurs groupes R¹ ;
R¹, R², R³ et R⁴ sont, indépendamment les uns des autres, sélectionnés parmi alkyle ou alcoxy en chaîne droite, ramifié ou cyclique avec de 1 à 30 atome(s) de C, lequel est non substitué ou substitué par un ou par plusieurs atome(s) de F, ou aryle ou hétéroaryle, dont chacun est non substitué ou substitué et comporte 4 à 30 atomes de cycle ;
m est 0 ou 1 ;
le symbole * indique les liaisons qui lient l'unité soit avec un polymère, soit avec d'autres groupes optoélectroniquement fonctionnels et le signe # dans un cycle représente un atome de C qui est fusionné sur les cinq cycles adjacents dans la formule I.

2. Composé selon la revendication 1, **caractérisé en ce que** les unités de la formule I sont sélectionnées parmi les formules qui suivent : 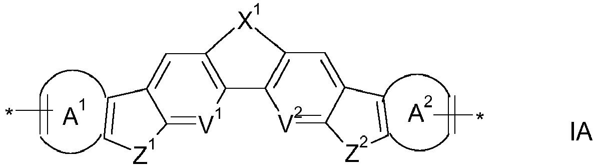 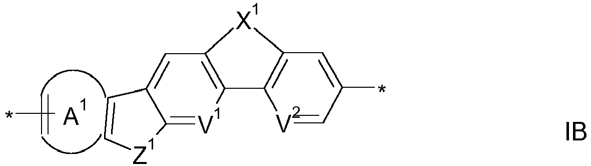 formules dans lesquelles X¹, A¹, A², V¹, V², Z¹ et Z² sont tels que définis selon la revendication 1.

3. Composé selon la revendication 1, **caractérisé en ce que** l'unité de la formule I est sélectionnée parmi les sous-formules qui suivent : 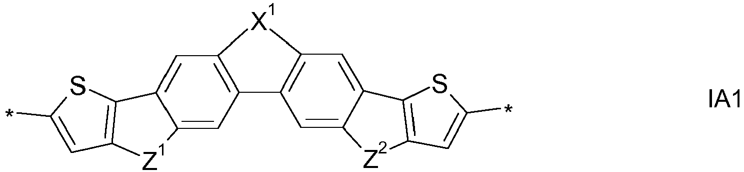 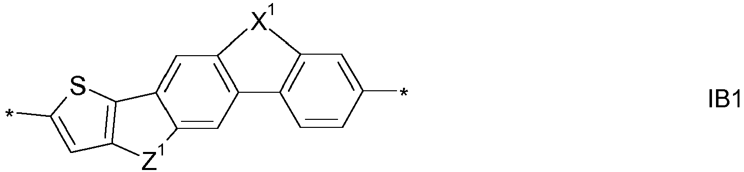 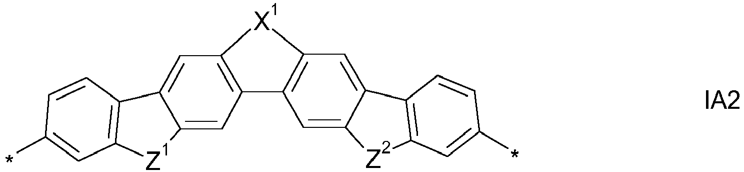 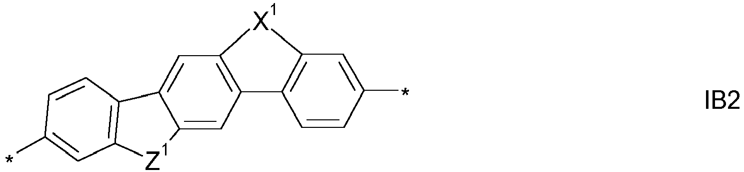 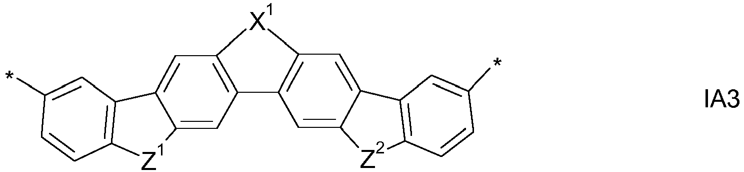 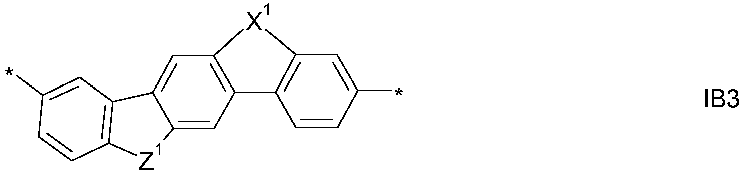 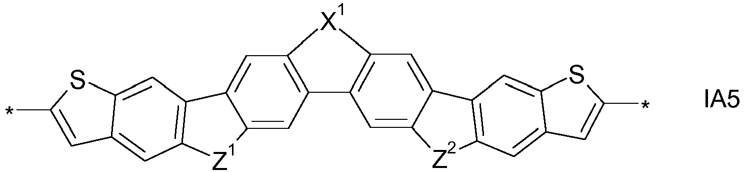 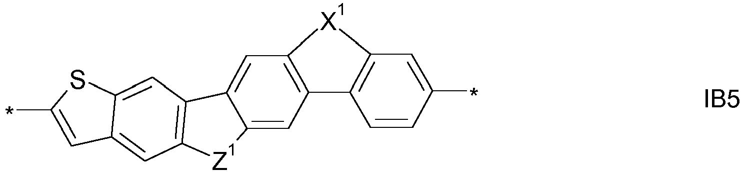 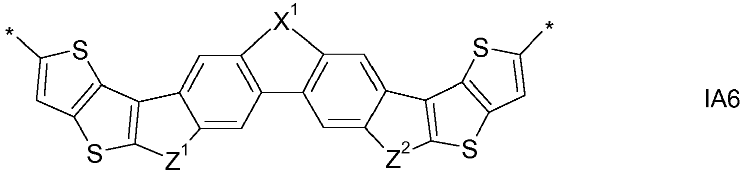 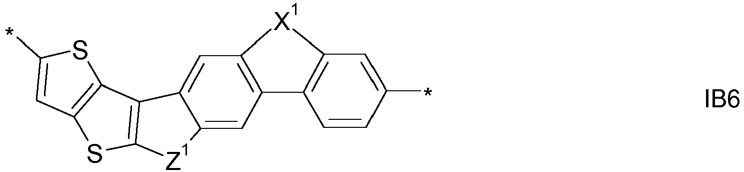 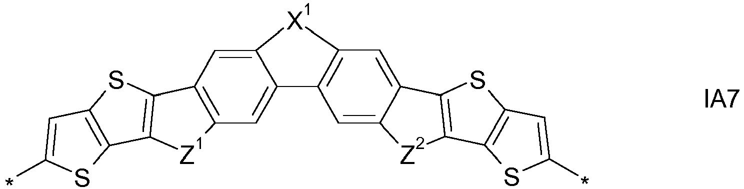 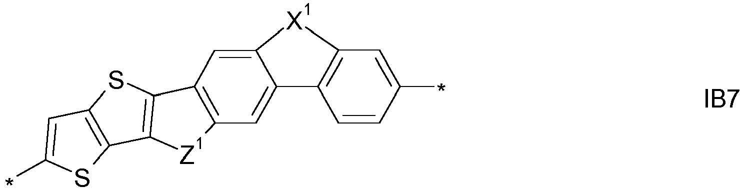 sous-formules dans lesquelles X¹, Z et Z² sont tels que définis selon la revendication 1.

4. Composé selon une ou plusieurs des revendications 1 à 3, **caractérisé en ce que** l'unité de la formule I est sélectionnée parmi les sous-formules qui suivent : 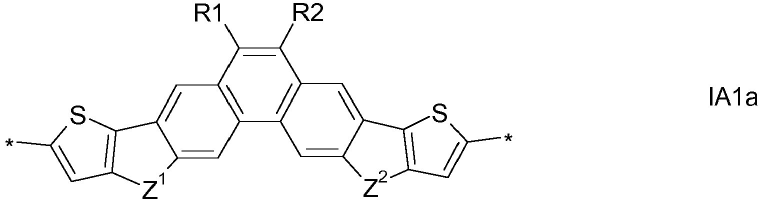 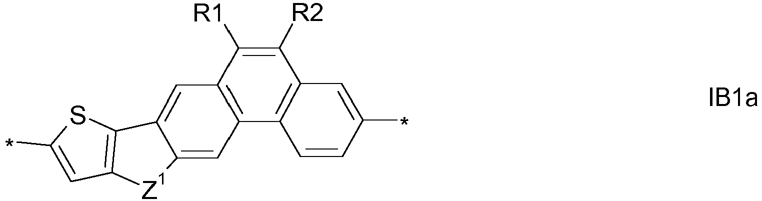 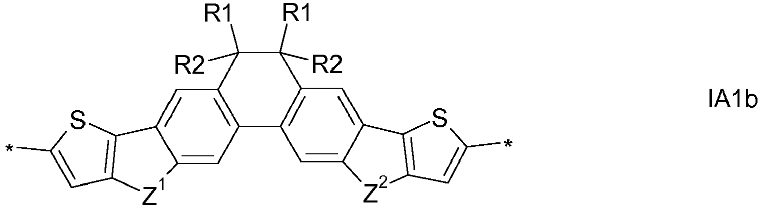 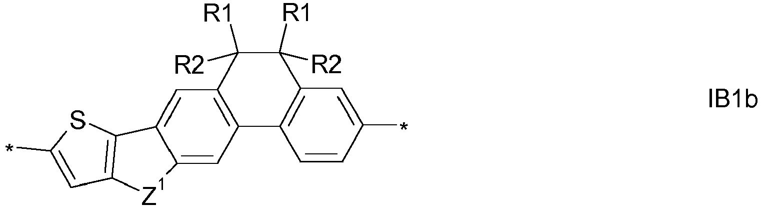 sous-formules dans lesquelles R¹, R², Z¹ et Z² sont tels que définis selon la revendication 1.

5. Composé selon une ou plusieurs des revendications 1 à 4, **caractérisé en ce que** l'unité de la formule I est sélectionnée parmi les sous-formules qui suivent : 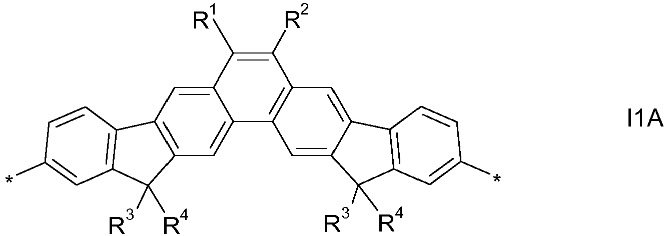 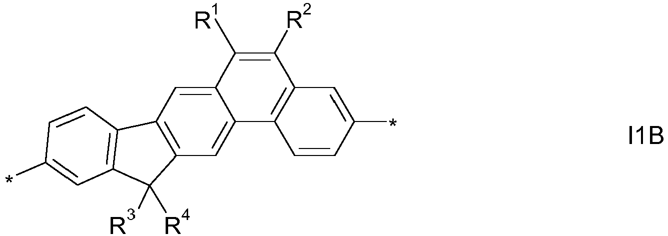 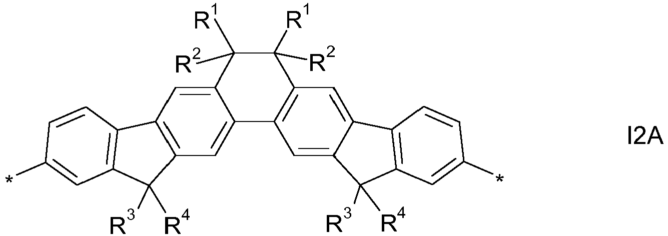 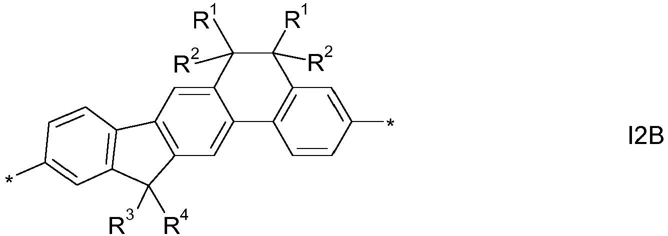 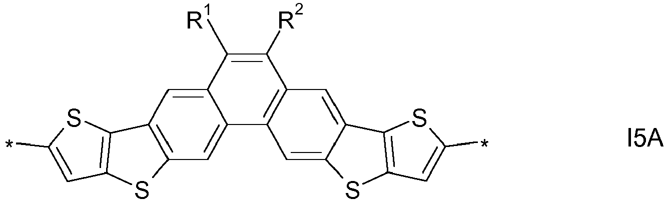 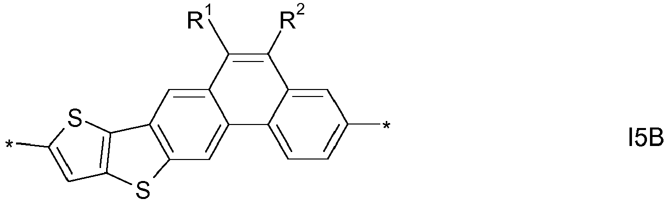 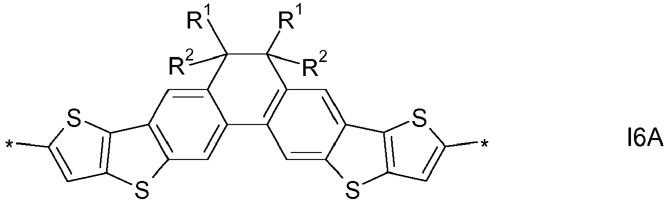 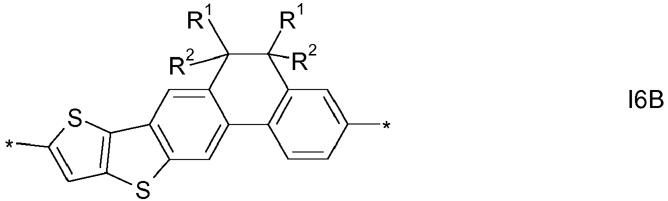 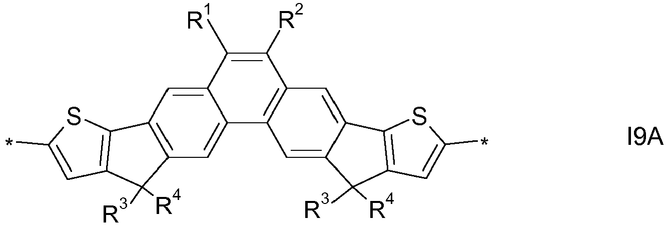 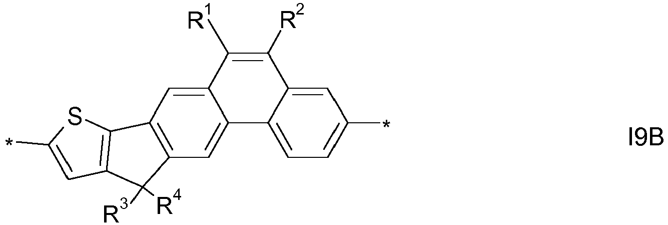 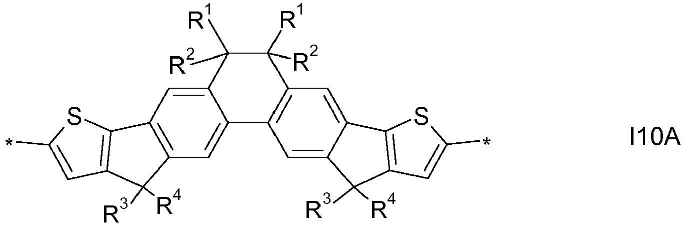 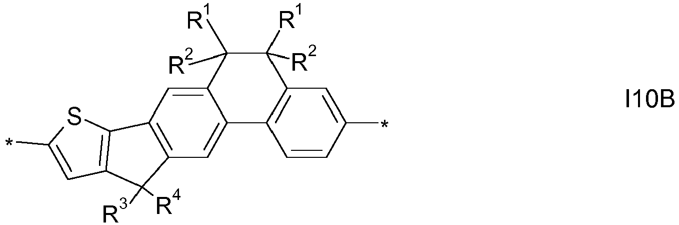 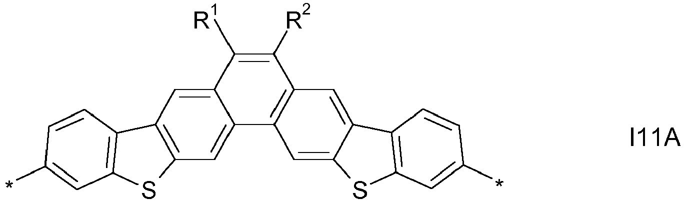 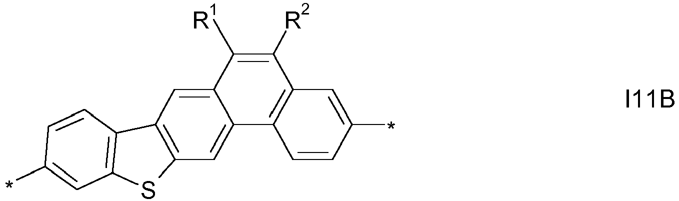 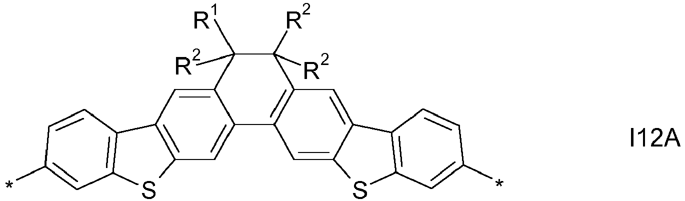 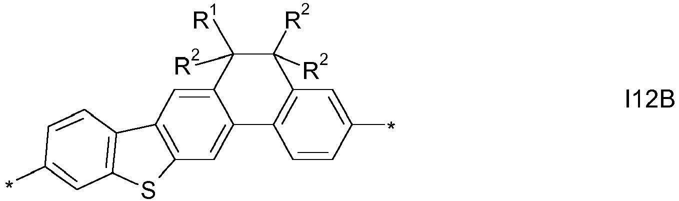 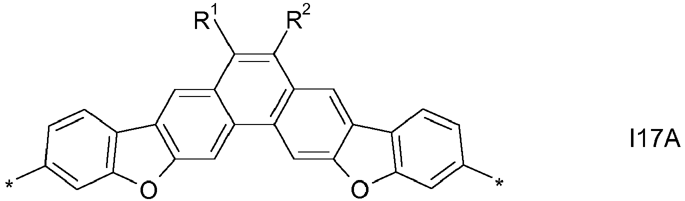 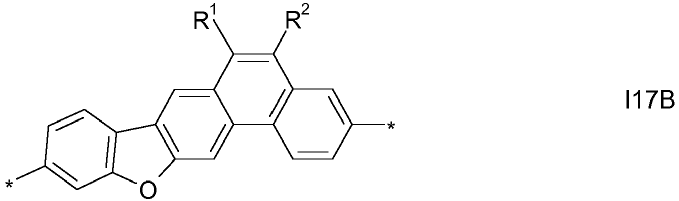 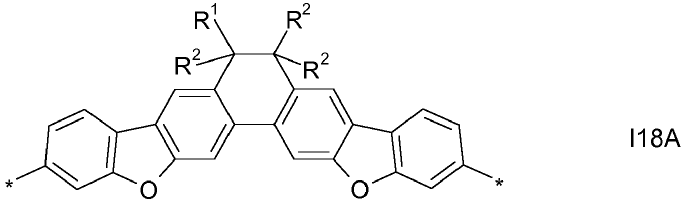 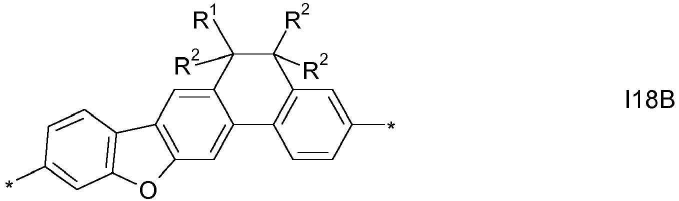 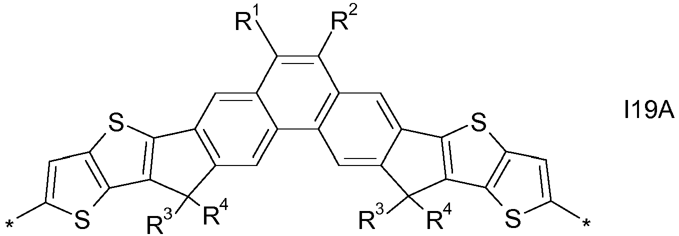 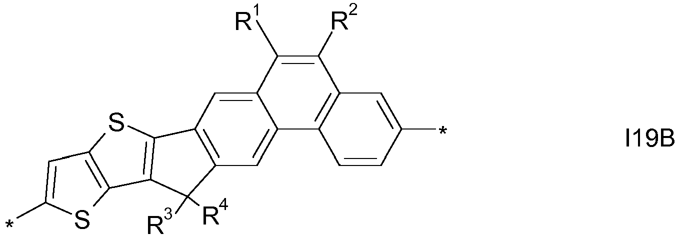 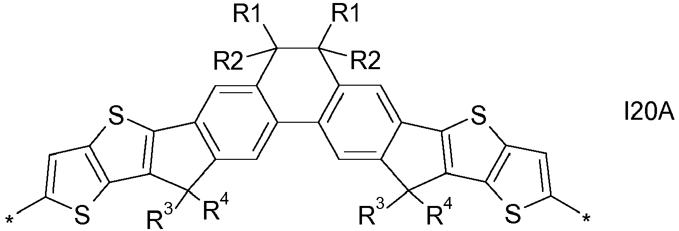 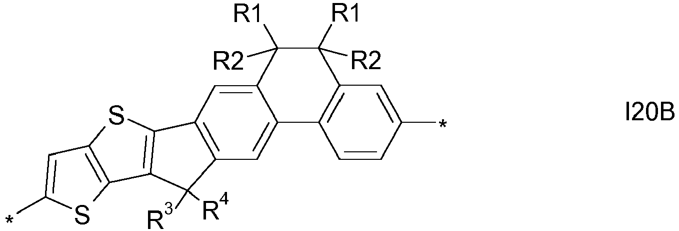 sous-formules dans lesquelles R^{1,2,3,4} présentent les significations qui ont été données selon la revendication 1, et dans lesquelles les cycles benzène externes peuvent également être substitués par un ou deux groupe(s) R¹.

6. Composé selon une ou plusieurs des revendications 1 à 5, **caractérisé en ce que** R¹ et R² représentent alkyle ou alcoxy en chaîne droite, ramifié ou cyclique avec de 1 à 30 atome(s) de C, lequel est en option fluoré, et R³ et R⁴ représentent aryle, aryloxy, hétéroaryle ou hétéroaryloxy, dont chacun comporte de 4 à 30 atomes de cycle et en option, est fluoré, alkylaté, fluoroalkylaté ou alcoxylaté.

7. Composé selon une ou plusieurs des revendications 1 à 6, **caractérisé en ce qu'**il s'agit d'un polymère conjugué qui comprend une ou plusieurs unité(s) de la formule I, tel que défini selon une ou plusieurs des revendications 1 à 6, et qui comprend en outre une ou plusieurs unité(s) aryle ou hétéroaryle qui est/sont en option substituée(s).

8. Composé selon la revendication 1 ou 7, **caractérisé en ce qu'**il est sélectionné à partir de la formule II :
*-[(Ar¹)ₐ-(U)_{b}-(Ar²)_{c}-(Ar³)_{d}]ₙ-* II
formule dans laquelle :
U est une unité de la formule I, IA, IB ou de ses sous-formules, tel que défini selon une ou plusieurs des revendications 1 à 6 ;
Ar¹, Ar², Ar³ sont, pour chaque occurrence, identiques ou différents, et représentent, indépendamment les uns des autres, aryle ou hétéroaryle qui est différent de U, qui, de préférence, comporte 5 à 30 atomes de cycle, et qui est en option substitué par un ou par plusieurs groupe(s) R¹ à R⁴ tels que définis selon la revendication 1 ou 6 ;
a, c, d sont pour chaque occurrence, de manière identique ou différente, 0 ou un entier de 1 à 10 ;
b est un entier de 1 à 10 ;
n est un entier > 1 ;
dans lequel le polymère comprend au moins une unité de répétition -[(Ar¹)ₐ-(U)_{b}-(Ar²)_{c}-(Ar³)_{d}]- dans laquelle b est au moins 1 ;
et dans lequel le symbole * indique les liaisons qui lient l'unité soit avec un polymère, soit avec des groupes fonctionnels.

9. Composé selon la revendication 8, **caractérisé en ce qu'**il est sélectionné à partir de la formule III :
R⁵-[(Ar¹)ₐ-(U)_{b}-(Ar²)_{c}-(Ar³)_{d}]ₙ-R⁶ III
formule dans laquelle Ar¹, Ar², Ar³, U, a, b, c, d et n sont tels que définis selon la revendication 8, R⁵ et R⁶ représentent, indépendamment l'un de l'autre, H, F, Br, Cl, I, -CH₂Cl, -CHO, - CR'=CR"₂, -SiR'R"R''', -SiR'X'X", -SiR'R"X', -SnR'R"R''', -BR'R", - B(OR')(OR"), -B(OH)₂, -O-SO₂-R', -C≡CH, -C≡C-SiR'₃, -ZnX' ou un groupe de coiffe terminale/d'extrémité, X' et X" représentent halogène, R', R" et R''' présentent, indépendamment les uns des autres, l'une des significations de R⁰, et deux de R', R" et R''' peuvent également former un cycle en association avec l'hétéroatome auquel ils sont liés et R⁰ est H, C₁₋₄₀ carbyle ou hydrocarbyle en option substitué, ou aryle ou hétéroaryle en option substitué, et de préférence, représentent alkyle avec de 1 à 12 atome(s) de C, ou aryle ou hétéroaryle avec de 5 à 12 atomes de C, de façon très préférable méthyle, tert-butyle, phényle, tolyle ou tert-butyl-phényle.

10. Monomère de la formule IV :
R⁷-(Ar¹)ₐ-U-(Ar²)_{c}-R⁸ IV
formule dans laquelle U, Ar¹, Ar², a et c présentent les significations qui ont été données selon la revendication 8, et R⁷ et R⁸ sont, indépendamment l'un de l'autre, sélectionnés parmi le groupe qui est constitué par Cl, Br, I, O-tosylate, O-triflate, O-mésylate, O-nonaflate, -SiMe2F, -SiMeF₂, -O-SO₂Z¹, -B(OZ²)₂, - CZ³=C(Z³)₂, -C≡CH, -C≡CSi(Z¹)₃, -ZnX⁰ et -Sn(Z⁴)₃, où X⁰ est halogène, de préférence Cl, Br ou I, Z¹⁻⁴ sont sélectionnés parmi le groupe qui est constitué par alkyle et aryle, chacun étant en option substitué, et dans -B(OZ²)₂, deux groupes Z² peuvent également former ensemble un groupe cyclique.

11. Procédé de préparation d'un polymère conjugué selon une ou plusieurs des revendications 1 à 9, en couplant un ou plusieurs monomère(s) selon la revendication 10, où R⁷ et R⁸ sont sélectionnés parmi Cl, Br, I, -B(OZ²)₂ et -Sn(Z⁴)₃, les uns avec les autres et/ou avec un ou plusieurs monomère(s) qui est/sont sélectionné(s) parmi les formules qui suivent :
R⁷-(Ar¹)ₐ-(Ar)²_{c}-(Ar³)_{d}-R⁸ V
R⁷-Ar¹-R⁸ VI
R⁷-Ar²-R⁸ VII
R⁷-Ar³-R⁸ VIII
formules dans lesquelles Ar¹, Ar², Ar³, a, c et d présentent l'une des significations qui ont été données selon la revendication 8, où a+c+d ≥ 2, et R⁷ et R⁸ sont sélectionnés parmi Cl, Br, I, -B(OZ²)₂ et -Sn(Z⁴)₃, selon une réaction de couplage aryle-aryle.

12. Utilisation d'un composé selon une ou plusieurs des revendications 1 à 9 en tant que semiconducteur donneur ou de type p dans un matériau de semiconduction, une formulation, un mélange de polymères, un dispositif ou un composant d'un dispositif.

13. Utilisation d'un composé selon une ou plusieurs des revendications 1 à 9 dans un matériau de transport de trous, de transport d'électrons, de blocage d'électrons, de blocage de trous, de génération de charges, de conduction, de semiconduction ou d'émission de lumière.

14. Matériau de semiconduction, formulation, mixture, mélange de polymères, dispositif ou composant d'un dispositif, comprenant un composé selon une ou plusieurs des revendications 1 à 9 en tant que composant donneur d'électrons.

15. Matériau de semiconduction, formulation, mixture, mélange de polymères, dispositif ou composant d'un dispositif selon la revendication 14, comprenant en outre un ou plusieurs composé(s) présentant des propriétés d'accepteur d'électrons.

16. Mixture ou mélange de polymères comprenant un ou plusieurs composé(s) selon une ou plusieurs des revendications 1 à 9, et comprenant en outre un ou plusieurs composé(s) présentant une ou plusieurs propriété(s) prise(s) parmi des propriétés de semiconduction, de transport de charges, de transport de trous ou d'électrons, de blocage de trous ou d'électrons, de conduction électrique, de photoconduction ou d'émission de lumière.

17. Formulation comprenant un(e) ou plusieurs composé(s), mixture(s) ou mélange(s) de polymères selon une ou plusieurs des revendications 1 à 9 et 16, et comprenant en outre un ou plusieurs solvant(s) qui est/sont sélectionné(s) parmi les solvants organiques.

18. Formulation de semiconduction organique comprenant un ou plusieurs composé(s) selon une ou plusieurs des revendications 1 à 9, un ou plusieurs liant(s) organique(s) ou son/leurs précurseur(s), et en option, un ou plusieurs solvant(s).

19. Matériau de transport de charges, de semiconduction, de conduction électrique, de photoconduction ou d'émission de lumière comprenant un composé, un polymère, une formulation, une mixture ou un mélange de polymères selon une ou plusieurs des revendications 1 à 9 et 16 à 18.

20. Utilisation d'un composé, d'un polymère, d'une formulation, d'une mixture ou d'un mélange de polymères selon une ou plusieurs des revendications 1 à 9 et 16 à 18 en tant que matériau de transport de charges, de semiconduction, de conduction électrique, de photoconduction ou d'émission de lumière, ou dans un dispositif optique, électrooptique, électronique, électroluminescent ou photoluminescent, ou dans un composant d'un tel dispositif ou dans un assemblage comprenant un tel dispositif ou composant.

21. Dispositif optique, électrooptique, électronique, électroluminescent ou photoluminescent, ou son composant, ou assemblage le comprenant, lequel comprend un composé, un polymère, une formulation, une mixture ou un mélange de polymères selon une ou plusieurs des revendications 1 à 9 et 16 à 18.

22. Dispositif optique, électrooptique, électronique, électroluminescent ou photoluminescent selon la revendication 21, lequel est un transistor à effet de champ organique (OFET), un transistor à film mince organique (OTFT), une diode à émission de lumière organique (OLED), un transistor à émission de lumière organique (OLET), un dispositif photovoltaïque organique (OPV), un photodétecteur organique (OPD), une cellule solaire organique, une diode laser, une diode Schottky ou un photoconducteur organique.

23. Composant selon la revendication 21, lequel est une couche d'injection de charges, une couche de transport de charges, une inter-couche, une couche de planarisation, un film antistatique, une membrane électrolytique polymère (PEM), un substrat de conduction ou un motif de conduction.

24. Assemblage selon la revendication 21, lequel est un circuit intégré (IC), une étiquette d'identification radiofréquence (RFID), un marquage de sécurité, un dispositif de sécurité, un affichage à écran plat, un éclairage arrière ou rétro-éclairage, un dispositif électrophotographique, un dispositif d'enregistrement électrophotographique, un dispositif de mémoire organique, un dispositif de capteur, un bio-capteur ou une biopuce.
